(19) Europäisches Patentamt
European Patent Office
Office européen des brevets

(11) EP 3 317 388 B1

# (12) EUROPEAN PATENT SPECIFICATION

(45) Date of publication and mention of the grant of the patent:
**13.11.2019 Bulletin 2019/46**

(21) Application number: **16730861.8**

(22) Date of filing: **23.06.2016**

(51) Int Cl.:
***C11D 3/386*** *(2006.01)*

(86) International application number:
**PCT/EP2016/064526**

(87) International publication number:
**WO 2016/135351 (01.09.2016 Gazette 2016/35)**

## (54) LAUNDRY DETERGENT COMPOSITION, METHOD FOR WASHING AND USE OF COMPOSITION

WASCHMITTELZUSAMMENSETZUNG, VERFAHREN ZUM WASCHEN UND VERWENDUNG DER ZUSAMMENSETZUNG

COMPOSITION DE DÉTERGENT À LESSIVE, PROCÉDÉ POUR LE LAVAGE ET UTILISATION DE LA COMPOSITION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.06.2015 EP 15174596**

(43) Date of publication of application:
**09.05.2018 Bulletin 2018/19**

(73) Proprietor: **Novozymes A/S**
**2880 Bagsvaerd (DK)**

(72) Inventors:
- **SKAGERLIND, Jan, Peter**
  **25220 Helsingborg (SE)**
- **BARJONA, Paulo, Cesar**
  **CEP-83707-660 Araucária-PR (BR)**

(74) Representative: **NZ EPO Representatives**
**Krogshoejvej 36**
**2880 Bagsvaerd (DK)**

(56) References cited:
**WO-A1-2013/028928**
**WO-A1-2013/182740**
**WO-A1-2014/026630**
**WO-A2-2004/099228**
**WO-A2-2013/119302**
**WO-A2-2014/018368**
**US-A1- 2013 095 554**
**US-A1- 2013 302 879**
**US-A1- 2015 064 773**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 317 388 B1

SEQUENCE LISTING

<110> Novozymes A/S

<120> Laundry detergent composition, method for washing and use of composition.

<130> 12928-WO-PCT

<140>
<141> 2016-06-23

<160> 18

<170> PatentIn version 3.5

<210> 1
<211> 1599
<212> DNA
<213> Aspergillus fumigatus

<400> 1

```
atgctggcct ccaccttctc ctaccgcatg tacaagaccg cgctcatcct ggccgccctt      60
ctgggctctg gccaggctca gcaggtcggt acttcccagg cggaagtgca tccgtccatg     120
acctggcaga gctgcacggc tggcggcagc tgcaccacca acaacggcaa ggtggtcatc     180
gacgcgaact ggcgttgggt gcacaaagtc ggcgactaca ccaactgcta caccggcaac     240
acctgggaca cgactatctg ccctgacgat gcgacctgcg catccaactg cgcccttgag     300
ggtgccaact acgaatccac ctatggtgtg accgccagcg gcaattccct ccgcctcaac     360
ttcgtcacca ccagccagca gaagaacatt ggctcgcgtc tgtacatgat gaaggacgac     420
tcgacctacg agatgtttaa gctgctgaac caggagttca ccttcgatgt cgatgtctcc     480
aacctcccct gcggtctcaa cggtgctctg tactttgtcg ccatggacgc cgacggtggc     540
atgtccaagt acccaaccaa caaggccggt gccaagtacg gtactggata ctgtgactcg     600
cagtgccctc gcgacctcaa gttcatcaac ggtcaggcca acgtcgaagg gtggcagccc     660
tcctccaacg atgccaatgc gggtaccggc aaccacgggt cctgctgcgc ggagatggat     720
atctgggagg ccaacagcat ctccacggcc ttcaccccca atccgtgcga cacgcccggc     780
caggtgatgt gcaccggtga tgcctgcggt ggcacctaca gctccgaccg ctacggcggc     840
acctgcgacc ccgacggatg tgatttcaac tccttccgcc agggcaacaa gaccttctac     900
ggccctggca tgaccgtcga caccaagagc aagtttaccg tcgtcaccca gttcatcacc     960
gacgacggca cctccagcgg caccctcaag gagatcaagc gcttctacgt gcagaacggc    1020
aaggtgatcc ccaactcgga gtcgacctgg accggcgtca gcggcaactc catcaccacc    1080
gagtactgca ccgcccagaa gagcctgttc caggaccaga acgtcttcga aaagcacggc    1140
ggcctcgagg gcatgggtgc tgccctcgcc cagggtatgg ttctcgtcat gtccctgtgg    1200
gatgatcact cggccaacat gctctggctc gacagcaact acccgaccac tgcctcttcc    1260
accactcccg gcgtcgcccg tggtacctgc gacatctcct ccggcgtccc tgcggatgtc    1320
gaggcgaacc accccgacgc ctacgtcgtc tactccaaca tcaaggtcgg ccccatcggc    1380
tcgaccttca acagcggtgg ctcgaacccc ggtggcggaa ccaccacgac aactaccacc    1440
cagcctacta ccaccacgac cacggctgga aaccctggcg gcaccggagt cgcacagcac    1500
tatggccagt gtggtggaat cggatggacc ggacccacaa cctgtgccag cccttatacc    1560
tgccagaagc tgaatgatta ttactctcag tgcctgtag                          1599
```

<210> 2
<211> 532
<212> PRT
<213> Aspergillus fumigatus

<400> 2

```
Met Leu Ala Ser Thr Phe Ser Tyr Arg Met Tyr Lys Thr Ala Leu Ile
1               5                   10                  15

Leu Ala Ala Leu Leu Gly Ser Gly Gln Ala Gln Gln Val Gly Thr Ser
            20                  25                  30

Gln Ala Glu Val His Pro Ser Met Thr Trp Gln Ser Cys Thr Ala Gly
        35                  40                  45

Gly Ser Cys Thr Thr Asn Asn Gly Lys Val Val Ile Asp Ala Asn Trp
    50                  55                  60

Arg Trp Val His Lys Val Gly Asp Tyr Thr Asn Cys Tyr Thr Gly Asn
65                  70                  75                  80

Thr Trp Asp Thr Thr Ile Cys Pro Asp Asp Ala Thr Cys Ala Ser Asn
                85                  90                  95

Cys Ala Leu Glu Gly Ala Asn Tyr Glu Ser Thr Tyr Gly Val Thr Ala
            100                 105                 110

Ser Gly Asn Ser Leu Arg Leu Asn Phe Val Thr Thr Ser Gln Gln Lys
        115                 120                 125

Asn Ile Gly Ser Arg Leu Tyr Met Met Lys Asp Asp Ser Thr Tyr Glu
    130                 135                 140

Met Phe Lys Leu Leu Asn Gln Glu Phe Thr Phe Asp Val Asp Val Ser
145                 150                 155                 160

Asn Leu Pro Cys Gly Leu Asn Gly Ala Leu Tyr Phe Val Ala Met Asp
                165                 170                 175
```

```
Ala Asp Gly Gly Met Ser Lys Tyr Pro Thr Asn Lys Ala Gly Ala Lys
            180                 185                 190

Tyr Gly Thr Gly Tyr Cys Asp Ser Gln Cys Pro Arg Asp Leu Lys Phe
        195                 200                 205

Ile Asn Gly Gln Ala Asn Val Glu Gly Trp Gln Pro Ser Ser Asn Asp
    210                 215                 220

Ala Asn Ala Gly Thr Gly Asn His Gly Ser Cys Cys Ala Glu Met Asp
225                 230                 235                 240

Ile Trp Glu Ala Asn Ser Ile Ser Thr Ala Phe Thr Pro His Pro Cys
                245                 250                 255

Asp Thr Pro Gly Gln Val Met Cys Thr Gly Asp Ala Cys Gly Gly Thr
            260                 265                 270

Tyr Ser Ser Asp Arg Tyr Gly Gly Thr Cys Asp Pro Asp Gly Cys Asp
        275                 280                 285

Phe Asn Ser Phe Arg Gln Gly Asn Lys Thr Phe Tyr Gly Pro Gly Met
    290                 295                 300

Thr Val Asp Thr Lys Ser Lys Phe Thr Val Val Thr Gln Phe Ile Thr
305                 310                 315                 320

Asp Asp Gly Thr Ser Ser Gly Thr Leu Lys Glu Ile Lys Arg Phe Tyr
                325                 330                 335

Val Gln Asn Gly Lys Val Ile Pro Asn Ser Glu Ser Thr Trp Thr Gly
            340                 345                 350

Val Ser Gly Asn Ser Ile Thr Thr Glu Tyr Cys Thr Ala Gln Lys Ser
        355                 360                 365

Leu Phe Gln Asp Gln Asn Val Phe Glu Lys His Gly Gly Leu Glu Gly
    370                 375                 380

Met Gly Ala Ala Leu Ala Gln Gly Met Val Leu Val Met Ser Leu Trp
385                 390                 395                 400

Asp Asp His Ser Ala Asn Met Leu Trp Leu Asp Ser Asn Tyr Pro Thr
                405                 410                 415

Thr Ala Ser Ser Thr Thr Pro Gly Val Ala Arg Gly Thr Cys Asp Ile
```

```
                420                 425                 430

Ser Ser Gly Val Pro Ala Asp Val Glu Ala Asn His Pro Asp Ala Tyr
        435                 440                 445

Val Val Tyr Ser Asn Ile Lys Val Gly Pro Ile Gly Ser Thr Phe Asn
    450                 455                 460

Ser Gly Gly Ser Asn Pro Gly Gly Gly Thr Thr Thr Thr Thr Thr Thr
465                 470                 475                 480

Gln Pro Thr Thr Thr Thr Thr Thr Ala Gly Asn Pro Gly Gly Thr Gly
                485                 490                 495

Val Ala Gln His Tyr Gly Gln Cys Gly Gly Ile Gly Trp Thr Gly Pro
            500                 505                 510

Thr Thr Cys Ala Ser Pro Tyr Thr Cys Gln Lys Leu Asn Asp Tyr Tyr
        515                 520                 525

Ser Gln Cys Leu
    530
```

<210> 3
<211> 1713
<212> DNA
<213> Aspergillus fumigatus

<400> 3

```
atgaagcacc ttgcatcttc catcgcattg actctactgt tgcctgccgt gcaggcccag        60
cagaccgtat ggggccaatg tatgttctgg ctgtcactgg aataagactg tatcaactgc       120
tgatatgctt ctaggtggcg gccaaggctg gtctggcccg acgagctgtg ttgccggcgc       180
agcctgtagc acactgaatc cctgtatgtt agatatcgtc ctgagtggag acttatactg       240
acttccttag actacgctca gtgtatcccg ggagccaccg cgacgtccac caccctcacg       300
acgacgacgg cggcgacgac gacatcccag accaccacca aacctaccac gactggtcca       360
actacatccg cacccaccgt gaccgcatcc ggtaaccctt tcagcggcta ccagctgtat       420
gccaacccct actactcctc cgaggtccat actctggcca tgccttctct gcccagctcg       480
ctgcagccca aggctagtgc tgttgctgaa gtgccctcat ttgtttggct gtaagtggcc       540
ttatcccaat actgagacca actctctgac agtcgtagcg acgttgccgc caaggtgccc       600
actatgggaa cctacctggc cgacattcag gccaagaaca aggccggcgc caaccctcct       660
atcgctggta tcttcgtggt ctacgacttg ccggaccgtg actgcgccgc tctggccagt       720
aatggcgagt actcaattgc caacaacggt gtggccaact acaaggcgta cattgacgcc       780

atccgtgctc agctggtgaa gtactctgac gttcacacca tcctcgtcat cggtaggccg       840
tacacctccg ttgcgcgccg cctttctctg acatcttgca gaacccgaca gcttggccaa       900
cctggtgacc aacctcaacg tcgccaaatg cgccaatgcg cagagcgcct acctggagtg       960
tgtcgactat gctctgaagc agctcaacct gcccaacgtc gccatgtacc tcgacgcagg      1020
tatgcctcac ttcccgcatt ctgtatccct tccagacact aactcatcag gccatgcggg      1080
ctggctcgga tggcccgcca acttgggccc cgccgcaaca ctcttcgcca aagtctacac      1140
cgacgcgggt tcccccgcgg ctgttcgtgg cctggccacc aacgtcgcca actacaacgc      1200
ctggtcgctc agtacctgcc cctcctacac ccagggagac cccaactgcg acgagaagaa      1260
gtacatcaac gccatggcgc ctcttctcaa ggaagccggc ttcgatgccc acttcatcat      1320
ggatacctgt aagtgcttat tccaatcgcc gatgtgtgcc gactaatcaa tgtttcagcc      1380
cggaatggcg tccagcccac gaagcaaaac gcctggggtg actggtgcaa cgtcatcggc      1440
accggcttcg gtgttcgccc ctcgactaac accggcgatc cgctccagga tgcctttgtg      1500
tggatcaagc ccggtggaga gagtgatggc acgtccaact cgacttcccc ccggtatgac      1560
gcgcactgcg gatatagtga tgctctgcag cctgctcctg aggctggtac ttggttccag      1620
gtatgtcatc cattagccag atgagggata agtgactgac ggacctaggc ctactttgag      1680
cagcttctga ccaacgctaa cccgtccttt taa                                   1713
```

<210> 4
<211> 454

<212> PRT
<213> Aspergillus fumigatus

<400> 4

```
Met Lys His Leu Ala Ser Ser Ile Ala Leu Thr Leu Leu Leu Pro Ala
1               5                   10                  15

Val Gln Ala Gln Gln Thr Val Trp Gly Gln Cys Gly Gly Gln Gly Trp
            20                  25                  30

Ser Gly Pro Thr Ser Cys Val Ala Gly Ala Ala Cys Ser Thr Leu Asn
        35                  40                  45

Pro Tyr Tyr Ala Gln Cys Ile Pro Gly Ala Thr Ala Thr Ser Thr Thr
    50                  55                  60

Leu Thr Thr Thr Thr Ala Ala Thr Thr Thr Ser Gln Thr Thr Thr Lys
65                  70                  75                  80

Pro Thr Thr Thr Gly Pro Thr Thr Ser Ala Pro Thr Val Thr Ala Ser
                85                  90                  95
```

```
Gly Asn Pro Phe Ser Gly Tyr Gln Leu Tyr Ala Asn Pro Tyr Tyr Ser
            100                 105                 110

Ser Glu Val His Thr Leu Ala Met Pro Ser Leu Pro Ser Ser Leu Gln
        115                 120                 125

Pro Lys Ala Ser Ala Val Ala Glu Val Pro Ser Phe Val Trp Leu Asp
    130                 135                 140

Val Ala Ala Lys Val Pro Thr Met Gly Thr Tyr Leu Ala Asp Ile Gln
145                 150                 155                 160

Ala Lys Asn Lys Ala Gly Ala Asn Pro Pro Ile Ala Gly Ile Phe Val
                165                 170                 175

Val Tyr Asp Leu Pro Asp Arg Asp Cys Ala Ala Leu Ala Ser Asn Gly
            180                 185                 190

Glu Tyr Ser Ile Ala Asn Asn Gly Val Ala Asn Tyr Lys Ala Tyr Ile
        195                 200                 205

Asp Ala Ile Arg Ala Gln Leu Val Lys Tyr Ser Asp Val His Thr Ile
    210                 215                 220

Leu Val Ile Glu Pro Asp Ser Leu Ala Asn Leu Val Thr Asn Leu Asn
225                 230                 235                 240

Val Ala Lys Cys Ala Asn Ala Gln Ser Ala Tyr Leu Glu Cys Val Asp
                245                 250                 255

Tyr Ala Leu Lys Gln Leu Asn Leu Pro Asn Val Ala Met Tyr Leu Asp
            260                 265                 270

Ala Gly His Ala Gly Trp Leu Gly Trp Pro Ala Asn Leu Gly Pro Ala
        275                 280                 285

Ala Thr Leu Phe Ala Lys Val Tyr Thr Asp Ala Gly Ser Pro Ala Ala
    290                 295                 300

Val Arg Gly Leu Ala Thr Asn Val Ala Asn Tyr Asn Ala Trp Ser Leu
305                 310                 315                 320

Ser Thr Cys Pro Ser Tyr Thr Gln Gly Asp Pro Asn Cys Asp Glu Lys
                325                 330                 335

Lys Tyr Ile Asn Ala Met Ala Pro Leu Leu Lys Glu Ala Gly Phe Asp
            340                 345                 350
```

```
Ala His Phe Ile Met Asp Thr Ser Arg Asn Gly Val Gln Pro Thr Lys
        355                 360                 365

Gln Asn Ala Trp Gly Asp Trp Cys Asn Val Ile Gly Thr Gly Phe Gly
    370                 375                 380

Val Arg Pro Ser Thr Asn Thr Gly Asp Pro Leu Gln Asp Ala Phe Val
385                 390                 395                 400

Trp Ile Lys Pro Gly Gly Glu Ser Asp Gly Thr Ser Asn Ser Thr Ser
                405                 410                 415

Pro Arg Tyr Asp Ala His Cys Gly Tyr Ser Asp Ala Leu Gln Pro Ala
            420                 425                 430

Pro Glu Ala Gly Thr Trp Phe Gln Ala Tyr Phe Glu Gln Leu Leu Thr
        435                 440                 445

Asn Ala Asn Pro Ser Phe
    450
```

<210> 5
<211> 3060
<212> DNA
<213> Aspergillus fumigatus

<400> 5

```
atgagattcg gttggctcga ggtggccgct ctgacggccg cttctgtagc caatgcccag      60
gtttgtgatg ctttcccgtc attgtttcgg atatagttga caatagtcat ggaaataatc     120
aggaattggc tttctctcca ccattctacc cttcgccttg ggctgatggc cagggagagt     180
gggcagatgc ccatcgacgc gccgtcgaga tcgtttctca gatgacactg gcggagaagg     240
ttaaccttac aacgggtact gggtgggttg cgactttttt gttgacagtg agctttcttc     300
actgaccatc tacacagatg ggaaatggac cgatgcgtcg gtcaaaccgg cagcgttccc     360
aggtaagctt gcaattctgc aacaacgtgc aagtgtagtt gctaaaacgc ggtggtgcag     420
acttggtatc aactggggtc tttgtggcca ggattcccct ttgggtatcc gtttctgtga     480
gctatacccg cggagtcttt cagtccttgt attatgtgct gatgattgtc tctgtatagc     540
tgacctcaac tccgccttcc ctgctggtac taatgtcgcc gcgacatggg acaagacact     600
cgcctacctt cgtggcaagg ccatgggtga ggaattcaac gacaagggcg tggacatttt     660
gctggggcct gctgctggtc ctctcggcaa atacccggac ggcggcagaa tctgggaagg     720
cttctctcct gatccggttc tcactggtgt acttttcgcc gaaactatca agggtatcca     780
agacgcgggt gtgattgcta ctgccaagca ttacattctg aatgaacagg agcatttccg     840
```

acaggttggc gaggcccagg gatatggtta caacatcacg gagacgatca gctccaacgt 900
ggatgacaag accatgcacg agttgtacct ttggtgagta gttgacactg caaatgagga 960
ccttgattga tttgactgac ctggaatgca ggccctttgc agatgctgtg cgcggtaaga 1020
ttttccgtag acttgacctc gcgacgaaga aatcgctgac gaaccatcgt agctggcgtt 1080
ggcgctgtca tgtgttccta caatcaaatc aacaacagct acggttgtca aaacagtcaa 1140
actctcaaca agctcctcaa ggctgagctg ggcttccaag gcttcgtcat gagtgactgg 1200
agcgctcacc acagcggtgt cggcgctgcc ctcgctgggt tggatatgtc gatgcctgga 1260
gacatttcct tcgacgacgg actctccttc tggggcacga acctaactgt cagtgttctt 1320
aacggcaccg ttccagcctg gcgtgtcgat gacatggctg ttcgtatcat gaccgcgtac 1380
tacaaggttg gtcgtgaccg tcttcgtatt ccccctaact tcagctcctg gacccgggat 1440
gagtacggct gggagcattc tgctgtctcc gagggagcct ggaccaaggt gaacgacttc 1500
gtcaatgtgc agcgcagtca ctctcagatc atccgtgaga ttggtgccgc tagtacagtg 1560
ctcttgaaga acacgggtgc tcttcctttg accggcaagg aggttaaagt gggtgttctc 1620
ggtgaagacg ctggttccaa cccgtggggt gctaacggct gccccgaccg cggctgtgat 1680
aacggcactc ttgctatggc ctggggtagt ggtactgcca acttccctta ccttgtcacc 1740
cccgagcagg ctatccagcg agaggtcatc agcaacggcg gcaatgtctt tgctgtgact 1800
gataacgggg ctctcagcca gatggcagat gttgcatctc aatccaggtg agtgcgggct 1860
cttagaaaaa gaacgttctc tgaatgaagt tttttaacca ttgcgaacag cgtgtctttg 1920
gtgtttgtca acgccgactc tggagagggt ttcatcagtg tcgacggcaa cgagggtgac 1980
cgcaaaaatc tcactctgtg gaagaacggc gaggccgtca ttgacactgt tgtcagccac 2040
tgcaacaaca cgattgtggt tattcacagt gttgggcccg tcttgatcga ccggtggtat 2100
gataacccca acgtcactgc catcatctgg gccggcttgc ccggtcagga gagtggcaac 2160
tccctggtcg acgtgctcta tggccgcgtc aaccccagcg ccaagacccc gttcacctgg 2220
ggcaagactc gggagtctta cggggctccc ttgctcaccg agcctaacaa tggcaatggt 2280
gctccccagg atgatttcaa cgagggcgtc ttcattgact accgtcactt tgacaagcgc 2340
aatgagaccc ccatttatga gtttggccat ggcttgagct acaccacctt tggttactct 2400
caccttcggg ttcaggccct caatagttcg agttcggcat atgtcccgac tagcggagag 2460
accaagcctg cgccaaccta tggtgagatc ggtagtgccg ccgactacct gtatcccgag 2520
ggtctcaaaa gaattaccaa gtttatttac ccttggctca actcgaccga cctcgaggat 2580
tcttctgacg acccgaacta cggctgggag gactcggagt acattcccga aggcgctagg 2640
gatgggtctc ctcaacccct cctgaaggct ggcggcgctc ctggtggtaa ccctaccctt 2700

```
tatcaggatc ttgttagggt gtcggccacc ataaccaaca ctggtaacgt cgccggttat      2760
gaagtccctc aattggtgag tgacccgcat gttccttgcg ttgcaatttg gctaactcgc      2820
ttctagtatg tttcactggg cggaccgaac gagcctcggg tcgttctgcg caagttcgac      2880
cgaatcttcc tggctcctgg ggagcaaaag gtttggacca cgactcttaa ccgtcgtgat      2940
ctcgccaatt gggatgtgga ggctcaggac tgggtcatca caaagtaccc caagaaagtg      3000
cacgtcggca gctcctcgcg taagctgcct ctgagagcgc ctctgccccg tgtctactag      3060
```

<210> 6
<211> 863
<212> PRT
<213> Aspergillus fumigatus

<400> 6

```
Met Arg Phe Gly Trp Leu Glu Val Ala Ala Leu Thr Ala Ala Ser Val
1               5                   10                  15

Ala Asn Ala Gln Glu Leu Ala Phe Ser Pro Pro Phe Tyr Pro Ser Pro
            20                  25                  30

Trp Ala Asp Gly Gln Gly Glu Trp Ala Asp Ala His Arg Arg Ala Val
        35                  40                  45

Glu Ile Val Ser Gln Met Thr Leu Ala Glu Lys Val Asn Leu Thr Thr
    50                  55                  60

Gly Thr Gly Trp Glu Met Asp Arg Cys Val Gly Gln Thr Gly Ser Val
65                  70                  75                  80

Pro Arg Leu Gly Ile Asn Trp Gly Leu Cys Gly Gln Asp Ser Pro Leu
                85                  90                  95

Gly Ile Arg Phe Ser Asp Leu Asn Ser Ala Phe Pro Ala Gly Thr Asn
            100                 105                 110

Val Ala Ala Thr Trp Asp Lys Thr Leu Ala Tyr Leu Arg Gly Lys Ala
        115                 120                 125

Met Gly Glu Glu Phe Asn Asp Lys Gly Val Asp Ile Leu Leu Gly Pro
    130                 135                 140

Ala Ala Gly Pro Leu Gly Lys Tyr Pro Asp Gly Gly Arg Ile Trp Glu
145                 150                 155                 160

Gly Phe Ser Pro Asp Pro Val Leu Thr Gly Val Leu Phe Ala Glu Thr
                165                 170                 175
```

Ile Lys Gly Ile Gln Asp Ala Gly Val Ile Ala Thr Ala Lys His Tyr
180 185 190

Ile Leu Asn Glu Gln Glu His Phe Arg Gln Val Gly Glu Ala Gln Gly
195 200 205

Tyr Gly Tyr Asn Ile Thr Glu Thr Ile Ser Ser Asn Val Asp Asp Lys
210 215 220

Thr Met His Glu Leu Tyr Leu Trp Pro Phe Ala Asp Ala Val Arg Ala
225 230 235 240

Gly Val Gly Ala Val Met Cys Ser Tyr Asn Gln Ile Asn Asn Ser Tyr
245 250 255

Gly Cys Gln Asn Ser Gln Thr Leu Asn Lys Leu Leu Lys Ala Glu Leu
260 265 270

Gly Phe Gln Gly Phe Val Met Ser Asp Trp Ser Ala His His Ser Gly
275 280 285

Val Gly Ala Ala Leu Ala Gly Leu Asp Met Ser Met Pro Gly Asp Ile
290 295 300

Ser Phe Asp Asp Gly Leu Ser Phe Trp Gly Thr Asn Leu Thr Val Ser
305 310 315 320

Val Leu Asn Gly Thr Val Pro Ala Trp Arg Val Asp Asp Met Ala Val
325 330 335

Arg Ile Met Thr Ala Tyr Tyr Lys Val Gly Arg Asp Arg Leu Arg Ile
340 345 350

Pro Pro Asn Phe Ser Ser Trp Thr Arg Asp Glu Tyr Gly Trp Glu His
355 360 365

Ser Ala Val Ser Glu Gly Ala Trp Thr Lys Val Asn Asp Phe Val Asn
370 375 380

Val Gln Arg Ser His Ser Gln Ile Ile Arg Glu Ile Gly Ala Ala Ser
385 390 395 400

Thr Val Leu Leu Lys Asn Thr Gly Ala Leu Pro Leu Thr Gly Lys Glu
405 410 415

Val Lys Val Gly Val Leu Gly Glu Asp Ala Gly Ser Asn Pro Trp Gly
420 425 430

```
Ala Asn Gly Cys Pro Asp Arg Gly Cys Asp Asn Gly Thr Leu Ala Met
            435                 440                 445

Ala Trp Gly Ser Gly Thr Ala Asn Phe Pro Tyr Leu Val Thr Pro Glu
    450                 455                 460

Gln Ala Ile Gln Arg Glu Val Ile Ser Asn Gly Gly Asn Val Phe Ala
465                 470                 475                 480

Val Thr Asp Asn Gly Ala Leu Ser Gln Met Ala Asp Val Ala Ser Gln
                485                 490                 495

Ser Ser Val Ser Leu Val Phe Val Asn Ala Asp Ser Gly Glu Gly Phe
            500                 505                 510

Ile Ser Val Asp Gly Asn Glu Gly Asp Arg Lys Asn Leu Thr Leu Trp
        515                 520                 525

Lys Asn Gly Glu Ala Val Ile Asp Thr Val Val Ser His Cys Asn Asn
    530                 535                 540

Thr Ile Val Val Ile His Ser Val Gly Pro Val Leu Ile Asp Arg Trp
545                 550                 555                 560

Tyr Asp Asn Pro Asn Val Thr Ala Ile Ile Trp Ala Gly Leu Pro Gly
                565                 570                 575

Gln Glu Ser Gly Asn Ser Leu Val Asp Val Leu Tyr Gly Arg Val Asn
            580                 585                 590

Pro Ser Ala Lys Thr Pro Phe Thr Trp Gly Lys Thr Arg Glu Ser Tyr
        595                 600                 605

Gly Ala Pro Leu Leu Thr Glu Pro Asn Asn Gly Asn Gly Ala Pro Gln
    610                 615                 620

Asp Asp Phe Asn Glu Gly Val Phe Ile Asp Tyr Arg His Phe Asp Lys
625                 630                 635                 640

Arg Asn Glu Thr Pro Ile Tyr Glu Phe Gly His Gly Leu Ser Tyr Thr
                645                 650                 655

Thr Phe Gly Tyr Ser His Leu Arg Val Gln Ala Leu Asn Ser Ser Ser
            660                 665                 670

Ser Ala Tyr Val Pro Thr Ser Gly Glu Thr Lys Pro Ala Pro Thr Tyr
```

```
                675                 680                 685

Gly Glu Ile Gly Ser Ala Ala Asp Tyr Leu Tyr Pro Glu Gly Leu Lys
    690                 695                 700

Arg Ile Thr Lys Phe Ile Tyr Pro Trp Leu Asn Ser Thr Asp Leu Glu
705                 710                 715                 720

Asp Ser Ser Asp Asp Pro Asn Tyr Gly Trp Glu Asp Ser Glu Tyr Ile
                725                 730                 735

Pro Glu Gly Ala Arg Asp Gly Ser Pro Gln Pro Leu Leu Lys Ala Gly
            740                 745                 750

Gly Ala Pro Gly Gly Asn Pro Thr Leu Tyr Gln Asp Leu Val Arg Val
        755                 760                 765

Ser Ala Thr Ile Thr Asn Thr Gly Asn Val Ala Gly Tyr Glu Val Pro
    770                 775                 780

Gln Leu Tyr Val Ser Leu Gly Gly Pro Asn Glu Pro Arg Val Val Leu
785                 790                 795                 800

Arg Lys Phe Asp Arg Ile Phe Leu Ala Pro Gly Glu Gln Lys Val Trp
                805                 810                 815

Thr Thr Thr Leu Asn Arg Arg Asp Leu Ala Asn Trp Asp Val Glu Ala
            820                 825                 830

Gln Asp Trp Val Ile Thr Lys Tyr Pro Lys Lys Val His Val Gly Ser
        835                 840                 845

Ser Ser Arg Lys Leu Pro Leu Arg Ala Pro Leu Pro Arg Val Tyr
    850                 855                 860
```

<210> 7
<211> 835
<212> DNA
<213> Penicillium sp.

<400> 7

```
atgctgtctt cgacgactcg caccctcgcc tttacaggcc ttgcgggcct tctgtccgct      60
cccctggtca aggcccatgg ctttgtccag ggcattgtca tcggtgacca attgtaagtc     120
cctctcttgc agttctgtcg attaactgct ggactgcttg cttgactccc tgctgactcc     180
caacagctac agcgggtaca tcgtcaactc gttcccctac gaatccaacc caccccccgt     240
catcggctgg gccacgaccg ccaccgacct gggcttcgtc gacggcacag gataccaagg     300
cccggacatc atctgccacc ggaatgcgac gcccgcgccg ctgacagccc ccgtggccgc     360
cggcggcacc gtcgagctgc agtggacgcc gtggccggac agccaccacg gacccgtcat     420
cacctacctg gcgccgtgca acggcaactg ctcgaccgtc gacaagacga cgctggagtt     480
cttcaagatc gaccagcagg gcctgatcga cgacacgagc ccgccgggca cctgggcgtc     540
ggacaacctc atcgccaaca acaatagctg gaccgtcacc attcccaaca gcgtcgcccc     600
cggcaactac gtcctgcgcc acgagatcat cgccctgcac tcggccaaca acaaggacgg     660
cgcccagaac tacccccagt gcatcaacat cgaggtcacg ggcggcggct ccgacgcgcc     720
tgagggtact ctgggcgagg atctctacca tgacaccgac ccgggcattc tggtcgacat     780
ttacgagccc attgcgacgt ataccattcc ggggccgcct gagccgacgt tctag          835
```

<210> 8
<211> 253
<212> PRT
<213> Penicillium sp.

<400> 8

```
Met Leu Ser Ser Thr Thr Arg Thr Leu Ala Phe Thr Gly Leu Ala Gly
1               5                   10                  15

Leu Leu Ser Ala Pro Leu Val Lys Ala His Gly Phe Val Gln Gly Ile
            20                  25                  30

Val Ile Gly Asp Gln Phe Tyr Ser Gly Tyr Ile Val Asn Ser Phe Pro
        35                  40                  45

Tyr Glu Ser Asn Pro Pro Pro Val Ile Gly Trp Ala Thr Thr Ala Thr
    50                  55                  60

Asp Leu Gly Phe Val Asp Gly Thr Gly Tyr Gln Gly Pro Asp Ile Ile
65                  70                  75                  80

Cys His Arg Asn Ala Thr Pro Ala Pro Leu Thr Ala Pro Val Ala Ala
                85                  90                  95

Gly Gly Thr Val Glu Leu Gln Trp Thr Pro Trp Pro Asp Ser His His
            100                 105                 110

Gly Pro Val Ile Thr Tyr Leu Ala Pro Cys Asn Gly Asn Cys Ser Thr
        115                 120                 125

Val Asp Lys Thr Thr Leu Glu Phe Phe Lys Ile Asp Gln Gln Gly Leu
    130                 135                 140

Ile Asp Asp Thr Ser Pro Pro Gly Thr Trp Ala Ser Asp Asn Leu Ile
```

145 150 155 160

Ala Asn Asn Asn Ser Trp Thr Val Thr Ile Pro Asn Ser Val Ala Pro
165 170 175

Gly Asn Tyr Val Leu Arg His Glu Ile Ile Ala Leu His Ser Ala Asn
180 185 190

Asn Lys Asp Gly Ala Gln Asn Tyr Pro Gln Cys Ile Asn Ile Glu Val
195 200 205

Thr Gly Gly Gly Ser Asp Ala Pro Glu Gly Thr Leu Gly Glu Asp Leu
210 215 220

Tyr His Asp Thr Asp Pro Gly Ile Leu Val Asp Ile Tyr Glu Pro Ile
225 230 235 240

Ala Thr Tyr Thr Ile Pro Gly Pro Pro Glu Pro Thr Phe
245 250

<210> 9
<211> 1145
<212> DNA
<213> Aspergillus fumigatus

<400> 9

atgcgtttct cccttgccgc caccgctctt ctcgctggcc tggccacggc agcgccttcg 60

agcaacaaga acaacgtcaa tcttgataag cttgctcggc gtaatggcat gctttggttc 120

ggcactgcag ccgatatccc tggtacctca gaaacaaccg acaagcctta tctgagcatc 180

ctgcgcaagc agttcggcga aatgacaccc gcaaacgcat tgaaggtgag ccagagtgat 240

agtacacctc atctcgtgtc ggcgctgacc agacgatgtt attcacatag ttcatgtata 300

ccgagcccga gcagaatgtc ttcaacttca ctcaagggga ctacttcatg gacttggccg 360

atcactatgg tcacgccgtg cgctgccata acctcgtctg ggccagccaa gtgtccgact 420

gggtcacctc caggaactgg accgccacag aactcaaaga agtgatgaag aaccacatat 480

tcaagaccgt ccaacatttt ggcaagcgct gctacgcgtg ggacgtcgtc aatgaagcta 540

ttaatgggga cgggaccttt tcctccagtg tgtggtacga cacaattggc gaggaatact 600

tctaccttgc attccagtat gcccaggaag ccctggcgca gattcacgcc aaccaggtca 660

agctttacta taacgactat ggcattgaga accccggccc caaggcagat gctgttctga 720

agctagtcgc cgagttgcgg aagcggggca ttcgcattga cggagtcggt ctcgagtccc 780

acttcatcgt cggcgagact ccttcgctgg ctgaccagct cgccaccaag aaggcttata 840

tcgaggccgg acttgaggtc gccatcaccg aacttgacgt ccgcttttct caggccccgt 900

tctacaccgc cgaggcccaa aagcagcagg ctgccgacta ctatgctagc gtcgccagtt 960

gcaagcatgc cggaccgcgc tgtgttggtg ttgtagtctg ggatttcgat gacgcctact 1020

cgtggattcc gggtaccttc gagggacagg gtggcgcctg tctatataat gagacactcg 1080

aggtgaagcc ggccttctat gctgctgccg aggcgttgga gaacaagccc tgcactgtat 1140

gctag 1145

<210> 10
<211> 364
<212> PRT
<213> Aspergillus fumigatus

<400> 10

Met Arg Phe Ser Leu Ala Ala Thr Ala Leu Leu Ala Gly Leu Ala Thr
1               5                   10                  15

Ala Ala Pro Ser Ser Asn Lys Asn Asn Val Asn Leu Asp Lys Leu Ala
            20                  25                  30

Arg Arg Asn Gly Met Leu Trp Phe Gly Thr Ala Ala Asp Ile Pro Gly
        35                  40                  45

Thr Ser Glu Thr Thr Asp Lys Pro Tyr Leu Ser Ile Leu Arg Lys Gln
    50                  55                  60

Phe Gly Glu Met Thr Pro Ala Asn Ala Leu Lys Val Ser Gln Ser Asp
65                  70                  75                  80

Phe Met Tyr Thr Glu Pro Glu Gln Asn Val Phe Asn Phe Thr Gln Gly
                85                  90                  95

Asp Tyr Phe Met Asp Leu Ala Asp His Tyr Gly His Ala Val Arg Cys
            100                 105                 110

His Asn Leu Val Trp Ala Ser Gln Val Ser Asp Trp Val Thr Ser Arg
        115                 120                 125

Asn Trp Thr Ala Thr Glu Leu Lys Glu Val Met Lys Asn His Ile Phe
    130                 135                 140

Lys Thr Val Gln His Phe Gly Lys Arg Cys Tyr Ala Trp Asp Val Val
145                 150                 155                 160

Asn Glu Ala Ile Asn Gly Asp Gly Thr Phe Ser Ser Ser Val Trp Tyr
                165                 170                 175

Asp Thr Ile Gly Glu Glu Tyr Phe Tyr Leu Ala Phe Gln Tyr Ala Gln

```
                180                 185                 190

Glu Ala Leu Ala Gln Ile His Ala Asn Gln Val Lys Leu Tyr Tyr Asn
        195                 200                 205

Asp Tyr Gly Ile Glu Asn Pro Gly Pro Lys Ala Asp Ala Val Leu Lys
    210                 215                 220

Leu Val Ala Glu Leu Arg Lys Arg Gly Ile Arg Ile Asp Gly Val Gly
225                 230                 235                 240

Leu Glu Ser His Phe Ile Val Gly Glu Thr Pro Ser Leu Ala Asp Gln
                245                 250                 255

Leu Ala Thr Lys Lys Ala Tyr Ile Glu Ala Gly Leu Glu Val Ala Ile
            260                 265                 270

Thr Glu Leu Asp Val Arg Phe Ser Gln Ala Pro Phe Tyr Thr Ala Glu
        275                 280                 285

Ala Gln Lys Gln Gln Ala Ala Asp Tyr Tyr Ala Ser Val Ala Ser Cys
    290                 295                 300

Lys His Ala Gly Pro Arg Cys Val Gly Val Val Val Trp Asp Phe Asp
305                 310                 315                 320

Asp Ala Tyr Ser Trp Ile Pro Gly Thr Phe Glu Gly Gln Gly Gly Ala
                325                 330                 335

Cys Leu Tyr Asn Glu Thr Leu Glu Val Lys Pro Ala Phe Tyr Ala Ala
            340                 345                 350

Ala Glu Ala Leu Glu Asn Lys Pro Cys Thr Val Cys
        355                 360
```

<210> 11
<211> 1400
<212> DNA
<213> Aspergillus fumigatus

<400> 11

atggtcgtcc tcagcaagct cgtcagcagc attctctttg tctccctggt ttcggcgggc 60
gtgatcgacg aacgccaggc agccggcatc aaccaggcgt ttacctccca tggcaagaag 120
tactttggca ccgccagtga ccaagctctg ctccagaagt cgcagaatga ggccattgtg 180
cgcaaagact ttggccagct gacgccggag aatagcatga agtgggatgc gactgagcgt 240
aggtctctcg gccactgtgg ctgacgttaa cttgttgaca tgactgtctg tgtagcatcg 300
caaggaagat tcaacttcgc tggtgctgat ttcctggtat gcaatctgct catctcggtc 360
gagctcctgc tgaaggacaa taaataggtc aactatgcaa aacagaatgg caagaaggtc 420
cgcggacaca ccttaggtat tcatgcgccc tcacggcatt tcgaggatac agccaagctg 480
acagtgtagt ctggcactcc caactcccgt cctgggtgtc ggctatcagc gacaaaaaca 540
ccctgacctc ggtgctgaag aaccacatca ccaccgtcat gacccggtac aagggccaga 600
tctacgcctg ggtattttgc cctctatccc acacaatgcc agccccagct aatagctgca 660
aaggacgtcg tcaacgagat cttcaacgag gacggctccc tccgcgacag cgtcttctcc 720
cgcgtgctgg gcgaggactt tgtgcggatt gccttcgaga cggcgcgctc tgtggatccc 780
tcggcgaagc tgtacatcaa cgattacaag taagcttgtg gttttgtcga gagatgtact 840
ccgtcctgga tctgaccatc acagtctcga ctcggctagc tatggcaaaa cccaggggat 900
ggtgagatat gtcaagaagt ggctggctgc gggcattcct atcgatggaa tcggtgagca 960
caggtcgcgg agctgtgtgt gatgattgta cgctgactct tcctgaaggc actcaaaccc 1020
accttggtgc gggtgcttcg tccagcgtca aaggataagt ctccttggtt ttcttgccta 1080
cgtaacgctg acccccccgtg tacagcattg actgctcttg cgtcttccgg cgtctctgag 1140
gtcgccatta ccgagctgga tatcgcgggt gcgagctccc aggactacgt caatgtatgt 1200
ctcctgattg ccagtggcag ggtcatcgat actaatagaa acaggtcgtc aaggcatgcc 1260
tggatgtccc caagtgtgtg ggaatcaccg tctgggggt gtcggacagg gactcgtggc 1320
gctccggctc gtctccgctg ctgttcgaca gcaactacca gcccaaggcg gcgtataatg 1380
ccatcattgc tgctctctga 1400

<210> 12
<211> 323
<212> PRT
<213> Aspergillus fumigatus

<400> 12

```
Met Val Val Leu Ser Lys Leu Val Ser Ser Ile Leu Phe Val Ser Leu
1               5                   10                  15

Val Ser Ala Gly Val Ile Asp Glu Arg Gln Ala Ala Gly Ile Asn Gln
            20                  25                  30

Ala Phe Thr Ser His Gly Lys Lys Tyr Phe Gly Thr Ala Ser Asp Gln
        35                  40                  45

Ala Leu Leu Gln Lys Ser Gln Asn Glu Ala Ile Val Arg Lys Asp Phe
    50                  55                  60

Gly Gln Leu Thr Pro Glu Asn Ser Met Lys Trp Asp Ala Thr Glu Ala
```

65 70 75 80

Ser Gln Gly Arg Phe Asn Phe Ala Gly Ala Asp Phe Leu Val Asn Tyr
85 90 95

Ala Lys Gln Asn Gly Lys Lys Val Arg Gly His Thr Leu Trp His Ser
100 105 110

Gln Leu Pro Ser Trp Val Ser Ala Ile Ser Asp Lys Asn Thr Leu Thr
115 120 125

Ser Val Leu Lys Asn His Ile Thr Thr Val Met Thr Arg Tyr Lys Gly
130 135 140

Gln Ile Tyr Ala Trp Asp Val Val Asn Glu Ile Phe Asn Glu Asp Gly
145 150 155 160

Ser Leu Arg Asp Ser Val Phe Ser Arg Val Leu Gly Glu Asp Phe Val
165 170 175

Arg Ile Ala Phe Glu Thr Ala Arg Ser Val Asp Pro Ser Ala Lys Leu
180 185 190

Tyr Ile Asn Asp Tyr Lys Leu Asp Ser Ala Ser Tyr Gly Lys Thr Gln
195 200 205

Gly Met Val Arg Tyr Val Lys Lys Trp Leu Ala Ala Gly Ile Pro Ile
210 215 220

Asp Gly Ile Gly Gln Thr His Leu Gly Ala Gly Ala Ser Ser Ser Val
225 230 235 240

Lys Gly Ala Leu Thr Ala Leu Ala Ser Ser Gly Val Ser Glu Val Ala
245 250 255

Ile Thr Glu Leu Asp Ile Ala Gly Ala Ser Ser Gln Asp Tyr Val Asn
260 265 270

Val Val Lys Ala Cys Leu Asp Val Pro Lys Cys Val Gly Ile Thr Val
275 280 285

Trp Gly Val Ser Asp Arg Asp Ser Trp Arg Ser Gly Ser Ser Pro Leu
290 295 300

Leu Phe Asp Ser Asn Tyr Gln Pro Lys Ala Ala Tyr Asn Ala Ile Ile
305 310 315 320

Ala Ala Leu

<210> 13
<211> 1415
<212> DNA
<213> Aspergillus fumigatus

<400> 13

```
atggtccatc tatcttcatt ggcagcagcc ctggctgctc tgcctctgta tgtttaccca        60
ctcacgagag gaggaacagc tttgacattg ctatagtgta tatggagctg gcctgaacac       120
agcagccaaa gccaaaggac taaagtactt tggttccgcc acggacaatc cagagctcac       180
ggactctgcg tatgtcgcgc aactgagcaa caccgatgat tttggtcaaa tcacacccgg       240
aaactccatg aaggtttgct tacgtctgcc tccctggagc attgcctcaa aagctaattg       300
gttgttttgt ttggatagtg ggatgccacc gagccttctc agaattcttt ttcgttcgca       360
aatggagacg ccgtggtcaa tctggcgaac aagaatggcc agctgatgcg atgccatact       420
ctggtctggc acagtcagct accgaactgg ggtatgtaaa cgtcttgtct attctcaaat       480
actctctaac agttgacagt ctctagcggg tcatggacca atgcgaccct tttggcggcc       540
atgaagaatc atatcaccaa tgtggttact cactacaagg ggaagtgcta cgcctgggat       600
gttgtcaatg aaggtttgtt gctccatcta tcctcaatag ttcttttgaa actgacaagc       660
ctgtcaatct agccctgaac gaggacggta ctttccgtaa ctctgtcttc taccagatca       720
tcggcccagc atacattcct attgcgttcg ccacggctgc tgccgcagat cccgacgtga       780
aactctacta caacgactac aacattgaat actcaggcgc caaagcgact gctgcgcaga       840
atatcgtcaa gatgatcaag gcctacggcg cgaagatcga cggcgtcggc ctccaggcac       900
actttatcgt cggcagcact ccgagtcaat cggatctgac gaccgtcttg aagggctaca       960
ctgctctcgg cgttgaggtg gcctataccg aacttgacat ccgcatgcag ctgccctcga      1020
ccgccgcaaa gctggcccag cagtccactg acttccaagg cgtggccgca gcatgcgtta      1080
gcaccactgg ctgcgtgggt gtcactatct gggactggac cgacaagtac tcctgggtcc      1140
ccagcgtgtt ccaaggctac ggcgccccat tgccttggga tgagaactat gtgaagaagc      1200
cagcgtacga tggcctgatg gcgggtcttg gagcaagcgg ctccggcacc acaacgacca      1260
ctactactac ttctactacg acaggaggta cggaccctac tggagtcgct cagaaatggg      1320
gacagtgtgg cggtattggc tggaccgggc caacaacttg tgtcagtggt accacttgcc      1380
aaaagctgaa tgactggtac tcacagtgcc tgtaa                                1415
```

<210> 14
<211> 397

<212> PRT
<213> Aspergillus fumigatus

<400> 14

```
Met Val His Leu Ser Ser Leu Ala Ala Ala Leu Ala Ala Leu Pro Leu
1               5                   10                  15

Val Tyr Gly Ala Gly Leu Asn Thr Ala Ala Lys Ala Lys Gly Leu Lys
            20                  25                  30

Tyr Phe Gly Ser Ala Thr Asp Asn Pro Glu Leu Thr Asp Ser Ala Tyr
        35                  40                  45

Val Ala Gln Leu Ser Asn Thr Asp Asp Phe Gly Gln Ile Thr Pro Gly
    50                  55                  60

Asn Ser Met Lys Trp Asp Ala Thr Glu Pro Ser Gln Asn Ser Phe Ser
65                  70                  75                  80

Phe Ala Asn Gly Asp Ala Val Val Asn Leu Ala Asn Lys Asn Gly Gln
                85                  90                  95

Leu Met Arg Cys His Thr Leu Val Trp His Ser Gln Leu Pro Asn Trp
            100                 105                 110

Val Ser Ser Gly Ser Trp Thr Asn Ala Thr Leu Leu Ala Ala Met Lys
        115                 120                 125

Asn His Ile Thr Asn Val Val Thr His Tyr Lys Gly Lys Cys Tyr Ala
    130                 135                 140

Trp Asp Val Val Asn Glu Ala Leu Asn Glu Asp Gly Thr Phe Arg Asn
145                 150                 155                 160

Ser Val Phe Tyr Gln Ile Ile Gly Pro Ala Tyr Ile Pro Ile Ala Phe
                165                 170                 175

Ala Thr Ala Ala Ala Ala Asp Pro Asp Val Lys Leu Tyr Tyr Asn Asp
            180                 185                 190

Tyr Asn Ile Glu Tyr Ser Gly Ala Lys Ala Thr Ala Ala Gln Asn Ile
        195                 200                 205

Val Lys Met Ile Lys Ala Tyr Gly Ala Lys Ile Asp Gly Val Gly Leu
    210                 215                 220

Gln Ala His Phe Ile Val Gly Ser Thr Pro Ser Gln Ser Asp Leu Thr
225                 230                 235                 240
```

```
Thr Val Leu Lys Gly Tyr Thr Ala Leu Gly Val Glu Val Ala Tyr Thr
                245                 250                 255

Glu Leu Asp Ile Arg Met Gln Leu Pro Ser Thr Ala Ala Lys Leu Ala
            260                 265                 270

Gln Gln Ser Thr Asp Phe Gln Gly Val Ala Ala Ala Cys Val Ser Thr
        275                 280                 285

Thr Gly Cys Val Gly Val Thr Ile Trp Asp Trp Thr Asp Lys Tyr Ser
    290                 295                 300

Trp Val Pro Ser Val Phe Gln Gly Tyr Gly Ala Pro Leu Pro Trp Asp
305                 310                 315                 320

Glu Asn Tyr Val Lys Lys Pro Ala Tyr Asp Gly Leu Met Ala Gly Leu
                325                 330                 335

Gly Ala Ser Gly Ser Gly Thr Thr Thr Thr Thr Thr Thr Thr Ser Thr
            340                 345                 350

Thr Thr Gly Gly Thr Asp Pro Thr Gly Val Ala Gln Lys Trp Gly Gln
        355                 360                 365

Cys Gly Gly Ile Gly Trp Thr Gly Pro Thr Thr Cys Val Ser Gly Thr
    370                 375                 380

Thr Cys Gln Lys Leu Asn Asp Trp Tyr Ser Gln Cys Leu
385                 390                 395
```

<210> 15
<211> 2376
<212> DNA
<213> Aspergillus fumigatus

<400> 15

```
atggcggttg ccaaatctat tgctgccgtg ctggtagcac tgttgcctgg tgcgcttgct        60
caggcgaata caagctatgt tgattacaat gtggaggcga atccggatct cacccctcag       120
tcggtcgcta cgattgacct gtcctttccc gactgcgaga atggaccgct cagcaagact       180
ctcgtttgcg acacgtcggc tcggccgcat gaccgagctg ctgccctggt ttccatgttc       240
accttcgagg agctggtgaa caacacaggc aacactagcc ctggtgttcc aagacttggt       300
ctccctccgt accaagtatg gagcgaggct ctccatggac ttgaccgcgc caacttcaca       360
aacgagggag agtacagctg ggccacctcg ttccccatgc ctatcctgac aatgtcggcc       420
ttgaaccgaa ccctgatcaa ccagatcgcg accatcatcg caactcaagg acgagctttc       480
aataacgttg ggcggtatgg gctggacgtg tacgccccga atataaatgc attcagatcg       540
```

gctatgtggg gaagaggtca agagaccccc ggagaagacg cttactgcct ggcatcggcg 600
tatgcgtacg agtatatcac tggcatccag ggtggtgttg atccggaaca cctcaagttg 660
gtggccactg ccaaacacta tgcgggctac gatcttgaga actgggacgg tcactcccgt 720
ttgggcaacg atatgaacat tacacagcag gaactttccg aatactacac ccctcagttc 780
cttgttgcag ccagagacgc caaagtgcac agtgtcatgt gctcctacaa cgcggtaaat 840
ggggtgccca gctgcgcaaa ctcgttcttc ctccagaccc tcctccgtga cacattcggc 900
ttcgtcgagg atggttatgt atccagcgac tgcgactcgg cgtacaatgt ctggaacccg 960
cacgagtttg cggccaacat cacgggggcc gctgcagact ctatccgggc ggggacggac 1020
attgattgcg gcactactta tcaatactat ttcggcgaag cctttgacga gcaagaggtc 1080
acccgtgcag aaatcgaaag aggtgtgatc cgcctgtaca gcaacttggt gcgtctcggc 1140
tatttcgatg gcaatggaag cgtgtatcgg gacctgacgt ggaatgatgt cgtgaccacg 1200
gatgcctgga atatctcata cgaagccgct gtagaaggca ttgtcctact gaagaacgat 1260
ggaaccttgc ctctcgccaa gtcggtccgc agtgttgcat tgattgggcc ctggatgaat 1320
gtgacgactc agcttcaggg caactacttt ggaccggcgc cttatctgat tagtccgttg 1380
aatgccttcc agaattctga cttcgacgtg aactacgctt tcggcacgaa catttcatcc 1440
cactccacag atgggttttc cgaggcgttg tctgctgcga agaaatccga cgtcatcata 1500
ttcgcgggcg ggattgacaa cactttggaa gcagaagcca tggatcgcat gaatatcaca 1560
tggcccggca atcagctaca gctcatcgac cagttgagcc aactcggcaa accgctgatc 1620
gtcctccaga tgggcggcgg ccaagtcgac tcctcctcgc tcaagtccaa caagaatgtc 1680
aactccctga tctggggtgg ataccccgga caatccggcg ggcaggctct cctagacatc 1740
atcaccggca agcgcgcccc cgccggccga ctcgtggtca cgcagtaccc ggccgaatac 1800
gcaacccagt tccccgccac cgacatgagc ctgcggcctc acggcaataa tcccggccag 1860
acctacatgt ggtacaccgg caccccgtc tacgagtttg gccacgggct cttctacacg 1920
accttccacg cctccctccc tggcaccggc aaggacaaga cctccttcaa catccaagac 1980
ctcctcacgc agccgcatcc gggcttcgca aacgtcgagc aaatgccttt gctcaacttc 2040
accgtgacga tcaccaatac cggcaaggtc gcttccgact acactgctat gctcttcgcg 2100
aacaccaccg cgggacctgc tccatacccg aacaagtggc tcgtcggctt cgaccggctg 2160
gcgagcctgg aaccgcacag gtcgcagact atgaccatcc ccgtgactat cgacagcgtg 2220
gctcgtacgg atgaggccgg caatcgggtt ctctacccgg gaaagtacga gttggccctg 2280
aacaatgagc ggtcggttgt ccttcagttt gtgctgacag gccgagaggc tgtgattttc 2340
aagtggcctg tagagcagca gcagatttcg tctgcg 2376

<210> 16
<211> 792
<212> PRT
<213> Aspergillus fumigatus

<400> 16

```
Met Ala Val Ala Lys Ser Ile Ala Ala Val Leu Val Ala Leu Leu Pro
1               5                   10                  15

Gly Ala Leu Ala Gln Ala Asn Thr Ser Tyr Val Asp Tyr Asn Val Glu
            20                  25                  30

Ala Asn Pro Asp Leu Thr Pro Gln Ser Val Ala Thr Ile Asp Leu Ser
        35                  40                  45

Phe Pro Asp Cys Glu Asn Gly Pro Leu Ser Lys Thr Leu Val Cys Asp
    50                  55                  60

Thr Ser Ala Arg Pro His Asp Arg Ala Ala Ala Leu Val Ser Met Phe
65                  70                  75                  80

Thr Phe Glu Glu Leu Val Asn Asn Thr Gly Asn Thr Ser Pro Gly Val
                85                  90                  95

Pro Arg Leu Gly Leu Pro Pro Tyr Gln Val Trp Ser Glu Ala Leu His
            100                 105                 110

Gly Leu Asp Arg Ala Asn Phe Thr Asn Glu Gly Glu Tyr Ser Trp Ala
        115                 120                 125

Thr Ser Phe Pro Met Pro Ile Leu Thr Met Ser Ala Leu Asn Arg Thr
    130                 135                 140

Leu Ile Asn Gln Ile Ala Thr Ile Ile Ala Thr Gln Gly Arg Ala Phe
145                 150                 155                 160

Asn Asn Val Gly Arg Tyr Gly Leu Asp Val Tyr Ala Pro Asn Ile Asn
                165                 170                 175

Ala Phe Arg Ser Ala Met Trp Gly Arg Gly Gln Glu Thr Pro Gly Glu
            180                 185                 190

Asp Ala Tyr Cys Leu Ala Ser Ala Tyr Ala Tyr Glu Tyr Ile Thr Gly
        195                 200                 205

Ile Gln Gly Gly Val Asp Pro Glu His Leu Lys Leu Val Ala Thr Ala
    210                 215                 220
```

Lys His Tyr Ala Gly Tyr Asp Leu Glu Asn Trp Asp Gly His Ser Arg
225 230 235 240

Leu Gly Asn Asp Met Asn Ile Thr Gln Gln Glu Leu Ser Glu Tyr Tyr
245 250 255

Thr Pro Gln Phe Leu Val Ala Ala Arg Asp Ala Lys Val His Ser Val
260 265 270

Met Cys Ser Tyr Asn Ala Val Asn Gly Val Pro Ser Cys Ala Asn Ser
275 280 285

Phe Phe Leu Gln Thr Leu Leu Arg Asp Thr Phe Gly Phe Val Glu Asp
290 295 300

Gly Tyr Val Ser Ser Asp Cys Asp Ser Ala Tyr Asn Val Trp Asn Pro
305 310 315 320

His Glu Phe Ala Ala Asn Ile Thr Gly Ala Ala Ala Asp Ser Ile Arg
325 330 335

Ala Gly Thr Asp Ile Asp Cys Gly Thr Thr Tyr Gln Tyr Tyr Phe Gly
340 345 350

Glu Ala Phe Asp Glu Gln Glu Val Thr Arg Ala Glu Ile Glu Arg Gly
355 360 365

Val Ile Arg Leu Tyr Ser Asn Leu Val Arg Leu Gly Tyr Phe Asp Gly
370 375 380

Asn Gly Ser Val Tyr Arg Asp Leu Thr Trp Asn Asp Val Val Thr Thr
385 390 395 400

Asp Ala Trp Asn Ile Ser Tyr Glu Ala Ala Val Glu Gly Ile Val Leu
405 410 415

Leu Lys Asn Asp Gly Thr Leu Pro Leu Ala Lys Ser Val Arg Ser Val
420 425 430

Ala Leu Ile Gly Pro Trp Met Asn Val Thr Thr Gln Leu Gln Gly Asn
435 440 445

Tyr Phe Gly Pro Ala Pro Tyr Leu Ile Ser Pro Leu Asn Ala Phe Gln
450 455 460

Asn Ser Asp Phe Asp Val Asn Tyr Ala Phe Gly Thr Asn Ile Ser Ser
465 470 475 480

His Ser Thr Asp Gly Phe Ser Glu Ala Leu Ser Ala Ala Lys Lys Ser
485 490 495

Asp Val Ile Ile Phe Ala Gly Gly Ile Asp Asn Thr Leu Glu Ala Glu
500 505 510

Ala Met Asp Arg Met Asn Ile Thr Trp Pro Gly Asn Gln Leu Gln Leu
515 520 525

Ile Asp Gln Leu Ser Gln Leu Gly Lys Pro Leu Ile Val Leu Gln Met
530 535 540

Gly Gly Gly Gln Val Asp Ser Ser Ser Leu Lys Ser Asn Lys Asn Val
545 550 555 560

Asn Ser Leu Ile Trp Gly Gly Tyr Pro Gly Gln Ser Gly Gly Gln Ala
565 570 575

Leu Leu Asp Ile Ile Thr Gly Lys Arg Ala Pro Ala Gly Arg Leu Val
580 585 590

Val Thr Gln Tyr Pro Ala Glu Tyr Ala Thr Gln Phe Pro Ala Thr Asp
595 600 605

Met Ser Leu Arg Pro His Gly Asn Asn Pro Gly Gln Thr Tyr Met Trp
610 615 620

Tyr Thr Gly Thr Pro Val Tyr Glu Phe Gly His Gly Leu Phe Tyr Thr
625 630 635 640

Thr Phe His Ala Ser Leu Pro Gly Thr Gly Lys Asp Lys Thr Ser Phe
645 650 655

Asn Ile Gln Asp Leu Leu Thr Gln Pro His Pro Gly Phe Ala Asn Val
660 665 670

Glu Gln Met Pro Leu Leu Asn Phe Thr Val Thr Ile Thr Asn Thr Gly
675 680 685

Lys Val Ala Ser Asp Tyr Thr Ala Met Leu Phe Ala Asn Thr Thr Ala
690 695 700

Gly Pro Ala Pro Tyr Pro Asn Lys Trp Leu Val Gly Phe Asp Arg Leu
705 710 715 720

Ala Ser Leu Glu Pro His Arg Ser Gln Thr Met Thr Ile Pro Val Thr

725 730 735

Ile Asp Ser Val Ala Arg Thr Asp Glu Ala Gly Asn Arg Val Leu Tyr
740 745 750

Pro Gly Lys Tyr Glu Leu Ala Leu Asn Asn Glu Arg Ser Val Val Leu
755 760 765

Gln Phe Val Leu Thr Gly Arg Glu Ala Val Ile Phe Lys Trp Pro Val
770 775 780

Glu Gln Gln Gln Ile Ser Ser Ala
785 790

<210> 17
<211> 299
<212> PRT
<213> Thielavia terrestris

<400> 17

```
Met Arg Ser Thr Pro Val Leu Arg Thr Thr Leu Ala Ala Ala Leu Pro
1               5                   10                  15

Leu Val Ala Ser Ala Ala Ser Gly Ser Gly Gln Ser Thr Arg Tyr Trp
            20                  25                  30

Asp Cys Cys Lys Pro Ser Cys Ala Trp Pro Gly Lys Ala Ala Val Ser
        35                  40                  45

Gln Pro Val Tyr Ala Cys Asp Ala Asn Phe Gln Arg Leu Ser Asp Phe
    50                  55                  60

Asn Val Gln Ser Gly Cys Asn Gly Gly Ser Ala Tyr Ser Cys Ala Asp
65                  70                  75                  80

Gln Thr Pro Trp Ala Val Asn Asp Asn Leu Ala Tyr Gly Phe Ala Ala
                85                  90                  95

Thr Ser Ile Ala Gly Gly Ser Glu Ser Ser Trp Cys Cys Ala Cys Tyr
            100                 105                 110

Ala Leu Thr Phe Thr Ser Gly Pro Val Ala Gly Lys Thr Met Val Val
        115                 120                 125

Gln Ser Thr Ser Thr Gly Gly Asp Leu Gly Ser Asn His Phe Asp Ile
    130                 135                 140

Ala Met Pro Gly Gly Gly Val Gly Ile Phe Asn Gly Cys Ser Ser Gln
```

145 150 155 160

Phe Gly Gly Leu Pro Gly Ala Gln Tyr Gly Gly Ile Ser Ser Arg Asp
165 170 175

Gln Cys Asp Ser Phe Pro Ala Pro Leu Lys Pro Gly Cys Gln Trp Arg
180 185 190

Phe Asp Trp Phe Gln Asn Ala Asp Asn Pro Thr Phe Thr Phe Gln Gln
195 200 205

Val Gln Cys Pro Ala Glu Ile Val Ala Arg Ser Gly Cys Lys Arg Asn
210 215 220

Asp Asp Ser Ser Phe Pro Val Phe Thr Pro Pro Ser Gly Gly Asn Gly
225 230 235 240

Gly Thr Gly Thr Pro Thr Ser Thr Ala Pro Gly Ser Gly Gln Thr Ser
245 250 255

Pro Gly Gly Gly Ser Gly Cys Thr Ser Gln Lys Trp Ala Gln Cys Gly
260 265 270

Gly Ile Gly Phe Ser Gly Cys Thr Thr Cys Val Ser Gly Thr Thr Cys
275 280 285

Gln Lys Leu Asn Asp Tyr Tyr Ser Gln Cys Leu
290 295

<210> 18
<211> 773
<212> PRT
<213> Bacillus sp.

<400> 18

Ala Glu Gly Asn Thr Arg Glu Asp Asn Phe Lys His Leu Leu Gly Asn
1 5 10 15

Asp Asn Val Lys Arg Pro Ser Glu Ala Gly Ala Leu Gln Leu Gln Glu
20 25 30

Val Asp Gly Gln Met Thr Leu Val Asp Gln His Gly Glu Lys Ile Gln
35 40 45

Leu Arg Gly Met Ser Thr His Gly Leu Gln Trp Phe Pro Glu Ile Leu
50 55 60

Asn Asp Asn Ala Tyr Lys Ala Leu Ala Asn Asp Trp Glu Ser Asn Met
65 70 75 80

Ile Arg Leu Ala Met Tyr Val Gly Glu Asn Gly Tyr Ala Ser Asn Pro
85 90 95

Glu Leu Ile Lys Ser Arg Val Ile Lys Gly Ile Asp Leu Ala Ile Glu
100 105 110

Asn Asp Met Tyr Val Ile Val Asp Trp His Val His Ala Pro Gly Asp
115 120 125

Pro Arg Asp Pro Val Tyr Ala Gly Ala Glu Asp Phe Phe Arg Asp Ile
130 135 140

Ala Ala Leu Tyr Pro Asn Asn Pro His Ile Ile Tyr Glu Leu Ala Asn
145 150 155 160

Glu Pro Ser Ser Asn Asn Asn Gly Gly Ala Gly Ile Pro Asn Asn Glu
165 170 175

Glu Gly Trp Asn Ala Val Lys Glu Tyr Ala Asp Pro Ile Val Glu Met
180 185 190

Leu Arg Asp Ser Gly Asn Ala Asp Asp Asn Ile Ile Ile Val Gly Ser
195 200 205

Pro Asn Trp Ser Gln Arg Pro Asp Leu Ala Ala Asp Asn Pro Ile Asn
210 215 220

Asp His His Thr Met Tyr Thr Val His Phe Tyr Thr Gly Ser His Ala
225 230 235 240

Ala Ser Thr Glu Ser Tyr Pro Pro Glu Thr Pro Asn Ser Glu Arg Gly
245 250 255

Asn Val Met Ser Asn Thr Arg Tyr Ala Leu Glu Asn Gly Val Ala Val
260 265 270

Phe Ala Thr Glu Trp Gly Thr Ser Gln Ala Asn Gly Asp Gly Gly Pro
275 280 285

Tyr Phe Asp Glu Ala Asp Val Trp Ile Glu Phe Leu Asn Glu Asn Asn
290 295 300

Ile Ser Trp Ala Asn Trp Ser Leu Thr Asn Lys Asn Glu Val Ser Gly
305 310 315 320

Ala Phe Thr Pro Phe Glu Leu Gly Lys Ser Asn Ala Thr Asn Leu Asp
325 330 335

Pro Gly Pro Asp His Val Trp Ala Pro Glu Glu Leu Ser Leu Ser Gly
340 345 350

Glu Tyr Val Arg Ala Arg Ile Lys Gly Val Asn Tyr Glu Pro Ile Asp
355 360 365

Arg Thr Lys Tyr Thr Lys Val Leu Trp Asp Phe Asn Asp Gly Thr Lys
370 375 380

Gln Gly Phe Gly Val Asn Ser Asp Ser Pro Asn Lys Glu Leu Ile Ala
385 390 395 400

Val Asp Asn Glu Asn Asn Thr Leu Lys Val Ser Gly Leu Asp Val Ser
405 410 415

Asn Asp Val Ser Asp Gly Asn Phe Trp Ala Asn Ala Arg Leu Ser Ala
420 425 430

Asp Gly Trp Gly Lys Ser Val Asp Ile Leu Gly Ala Glu Lys Leu Thr
435 440 445

Met Asp Val Ile Val Asp Glu Pro Thr Thr Val Ala Ile Ala Ala Ile
450 455 460

Pro Gln Ser Ser Lys Ser Gly Trp Ala Asn Pro Glu Arg Ala Val Arg
465 470 475 480

Val Asn Ala Glu Asp Phe Val Gln Gln Thr Asp Gly Lys Tyr Lys Ala
485 490 495

Gly Leu Thr Ile Thr Gly Glu Asp Ala Pro Asn Leu Lys Asn Ile Ala
500 505 510

Phe His Glu Glu Asp Asn Asn Met Asn Asn Ile Ile Leu Phe Val Gly
515 520 525

Thr Asp Ala Ala Asp Val Ile Tyr Leu Asp Asn Ile Lys Val Ile Gly
530 535 540

Thr Glu Val Glu Ile Pro Val Val His Asp Pro Lys Gly Glu Ala Val
545 550 555 560

Leu Pro Ser Val Phe Glu Asp Gly Thr Arg Gln Gly Trp Asp Trp Ala
565 570 575

```
Gly Glu Ser Gly Val Lys Thr Ala Leu Thr Ile Glu Glu Ala Asn Gly
            580                 585                 590

Ser Asn Ala Leu Ser Trp Glu Phe Gly Tyr Pro Glu Val Lys Pro Ser
        595                 600                 605

Asp Asn Trp Ala Thr Ala Pro Arg Leu Asp Phe Trp Lys Ser Asp Leu
    610                 615                 620

Val Arg Gly Glu Asn Asp Tyr Val Ala Phe Asp Phe Tyr Leu Asp Pro
625                 630                 635                 640

Val Arg Ala Thr Glu Gly Ala Met Asn Ile Asn Leu Val Phe Gln Pro
                645                 650                 655

Pro Thr Asn Gly Tyr Trp Val Gln Ala Pro Lys Thr Tyr Thr Ile Asn
            660                 665                 670

Phe Asp Glu Leu Glu Glu Ala Asn Gln Val Asn Gly Leu Tyr His Tyr
        675                 680                 685

Glu Val Lys Ile Asn Val Arg Asp Ile Thr Asn Ile Gln Asp Asp Thr
    690                 695                 700

Leu Leu Arg Asn Met Met Ile Ile Phe Ala Asp Val Glu Ser Asp Phe
705                 710                 715                 720

Ala Gly Arg Val Phe Val Asp Asn Val Arg Phe Glu Gly Ala Ala Thr
                725                 730                 735

Thr Glu Pro Val Glu Pro Glu Pro Val Asp Pro Gly Glu Glu Thr Pro
            740                 745                 750

Pro Val Asp Glu Lys Glu Ala Lys Lys Glu Gln Lys Glu Ala Glu Lys
        755                 760                 765

Glu Glu Lys Glu Glu
    770
```

## Description

## FIELD OF THE INVENTION

The present invention concerns a laundry detergent composition comprising an enzyme capable for degrading cellulosic material. The invention further concerns a washing method, the use of enzymes capable for degrading cellulosic material for washing and a method for cleaning washing machines.

## Reference to a Sequence Listing

This application contains a Sequence Listing in computer readable form.

## BACKGROUND OF THE INVENTION

Use of enzymes in laundry detergents is well known. Also use of enzymes capable for degrading cellulosic material is known for laundry purpose. However, cellulose degrading enzymes for laundry should be selected carefully as laundry textile serve as substrate for the enzymes.

The degradation of cellulosic material in washing machines is often a challenge. Cellulosic fibers may be cleaved from textile during wash with enzymes capable for degrading cellulosic material and tend to clog filters, pipes and drains in washing machines. The drains and filters thus need to be cleaned manually from time to time.

Wearing, washing and tumble drying of fabric and textile exposes the textile to mechanical stress which damages the textile and fabric by breaking the fibers in the fabric/textile and thereby causing the textile/fabric to be covered with fuzz and pills. This gives the fabric or textile a worn look.

WO 2013/028928 A1 discloses celluloytic enzyme compositions and uses thereof.

WO 2014/026630 A1 discloses methods for treating textiles with endoglucanases.

## SUMMARY OF THE INVENTION

The present invention concerns use of a laundry composition comprising one or more enzymes capable of degrading cellulosic material, a method for laundering a textile comprising contacting the textile to one or more enzymes capable of degrading cellulosic material and a cleaning method for cleaning the interior of an automated washing machine.

## Definitions

Anti-pilling: The term "anti-pilling" denotes removal of pills from the textile surface and/or prevention of formation of pills on the textile surface.

Beta-glucosidase: The term "beta-glucosidase" means a beta-D-glucoside glucohydrolase (E.C. 3.2.1.21) that catalyzes the hydrolysis of terminal non-reducing beta-D-glucose residues with the release of beta-D-glucose. For purposes of the present invention, beta-glucosidase activity is determined using p-nitrophenyl-beta-D-glucopyranoside as substrate according to the procedure of Venturi et al., 2002, Extracellular beta-D-glucosidase from Chaetomium thermophilum var. coprophilum: production, purification and some biochemical properties, J. Basic Microbiol. 42: 55-66. One unit of beta-glucosidase is defined as 1.0 μmole of *p*-nitrophenolate anion produced per minute at 25°C, pH 4.8 from 1 mM *p*-nitrophenyl-beta-D-glucopyranoside as substrate in 50 mM sodium citrate containing 0.01% TWEEN® 20 (polyoxyethylene sorbitan monolaurate).

Beta-xylosidase: The term "beta-xylosidase" means a beta-D-xyloside xylohydrolase (E.C. 3.2.1.37) that catalyzes the exo-hydrolysis of short beta (1→4)-xylooligosaccharides to remove successive D-xylose residues from non-reducing termini. For purposes of the present invention, one unit of beta-xylosidase is defined as 1.0 μmole of *p*-nitrophenolate anion produced per minute at 40°C, pH 5 from 1 mM *p*-nitrophenyl-beta-D-xyloside as substrate in 100 mM sodium citrate containing 0.01% TWEEN® 20.

Cellobiohydrolase: The term "cellobiohydrolase" means a 1,4-beta-D-glucan cellobiohydrolase (E.C. 3.2.1.91 and E.C. 3.2.1.176) that catalyzes the hydrolysis of 1,4-beta-D-glucosidic linkages in cellulose, cellooligosaccharides, or any beta-1,4-linked glucose containing polymer, releasing cellobiose from the reducing or non-reducing ends of the chain (Teeri, 1997, Crystalline cellulose degradation: New insight into the function of cellobiohydrolases, Trends in Biotechnology 15: 160-167; Teeri et al., 1998, Trichoderma reesei cellobiohydrolases: why so efficient on crystalline cellulose?, Biochem. Soc. Trans. 26: 173-178). Cellobiohydrolase activity is determined according to the procedures described by Lever et al., 1972, Anal. Biochem. 47: 273-279; van Tilbeurgh et al., 1982, FEBS Letters, 149: 152-156; van Tilbeurgh and Claeyssens, 1985, FEBS Letters, 187: 283-288; and Tomme et al., 1988, Eur. J. Biochem. 170: 575-581. In the present invention, the Tomme *et al*. method can be used to determine cellobiohydrolase activity.

Cellulolytic enzyme or cellulase: The term "cellulolytic enzyme" or "cellulase" means one or more (*e.g.*, several) enzymes that hydrolyze a cellulosic material. Such enzymes include endoglucanase(s), cellobiohydrolase(s), beta-glucosidase(s), or combinations thereof. The two basic approaches for measuring cellulolytic activity include: (1) measuring the total cellulolytic activity, and (2) measuring the individual cellulolytic activities (endoglucanases, cellobiohydrolases, and beta-glucosidases) as reviewed in Zhang et al., Outlook for cellulase improvement: Screening and selection strategies, 2006, Biotechnology Advances 24: 452-481. Total cellulolytic activity is usually measured using insoluble substrates, including Whatman №1 filter paper, microcrystalline cellulose, bacterial cellulose, algal cellulose, cotton, pretreated lignocellulose, *etc.* The most common total cellulolytic activity assay is the filter paper assay using Whatman №1 filter paper as the substrate. The assay was established by the International Union of Pure and Applied Chemistry (IUPAC) (Ghose, 1987, Measurement of cellulase activities, Pure Appl. Chem. 59: 257-68).

For purposes of the present invention, cellulolytic enzyme activity is determined by measuring the increase in hydrolysis of a cellulosic material by cellulolytic enzyme(s) under the following conditions: 1-50 mg of cellulolytic enzyme protein/g of cellulose in PCS (or other pretreated cellulosic material) for 3-7 days at a suitable temperature, *e.g.*, 50°C, 55°C, or 60°C, compared to a control hydrolysis without addition of cellulolytic enzyme protein. Typical conditions are 1 ml reactions, washed or unwashed PCS, 5% insoluble solids, 50 mM sodium acetate pH 5, 1 mM $MnSO_4$, 50°C, 55°C, or 60°C, 72 hours, sugar analysis by AMINEX® HPX-87H column (Bio-Rad Laboratories, Inc., Hercules, CA, USA).

Cellulosic material: The term "cellulosic material" means any material containing cellulose. The predominant polysaccharide in the primary cell wall of biomass is cellulose, the second most abundant is hemicellulose, and the third is pectin. The secondary cell wall, produced after the cell has stopped growing, also contains polysaccharides and is strengthened by polymeric lignin covalently cross-linked to hemicellulose. Cellulose is a homopolymer of anhydrocellobiose and thus a linear beta-(1-4)-D-glucan, while hemicelluloses include a variety of compounds, such as xylans, xyloglucans, arabinoxylans, and mannans in complex branched structures with a spectrum of substituents. Although generally polymorphous, cellulose is found in plant tissue primarily as an insoluble crystalline matrix of parallel glucan chains. Hemicelluloses usually hydrogen bond to cellulose, as well as to other hemicelluloses, which help stabilize the cell wall matrix.

Cellulose is generally found, for example, in vegetable food products, such as salad, tomatoes, spinach, cabbage, grain or the like.

Detergent component: The term "detergent component" is defined herein to mean the types of chemicals which can be used in detergent compositions for laundry. Examples of detergent components are surfactants, builders, chelators or chelating agents, bleach system or bleach components, polymers, fabric conditioners, foam boosters, suds suppressors, dyes, perfume, tannish inhibitors, bactericides, fungicides, soil suspending agents, anti-corrosion agents, enzyme inhibitors or stabilizers, enzyme activators, transferase(s), hydrolytic enzymes, oxido reductases, blueing agents and fluorescent dyes, antioxidants, and solubilizers.

Detergent Composition: The term "detergent composition" refers to compositions that find use in the removal of undesired compounds from surfaces to be cleaned, such as textile surfaces. The detergent composition may be used to e.g. clean textiles for both household cleaning and industrial cleaning. The terms encompass any materials/compounds selected for the particular type of cleaning composition desired and the form of the product (e.g., liquid, gel, powder, granulate, paste, or spray compositions) and includes, but is not limited to, detergent compositions (e.g., liquid and/or solid laundry detergents and fine fabric detergents; fabric fresheners; fabric softeners; and textile and laundry pre-spotters/pretreatment). The detergent composition may contain one or more enzymes such as hemicellulases, peroxidases, proteases, cellulases, xylanases, lipases, phospholipases, esterases, cutinases, pectinases, mannanases, pectate lyases, keratinases, reductases, oxidases, phenoloxidases, lipoxygenases, ligninases, pullulanases, tannases, pentosanases, malanases, beta-glucanases, arabinosidases, hyaluronidase, chondroitinase, laccase, DNase, chlorophyllases, amylases, perhydrolases, peroxidases, xanthanase and mixtures thereof. The detergent composition may further comprise detergent component such as surfactants, builders, chelators or chelating agents, bleach system or bleach components, polymers, fabric conditioners, foam boosters, suds suppressors, dyes, perfume, tannish inhibitors, bactericides, fungicides, soil suspending agents, anti-corrosion agents, enzyme inhibitors or stabilizers, enzyme activators, transferase(s), hydrolytic enzymes, oxido reductases, blueing agents and fluorescent dyes, antioxidants, and solubilizers.

Endoglucanase: The term "endoglucanase" means an endo-1,4-(1,3;1,4)-beta-D-glucan 4-glucanohydrolase (E.C. 3.2.1.4) that catalyzes endohydrolysis of 1,4-beta-D-glycosidic linkages in cellulose, cellulose derivatives (such as carboxymethyl cellulose and hydroxyethyl cellulose), lichenin, beta-1,4 bonds in mixed beta-1,3 glucans such as cereal beta-D-glucans or xyloglucans, and other plant material containing cellulosic components. Endoglucanase activity can be determined by measuring reduction in substrate viscosity or increase in reducing ends determined by a reducing sugar assay (Zhang et al., 2006, Biotechnology Advances 24: 452-481). For purposes of the present invention, endoglucanase activity is determined using carboxymethyl cellulose (CMC) as substrate according to the procedure of Ghose, 1987, Pure and Appl. Chem. 59: 257-268, at pH 5, 40°C.

Fabric softener: A Fabric softener (also called fabric conditioner) is a composition that is typically applied to

laundry during the rinse cycle in a washing machine or when washing by hand. Fabric softeners are available as solutions and solids, and may also be impregnated in dryer sheets used in a clothes dryer.

Fabric softener agent: A fabric softener agent is an ingredient that is comprised in fabric softener compositions such as chemical compounds that are electrically charged. These compounds causes threads in the fabric to lift up from the surface of the textile and thereby gives the fabric a softer feel of the textile. In one embodiment the fabric softener agent is one ore more cationic softeners. The cationic softeners bind by electrostatic attraction to the negatively charged groups on the surface of the textile and neutralize their charge and thereby impart lubricity.

Family 61 glycoside hydrolase: The term "Family 61 glycoside hydrolase" or "Family GH61" or "GH61" means a polypeptide falling into the glycoside hydrolase Family 61 according to Henrissat B., 1991, A classification of glycosyl hydrolases based on amino-acid sequence similarities, Biochem. J. 280: 309-316, and Henrissat B., and Bairoch A., 1996, Updating the sequence-based classification of glycosyl hydrolases, Biochem. J. 316: 695-696. The enzymes in this family were originally classified as a glycoside hydrolase family based on measurement of very weak endo-1,4-beta-D-glucanase activity in one family member. The structure and mode of action of these enzymes are non-canonical and they cannot be considered as bona fide glycosidases. However, they are kept in the CAZy classification on the basis of their capacity to enhance the breakdown of lignocellulose when used in conjunction with a cellulase or a mixture of cellulases.

Fragment: The term "fragment" means a polypeptide having one or more (*e.g.*, several) amino acids absent from the amino and/or carboxyl terminus of a mature polypeptide main; wherein the fragment has enzyme activity. In one aspect, a fragment contains at least 85%, *e.g.*, at least 90% or at least 95% of the amino acid residues of the mature polypeptide of an enzyme.

Hemicellulolytic enzyme or hemicellulase: The term "hemicellulolytic enzyme" or "hemicellulase" means one or more (*e.g.*, several) enzymes that hydrolyze a hemicellulosic material. See, for example, Shallom, D. and Shoham, Y. Microbial hemicellulases. Current Opinion In Microbiology, 2003, 6(3): 219-228). Hemicellulases are key components in the degradation of plant biomass. Examples of hemicellulases include, but are not limited to, an acetylmannan esterase, an acetylxylan esterase, an arabinanase, an arabinofuranosidase, a coumaric acid esterase, a feruloyl esterase, a galactosidase, a glucuronidase, a glucuronoyl esterase, a mannanase, a mannosidase, a xylanase, and a xylosidase. The substrates of these enzymes, the hemicelluloses, are a heterogeneous group of branched and linear polysaccharides that are bound via hydrogen bonds to the cellulose microfibrils in the plant cell wall, crosslinking them into a robust network. Hemicelluloses are also covalently attached to lignin, forming together with cellulose a highly complex structure. The variable structure and organization of hemicelluloses require the concerted action of many enzymes for its complete degradation. The catalytic modules of hemicellulases are either glycoside hydrolases (GHs) that hydrolyze glycosidic bonds, or carbohydrate esterases (CEs), which hydrolyze ester linkages of acetate or ferulic acid side groups. These catalytic modules, based on homology of their primary sequence, can be assigned into GH and CE families. Some families, with an overall similar fold, can be further grouped into clans, marked alphabetically (*e.g.*, GH-A). A most informative and updated classification of these and other carbohydrate active enzymes is available in the Carbohydrate-Active Enzymes (CAZy) database. Hemicellulolytic enzyme activities can be measured according to Ghose and Bisaria, 1987, Pure & Appl. Chem. 59: 1739-1752, at a suitable temperature, *e.g.*, 50°C, 55°C, or 60°C, and pH, *e.g.*, 5.0 or 5.5.

High stringency conditions: The term "high stringency conditions" means for probes of at least 100 nucleotides in length, prehybridization and hybridization at 42°C in 5X SSPE, 0.3% SDS, 200 micrograms/ml sheared and denatured salmon sperm DNA, and 50% formamide, following standard Southern blotting procedures for 12 to 24 hours. The carrier material is finally washed three times each for 15 minutes using 2X SSC, 0.2% SDS at 65°C.

Improved wash performance: The term "improved wash performance" is defined herein as a laundry detergent composition displaying an increased wash performance relative to the wash performance of a laundry detergent composition without the inventive enzyme preparation, e.g. by increased soil removal.

Isolated: The term "isolated" means a substance in a form or environment that does not occur in nature. Non-limiting examples of isolated substances include (1) any non-naturally occurring substance, (2) any substance including, but not limited to, any enzyme, variant, nucleic acid, protein, peptide or cofactor, that is at least partially removed from one or more or all of the naturally occurring constituents with which it is associated in nature; (3) any substance modified by the hand of man relative to that substance found in nature; or (4) any substance modified by increasing the amount of the substance relative to other components with which it is naturally associated (*e.g.*, recombinant production in a host cell; multiple copies of a gene encoding the substance; and use of a stronger promoter than the promoter naturally associated with the gene encoding the substance).

Low stringency conditions: The term "low stringency conditions" means for probes of at least 100 nucleotides in length, prehybridization and hybridization at 42°C in 5X SSPE, 0.3% SDS, 200 micrograms/ml sheared and denatured salmon sperm DNA, and 25% formamide, following standard Southern blotting procedures for 12 to 24 hours. The carrier material is finally washed three times each for 15 minutes using 2X SSC, 0.2% SDS at 50°C.

Medium stringency conditions: The term "medium stringency conditions" means for probes of at least 100 nucleotides in length, prehybridization and hybridization at 42°C in 5X SSPE, 0.3% SDS, 200 micrograms/ml sheared

and denatured salmon sperm DNA, and 35% formamide, following standard Southern blotting procedures for 12 to 24 hours. The carrier material is finally washed three times each for 15 minutes using 2X SSC, 0.2% SDS at 55°C.

Medium-high stringency conditions: The term "medium-high stringency conditions" means for probes of at least 100 nucleotides in length, prehybridization and hybridization at 42°C in 5X SSPE, 0.3% SDS, 200 micrograms/ml sheared and denatured salmon sperm DNA, and 35% formamide, following standard Southern blotting procedures for 12 to 24 hours. The carrier material is finally washed three times each for 15 minutes using 2X SSC, 0.2% SDS at 60°C.

Polypeptide having cellulolytic enhancing activity: The term "polypeptide having cellulolytic enhancing activity" means a GH61 polypeptide that catalyzes the enhancement of the hydrolysis of a cellulosic material by enzyme having cellulolytic activity. For purposes of the present invention, cellulolytic enhancing activity is determined by measuring the increase in reducing sugars or the increase of the total of cellobiose and glucose from the hydrolysis of a cellulosic material by cellulolytic enzyme under the following conditions: 1-50 mg of total protein/g of cellulose in PCS, wherein total protein is comprised of 50-99.5% w/w cellulolytic enzyme protein and 0.5-50% w/w protein of a GH61 polypeptide having cellulolytic enhancing activity for 1-7 days at a suitable temperature, *e.g.*, 50°C, 55°C, or 60°C, and pH, *e.g.*, 5.0 or 5.5, compared to a control hydrolysis with equal total protein loading without cellulolytic enhancing activity (1-50 mg of cellulolytic protein/g of cellulose in PCS). In a preferred aspect, a mixture of CELLUCLAST® 1.5L (Novozymes A/S, Bagsværd, Denmark) in the presence of 2-3% of total protein weight *Aspergillus oryzae* beta-glucosidase (recombinantly produced in *Aspergillus oryzae* according to WO 02/095014) or 2-3% of total protein weight *Aspergillus fumigatus* beta-glucosidase (recombinantly produced in *Aspergillus oryzae* as described in WO 2002/095014) of cellulase protein loading is used as the source of the cellulolytic activity.

The GH61 polypeptides having cellulolytic enhancing activity enhance the hydrolysis of a cellulosic material catalyzed by enzyme having cellulolytic activity by reducing the amount of cellulolytic enzyme required to reach the same degree of hydrolysis preferably at least 1.01-fold, *e.g.*, at least 1.05-fold, at least 1.10-fold, at least 1.25-fold, at least 1.5-fold, at least 2-fold, at least 3-fold, at least 4-fold, at least 5-fold, at least 10-fold, or at least 20-fold.

Pretreated corn stover: The term "PCS" or "Pretreated Corn Stover" means a cellulosic material derived from corn stover by treatment with heat and dilute sulfuric acid, alkaline pretreatment, or neutral pretreatment.

Remission value: Wash performance is expressed as a Remission value of the stained swatches. After washing and rinsing the swatches were spread out flat and allowed to air dry at room temperature overnight. All washes swatches are evaluated the day after the wash. Light reflectance evaluations of the swatches were done using a Macbeth Color Eye 7000 reflectance spectrophotometer with very small aperture. The measurements were made without UV in the incident light and remission at 460 nm was extracted.

Rinse cycle: The term "rinse cycle" is defined herein as a rinsing operation wherein textile is exposed to water for a period of time by circulating the water and optionally mechanically treat the textile in order to rinse the textile and finally the superfluous water is removed. A rinse cycle may be repeated one, two, three, four, five or even six times at the same or at different temperatures.

Sequence identity: The relatedness between two amino acid sequences or between two nucleotide sequences is described by the parameter "sequence identity".

For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

$$(\text{Identical Residues} \times 100)/(\text{Length of Alignment} - \text{Total Number of Gaps in Alignment})$$

For purposes of the present invention, the sequence identity between two deoxyribonucleotide sequences is determined using the Needleman-Wunsch algorithm (Needleman and Wunsch, 1970, *supra*) as implemented in the Needle program of the EMBOSS package (EMBOSS: The European Molecular Biology Open Software Suite, Rice et al., 2000, *supra*), preferably version 5.0.0 or later. The parameters used are gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

$$(\text{Identical Deoxyribonucleotides} \times 100)/(\text{Length of Alignment} - \text{Total Number of Gaps in Alignment})$$

Subsequence: The term "subsequence" means a polynucleotide having one or more (e.g., several) nucleotides absent from the 5' and/or 3' end of a mature polypeptide coding sequence; wherein the subsequence encodes a fragment having enzyme activity. In one aspect, a subsequence contains at least 85%, *e.g.*, at least 90% or at least 95% of the nucleotides of the mature polypeptide coding sequence of an enzyme.

Variant: The term "variant" means a polypeptide having enzyme activity comprising an alteration, *i.e.,* a substitution, insertion, and/or deletion, at one or more (*e.g.*, several) positions. A substitution means replacement of the amino acid occupying a position with a different amino acid; a deletion means removal of the amino acid occupying a position; and an insertion means adding an amino acid adjacent to and immediately following the amino acid occupying a position.

Wash cycle: The term "wash cycle" is defined herein as a washing operation wherein textile is exposed to the wash liquor for a period of time by circulating the wash liquor and optionally mechanically treat the textile in order to clean the textile and finally the superfluous wash liquor is removed. A wash cycle may be repeated one, two, three, four, five or even six times at the same or at different temperatures. Hereafter the textile is generally rinsed and dried. One of the wash cycles can be a soaking step, where the textile is left soaking in the wash liquor for a period.

Wash liquor: The term "wash liquor" is intended to mean the solution or mixture of water and a detergent component optionally including enzymes used for laundry.

Wash time: The term "wash time" is defined herein as the time it takes for the entire washing process; i.e. the time for the wash cycle(s) and rinse cycle(s) together.

Whiteness: The term "Whiteness" is defined herein as a broad term with different meanings in different regions and for different consumers. Loss of whiteness can e.g. be due to greying, yellowing, or removal of optical brighteners/hueing agents. Greying and yellowing can be due to soil redeposition, body soils, colouring from e.g. iron and copper ions or dye transfer. Whiteness might include one or several issues from the list below: colourant or dye effects; incomplete stain removal (e.g. body soils, sebum etc.); redeposition (greying, yellowing or other discolourations of the object) (removed soils reassociate with other parts of textile, soiled or unsoiled); chemical changes in textile during application; and clarification or brightening of colours.

Xylan-containing material: The term "xylan-containing material" means any material comprising a plant cell wall polysaccharide containing a backbone of beta-(1-4)-linked xylose residues. Xylans of terrestrial plants are heteropolymers possessing a beta-(1-4)-D-xylopyranose backbone, which is branched by short carbohydrate chains. They comprise D-glucuronic acid or its 4-O-methyl ether, L-arabinose, and/or various oligosaccharides, composed of D-xylose, L-arabinose, D- or L-galactose, and D-glucose. Xylan-type polysaccharides can be divided into homoxylans and heteroxylans, which include glucuronoxylans, (arabino)glucuronoxylans, (glucurono)arabinoxylans, arabinoxylans, and complex heteroxylans. See, for example, Ebringerova et al., 2005, Adv. Polym. Sci. 186: 1-67.

In the processes of the present invention, any material containing xylan may be used. In a preferred aspect, the xylan-containing material is lignocellulose.

Xylan degrading activity or xylanolytic activity: The term "xylan degrading activity" or "xylanolytic activity" means a biological activity that hydrolyzes xylan-containing material. The two basic approaches for measuring xylanolytic activity include: (1) measuring the total xylanolytic activity, and (2) measuring the individual xylanolytic activities (*e.g.*, endoxylanases, beta-xylosidases, arabinofuranosidases, alpha-glucuronidases, acetylxylan esterases, feruloyl esterases, and alpha-glucuronyl esterases). Recent progress in assays of xylanolytic enzymes was summarized in several publications including Biely and Puchard, Recent progress in the assays of xylanolytic enzymes, 2006, Journal of the Science of Food and Agriculture 86(11): 1636-1647; Spanikova and Biely, 2006, Glucuronoyl esterase - Novel carbohydrate esterase produced by Schizophyllum commune, FEBS Letters 580(19): 4597-4601; Herrmann, Vrsanska, Jurickova, Hirsch, Biely, and Kubicek, 1997, The beta-D-xylosidase of Trichoderma reesei is a multifunctional beta-D-xylan xylohydrolase, Biochemical Journal 321: 375-381.

Total xylan degrading activity can be measured by determining the reducing sugars formed from various types of xylan, including, for example, oat spelt, beechwood, and larchwood xylans, or by photometric determination of dyed xylan fragments released from various covalently dyed xylans. The most common total xylanolytic activity assay is based on production of reducing sugars from polymeric 4-O-methyl glucuronoxylan as described in Bailey, Biely, Poutanen, 1992, Interlaboratory testing of methods for assay of xylanase activity, Journal of Biotechnology 23(3): 257-270. Xylanase activity can also be determined with 0.2% AZCL-arabinoxylan as substrate in 0.01% TRITON® X-100 (4-(1,1,3,3-tetramethylbutyl)phenyl-polyethylene glycol) and 200 mM sodium phosphate buffer pH 6 at 37°C. One unit of xylanase activity is defined as 1.0 μmole of azurine produced per minute at 37°C, pH 6 from 0.2% AZCL-arabinoxylan as substrate in 200 mM sodium phosphate pH 6 buffer.

For purposes of the present invention, xylan degrading activity is determined by measuring the increase in hydrolysis of birchwood xylan (Sigma Chemical Co., Inc., St. Louis, MO, USA) by xylan-degrading enzyme(s) under the following typical conditions: 1 ml reactions, 5 mg/ml substrate (total solids), 5 mg of xylanolytic protein/g of substrate, 50 mM sodium acetate pH 5, 50°C, 24 hours, sugar analysis using p-hydroxybenzoic acid hydrazide (PHBAH) assay as described by Lever, 1972, A new reaction for colorimetric determination of carbohydrates, Anal. Biochem 47: 273-279.

Xylanase: The term "xylanase" means a 1,4-beta-D-xylan-xylohydrolase (E.C. 3.2.1.8) that catalyzes the endohydrolysis of 1,4-beta-D-xylosidic linkages in xylans. For purposes of the present invention, xylanase activity is determined with 0.2% AZCL-arabinoxylan as substrate in 0.01% TRITON® X-100 and 200 mM sodium phosphate buffer pH 6 at 37°C. One unit of xylanase activity is defined as 1.0 μmole of azurine produced per minute at 37°C, pH 6 from 0.2% AZCL-arabinoxylan as substrate in 200 mM sodium phosphate pH 6 buffer.

## DETAILED DESCRIPTION OF THE INVENTION

The present invention concerns the use of a laundry composition for use in an laundry processes. The composition comprises one or more enzymes capable of degrading cellulosic material. The composition can be a laundry detergent composition or a fabric softener composition.

The enzymes capable of degrading cellulosic material can be selected from the group consisting of *Aspergillus fumigatus* GH10 xylanases, *Aspergillus fumigatus* beta-xylosidases, *Aspergillus fumigatus* cellobiohydrolase I, *Aspergillus fumigatus* cellobiohydrolase II, *Aspergillus fumigatus* beta-glucosidase variants and *Penicillium* sp. (emersonii) GH61 polypeptide.

The inventor has found that these enzymes are superior in degrading cellulosic material released from laundry items during a laundry process, where the enzymes of the invention, in addition to the removal of fuzz and pills from cotton textile, improving whiteness and color clarification, is capable of degrading the cellulosic material to an extend so that the cellulosic material do not clog filters, pipes and drain of the laundry washing machine. Therefore the consumer needs not to clean manually the filter or drain of the laundry washing machine as often as usual. Further, the washed textile appears without fuzz and pills without losing too much textile strength as demonstrated in example 2.

The inventor has found that use of the laundry composition of the invention should comprise a surfactant or a fabric softener agent and an enzyme preparation comprising one or more enzymes capable of degrading cellulosic material, wherein the one or more enzymes capable of degrading cellulosic material comprises:

(i) an Aspergillus fumigatus cellobiohydrolase I;
(ii) an Aspergillus fumigatus cellobiohydrolase II;
(iii) an Aspergillus fumigatus beta-glucosidase or variant thereof; and
(iv) a Penicillium sp. GH61 polypeptide having cellulolytic enhancing activity; or homologs thereof.

The *Aspergillus fumigatus* cellobiohydrolase I or homolog thereof of the enzyme preparation is selected from the group consisting of:

(i) a cellobiohydrolase I comprising or consisting of the mature polypeptide of SEQ ID NO: 2;
(ii) a cellobiohydrolase I comprising or consisting of an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ ID NO: 2;
(iii) a cellobiohydrolase I encoded by a polynucleotide comprising or consisting of a nucleotide sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide coding sequence of SEQ ID NO: 1; and
(iv) a cellobiohydrolase I encoded by a polynucleotide that hybridizes under at least high stringency conditions, very high stringency conditions, with the mature polypeptide coding sequence of SEQ ID NO: 1 or the full-length complement thereof.

The *Aspergillus fumigatus* cellobiohydrolase II or homolog thereof is selected from the group consisting of:

(i) a cellobiohydrolase II comprising or consisting of the mature polypeptide of SEQ ID NO: 4;
(ii) a cellobiohydrolase II comprising or consisting of an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ ID NO: 4;
(iii) a cellobiohydrolase II encoded by a polynucleotide comprising or consisting of a nucleotide sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide

coding sequence of SEQ ID NO: 3; and

(iv) a cellobiohydrolase II encoded by a polynucleotide that hybridizes under at least high stringency conditions, very high stringency conditions, with the mature polypeptide coding sequence of SEQ ID NO: 3 or the full-length complement thereof.

The *Aspergillus fumigatus* beta-glucosidase or homolog thereof is selected from the group consisting of:

(i) a beta-glucosidase comprising or consisting of the mature polypeptide of SEQ ID NO: 6;

(ii) a beta-glucosidase comprising or consisting of an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ ID NO: 6;

(iii) a beta-glucosidase encoded by a polynucleotide comprising or consisting of a nucleotide sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide coding sequence of SEQ ID NO: 5;

(iv) a beta-glucosidase encoded by a polynucleotide that hybridizes under at least high stringency conditions, very high stringency conditions, with the mature polypeptide coding sequence of SEQ ID NO: 5 or the full-length complement thereof; and

(v) a beta-glucosidase variant comprising a substitution at one or more positions corresponding to positions 100, 283, 456, and 512 of the mature polypeptide of SEQ ID NO: 6, wherein the variant has beta-glucosidase activity; and

The *Penicillium* sp. GH61 polypeptide having cellulolytic enhancing activity or homolog thereof is selected from the group consisting of:

(i) a GH61 polypeptide having cellulolytic enhancing activity comprising or consisting of the mature polypeptide of SEQ ID NO: 6;

(ii) a GH61 polypeptide having cellulolytic enhancing activity comprising or consisting of an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ ID NO: 8;

(iii) a GH61 polypeptide having cellulolytic enhancing activity encoded by a polynucleotide comprising or consisting of a nucleotide sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide coding sequence of SEQ ID NO: 7; and

(iv) a GH61 polypeptide having cellulolytic enhancing activity encoded by a polynucleotide that hybridizes under at least high stringency conditions, very high stringency conditions, with the mature polypeptide coding sequence of SEQ ID NO: 7 or the full-length complement thereof.

The beta-glucosidase variant of the enzyme preparation comprises one or more (several) substitutions selected from the group consisting of G142S, Q183R, H266Q, and D703G.

In an embodiment of the invention the enzyme preparation further comprises *Penicillium emersonii* GH61A polypeptide having cellulolytic enhancing activity disclosed in WO 2011/041397 and *Aspergillus fumigatus beta-glucosidase* (SEQ ID NO: 2 of WO 2005/047499) or SEQ ID NO: 6 herein or a variant thereof with the following substitutions F100D, S283G, N456E, F512Y.

In a preferred embodiment the invention the enzyme preparation comprises:

i. an Aspergillus fumigatus cellobiohydrolase I preferably the one shown in SEQ ID NO: 2 herein;

ii. an Aspergillus fumigatus cellobiohydrolase II preferably the one shown in SEQ ID NO: 4 herein;

iii. an Aspergillus fumigatus beta-glucosidase preferably the one shown in SEQ ID NO: 6 herein; or variant thereof, such as one with the following substitutions: F100D, S283G, N456E, F512Y (using SEQ ID NO: 6 herein for numbering); and

iv. a Penicillium sp. GH61 polypeptide having cellulolytic enhancing activity preferably the one shown in SEQ ID NO: 8 herein; or homologs thereof.

The enzyme preparation can further comprise one or more enzymes selected from the group consisting of:

an *Aspergillus fumigatus* xylanase or homolog thereof,
an *Aspergillus fumigatus* beta-xylosidase or homolog thereof; or
a combination of (i) and (ii).

The *Aspergillus fumigatus* xylanase or homolog thereof is selected from the group consisting of:

an *Aspergillus fumigatus* xylanase comprising or consisting of the mature polypeptide of SEQ ID NO: 10, SEQ ID NO: 12, or SEQ ID NO: 14;
a xylanase comprising or consisting of an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ ID NO: 10, SEQ ID NO: 12, or SEQ ID NO: 14;
a xylanase encoded by a polynucleotide comprising or consisting of a nucleotide sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide coding sequence of SEQ ID NO: 9, SEQ ID NO: 11, or SEQ ID NO: 13; and
a xylanase encoded by a polynucleotide that hybridizes under at least high stringency conditions, very high stringency conditions, with the mature polypeptide coding sequence of SEQ ID NO: 9, SEQ ID NO: 11, or SEQ ID NO: 13; or the full-length complement thereof.

The *Aspergillus fumigatus* beta-xylosidase or homolog thereof is selected from the group consisting of:

beta-xylosidase comprising or consisting of the mature polypeptide of SEQ ID NO: 16;
a beta-xylosidase comprising or consisting of an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ ID NO: 16;
a beta-xylosidase encoded by a polynucleotide comprising or consisting of a nucleotide sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide coding sequence of SEQ ID NO: 15; and
a beta-xylosidase encoded by a polynucleotide that hybridizes under at least high stringency conditions, very high stringency conditions, with the mature polypeptide coding sequence of SEQ ID NO: 15 or the full-length complement thereof.

The enzymes capable of degrading cellulosic material can be present in a laundry detergent composition comprising at least one more enzyme. The additional enzyme can be selected from the group consisting of hemicellulases, peroxidases, proteases, cellulases, xylanases, lipases, phospholipases, esterases, cutinases, pectinases, mannanases, pectate lyases, keratinases, reductases, oxidases, phenoloxidases, lipoxygenases, ligninases, pullulanases, tannases, pentosanases, malanases, β-glucanases, arabinosidases, hyaluronidase, chondroitinase, laccase, DNase chlorophyllases, amylases, perhydrolases, peroxidases, xanthanase and mixtures thereof. In one embodiment of the invention the at least one more enzyme is selected from proteases, lipases, mannanases, pectate lyases and amylases.
The laundry detergent composition comprises a surfactant, which can be selected from the group consisting of anionic, cationic, non-ionic, semi-polar and zwitterionic surfactants. In addition other detergent components such as builders and polymers can be comprised in the laundry detergent composition.

1. The invention further concerns a method for laundering a textile comprising the steps of:

a) Contacting the textile with a wash liquor comprising laundry detergent composition;
b) Completing at least one wash cycle;
c) contacting the textile with water comprising a fabric softener composition; and
d) completing at least one rinse cycle;

wherein the detergent composition and/or the fabric softener composition comprise an enzyme preparation comprising one or more enzymes capable of degrading cellulosic material and wherein the one or more enzymes capable of degrading cellulosic material comprises:

i. an Aspergillus fumigatus cellobiohydrolase I;
ii. an Aspergillus fumigatus cellobiohydrolase II;
iii. an Aspergillus fumigatus beta-glucosidase or variant thereof; and
iv. a Penicillium sp. GH61 polypeptide having cellulolytic enhancing activity; or homologs thereof;

wherein the Aspergillus fumigatus cellobiohydrolase I or homolog thereof is selected from the group consisting of:

(i) a cellobiohydrolase I comprising or consisting of the mature polypeptide of SEQ ID NO: 2;
(ii) a cellobiohydrolase I comprising or consisting of an amino acid sequence having at least 70%, at least 75%, at least 80%, at least 81 %, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ ID NO: 2;
(iii) a cellobiohydrolase I encoded by a polynucleotide comprising or consisting of a nucleotide sequence having at least 70%, at least 75%, at least 80%, at least 81 %, at least 82%, at least 83%, at least 84%, at least 85%, at least 86%, at least 87%, at least 88%, at least 89%, at least 90%, at least 91 %, at least 92%, at least 93%, at least 94%, at least 95%, at least 96%, at least 97%, at least 98%, or at least 99% sequence identity to the mature polypeptide coding sequence of SEQ ID NO: 1; and
(iv) a cellobiohydrolase I encoded by a polynucleotide that hybridizes under at least high stringency conditions, very high stringency conditions, with the mature polypeptide coding sequence of SEQ ID NO: 1 or the full-length complement thereof;

wherein the Aspergillus fumigatus cellobiohydrolase II or homolog thereof is selected from the group consisting of:

(i) a cellobiohydrolase II comprising or consisting of the mature polypeptide of SEQ ID NO: 4;
(ii) a cellobiohydrolase II comprising or consisting of an amino acid sequence having at least 70%, at least 75%, at least 80%, at least 81 %, at least 82%, at least 83%, at least 84%, at least 85%, at least 86%, at least 87%, at least 88%, at least 89%, at least 90%, at least 91 %, at least 92%, at least 93%, at least 94%, at least 95%, at least 96%, at least 97%, at least 98%, or at least 99% sequence identity to the mature polypeptide of SEQ ID NO: 4;
(iii) a cellobiohydrolase II encoded by a polynucleotide comprising or consisting of a nucleotide sequence having at least 70%, at least 75%, at least 80%, at least 81 %, at least 82%, at least 83%, at least 84%, at least 85%, at least 86%, at least 87%, at least 88%, at least 89%, at least 90%, at least 91 %, at least 92%, at least 93%, at least 94%, at least 95%, at least 96%, at least 97%, at least 98%, or at least 99% sequence identity to the mature polypeptide coding sequence of SEQ ID NO: 3; and
(iv) a cellobiohydrolase II encoded by a polynucleotide that hybridizes under at least high stringency conditions, very high stringency conditions, with the mature polypeptide coding sequence of SEQ ID NO: 3 or the full-length complement thereof;

wherein the Aspergillus fumigatus beta-glucosidase or homolog thereof is selected from the group consisting of:

(i) a beta-glucosidase comprising or consisting of the mature polypeptide of SEQ ID NO: 6;
(ii) a beta-glucosidase comprising or consisting of an amino acid sequence having at least 70%, at least 75%, at least 80%, at least 81 %, at least 82%, at least 83%, at least 84%, at least 85%, at least 86%, at least 87%, at least 88%, at least 89%, at least 90%, at least 91 %, at least 92%, at least 93%, at least 94%, at least 95%, at least 96%, at least 97%, at least 98%, or at least 99% sequence identity to the mature polypeptide of SEQ ID NO: 6;
(iii) a beta-glucosidase encoded by a polynucleotide comprising or consisting of a nucleotide sequence having at least 70%, at least 75%, at least 80%, at least 81%, at least 82%, at least 83%, at least 84%, at least 85%, at least 86%, at least 87%, at least 88%, at least 89%, at least 90%, at least 91 %, at least 92%, at least 93%, at least 94%, at least 95%, at least 96%, at least 97%, at least 98%, or at least 99% sequence identity to the mature polypeptide coding sequence of SEQ ID NO: 5;
(iv) a beta-glucosidase encoded by a polynucleotide that hybridizes under at least high stringency conditions, very high stringency conditions, with the mature polypeptide coding sequence of SEQ ID NO: 5 or the full-length complement thereof; and
(v) a beta-glucosidase variant comprising a substitution at one or more positions corresponding to positions 100, 283, 456, and 512 of the mature polypeptide of SEQ ID NO: 6, wherein the variant has beta-glucosidase activity;

and wherein the Penicillium sp. GH61 polypeptide having cellulolytic enhancing activity or homolog thereof is selected from the group consisting of:

(i) a GH61 polypeptide having cellulolytic enhancing activity comprising or consisting of the mature polypeptide of SEQ ID NO: 8;
(ii) a GH61 polypeptide having cellulolytic enhancing activity comprising or consisting of an amino acid sequence having at least 70%, at least 75%, at least 80%, at least 81 %, at least 82%, at least 83%, at least 84%, at least 85%, at least 86%, at least 87%, at least 88%, at least 89%, at least 90%, at least 91 %, at least 92%, at least 93%, at least 94%, at least 95%, at least 96%, at least 97%, at least 98%, or at least 99% sequence identity to the mature polypeptide of SEQ ID NO: 8;
(iii) a GH61 polypeptide having cellulolytic enhancing activity encoded by a polynucleotide comprising or consisting of a nucleotide sequence having at least 70%, at least 75%, at least 80%, at least 81 %, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide coding sequence of SEQ ID NO: 7; and

a GH61 polypeptide having cellulolytic enhancing activity encoded by a polynucleotide that hybridizes under at least high stringency conditions, very high stringency conditions, with the mature polypeptide coding sequence of SEQ ID NO: 7 or the full-length complement thereof
and wherein the method prevents build-up and/or removal of fuzz and pills from a textile and improves the whiteness of the textile.

In order to facilitate the degradation of the cellulosic material, an aqueous solution of an acidic material can be used during the washing process. The acidic material should be capable of lowering the pH to below 5. It is believed that when an acidic solution is used on cellulosic material, the structure of the cellulosic material opens up and is more susceptible to the enzymes capable of degrading cellulosic material. The process of degrading the cellulosic material is thereby faster than the degradation process without use of acid.

The exposing of the acidic material can be done a separate step in the laundering process, where the textile is exposed to an aqueous solution of an acidic material before the textile is exposed to washing liquor. This step can be performed in several ways. One way is by simply adding an acidic material to the interior of the laundry washing machine before starting the washing process. The acidic material will then dissolve when contacted with water. Another way is by circulating an aqueous solution of the acidic material before exposing the textile to the wash liquor, e.g. in a pre-wash step. The acidic solution can be exposed to the textile during a rinsing step. In one embodiment the fabric softener used during rinsing has a pH below 5. The washing of textile can be carried out by hand or with a laundry washing machine.

Alternatively, the laundry detergent composition is a powder or a granule and the acidic material is the outer layer of the powder granules. The acidic material is thereby released from the laundry detergent composition and dissolved in the water in the washing machine before the laundry detergent composition is released. Another option is that the acidic material and the laundry detergent composition are contained in a pouch having two or more compartments, where the acidic material is contained in one compartment and the laundry detergent composition is contained in the other compartment. The compartment with the acidic material can then be released and dissolved before the laundry detergent composition is released from the other compartment. Further, the composition can be a tablet having two or more layers, wherein the acidic material is the outer layer of the bar, which will then be released as described above.

The acidic solution should be capable of lowering the pH below 4 during at least a period of the washing cycle. The pH may be even further lowered e.g. to below pH 3.5, such as below pH 3, below pH 2.5 or below pH 2. The period of lowering the pH may be at least 1 minute, such as at least 2 minutes, at least 3 minutes, at least 4 minutes, at least 5 minutes, at least 6 minutes or at least 7 minutes.

The ability of lowering the pH during the rinsing step is due to a buffering agent selected from the group consisting of citric acid, acetic acid, potassium dihydrogen phosphate, boric acid, diethyl barbituric acid, Carmody buffer and Britton-Robinson buffer.

The laundry detergent composition may be in the form of a powder, a bar, a homogenous tablet, a tablet having two or more layers, a pouch having one or more compartments, a regular or compact powder, a granule, a paste, a gel, or a regular, compact or concentrated liquid. The composition can be a powder or a granule where the acidic material is coated on the powder or granule as an outer layer. Alternatively the composition is a tablet having two or more layers, wherein the acidic material is the outer layer of the bar.

The composition can be a pouch having at least two compartments, wherein the acidic material is present in one compartment and is released before content of the other compartment(s).

The inventor has found that the enzyme preparation of the invention is very good at degrading cellulosic material. When the enzyme preparation is used in a laundering process as described above in combination with an aqueous solution of an acidic material, the degradation of cellulosic material from fuzz and pills can be even better than degradation

of cellulosic material in a similar process without the use of an acidic solution. The enzyme preparation comprises: *Aspergillus fumigatus* GH10 xylanase, *Aspergillus fumigatus* beta-xylosidase, *Aspergillus fumigatus* cellobiohydrolase I, *Aspergillus fumigatus* cellobiohydrolase II, *Aspergillus fumigatus* beta-glucosidase variant and *Penicillium* sp. (emersonii) GH61 polypeptide.

The one or more enzymes capable of degrading cellulosic material which is used in the laundry washing process can be comprised in a laundry detergent composition according to the present invention.

The one or more enzymes capable of degrading cellulosic material can be used for degrading cellulosic material during a laundry process. An acidic material may be used during the laundry process. The one or more enzymes capable of degrading cellulosic material can also be used for cleaning the interior of a laundry washing machine, e.g. cleaning of the drain for surplus of cellulose from fuzz and pills, where the cellulosic material remains after a washing process.

The invention further concerns a method for cleaning the interior of a laundry washing machine, which method comprises exposing the interior of the laundry washing machine to one or more enzymes capable of degrading cellulosic material.

The one or more enzymes capable of degrading cellulosic material can be an enzyme preparation comprising:

(i) an Aspergillus fumigatus cellobiohydrolase I;
(ii) an Aspergillus fumigatus cellobiohydrolase II;
(iii) an Aspergillus fumigatus beta-glucosidase or variant thereof; and
(iv) a Penicillium sp. GH61 polypeptide having cellulolytic enhancing activity; or homologs thereof, and which enzymes are described in details above.

**Concentration of the enzyme**

In one embodiment of the present invention, the polypeptide of the present invention may be used in the laundry detergent composition in an amount corresponding to 0.001-200 mg of protein, such as 0.005-100 mg of protein, preferably 0.01-50 mg of protein, more preferably 0.05-20 mg of protein, even more preferably 0.1-10 mg of protein per liter of wash liquor.

The enzyme(s) of the detergent composition of the invention may be stabilized using conventional stabilizing agents, e.g. a polyol such as propylene glycol or glycerol, a sugar or sugar alcohol, lactic acid, boric acid, or a boric acid derivative, *e.g.* an aromatic borate ester, or a phenyl boronic acid derivative such as 4-formylphenyl boronic acid, and the composition may be formulated as described in, for example, WO92/19709 and WO92/19708.

A polypeptide of the present invention may also be incorporated in the detergent formulations disclosed in WO97/07202.

***Surfactants***

The laundry detergent composition may comprise one or more surfactants, which may be anionic and/or cationic and/or non-ionic and/or semi-polar and/or zwitterionic, or a mixture thereof.

In a particular embodiment, the detergent composition includes a mixture of one or more nonionic surfactants and one or more anionic surfactants. The surfactant(s) is typically present at a level of from about 0.1% to 60% by weight, such as about 1% to about 40%, or about 3% to about 20%, or about 3% to about 10%. The surfactant(s) is chosen based on the desired cleaning application, and may include any conventional surfactant(s) known in the art.

When included therein, the detergent will usually contain from about 1% to about 40% by weight of an anionic surfactant, such as from about 5% to about 30%, including from about 5% to about 15%, or from about 15% to about 20%, or from about 20% to about 25% of an anionic surfactant. Non-limiting examples of anionic surfactants include sulfates and sulfonates, in particular, linear alkylbenzenesulfonates (LAS), isomers of LAS, branched alkylbenzenesulfonates (BABS), phenylalkanesulfonates, alpha-olefinsulfonates (AOS), olefin sulfonates, alkene sulfonates, alkane-2,3-diylbis(sulfates), hydroxyalkanesulfonates and disulfonates, alkyl sulfates (AS) such as sodium dodecyl sulfate (SDS), fatty alcohol sulfates (FAS), primary alcohol sulfates (PAS), alcohol ethersulfates (AES or AEOS or FES, also known as alcohol ethoxysulfates or fatty alcohol ether sulfates), secondary alkanesulfonates (SAS), paraffin sulfonates (PS), ester sulfonates, sulfonated fatty acid glycerol esters, alpha-sulfo fatty acid methyl esters (alpha-SFMe or SES) including methyl ester sulfonate (MES), alkyl- or alkenylsuccinic acid, dodecenyl/tetradecenyl succinic acid (DTSA), fatty acid derivatives of amino acids, diesters and monoesters of sulfo-succinic acid or salt of fatty acids (soap), and combinations thereof.

When included therein, the detergent will usually contain from about from about 1% to about 40% by weigh of a cationic surfactant, for example from about 0.5% to about 30%, in particular from about 1% to about 20%, from about 3% to about 10%, such as from about 3% to about 5%, from about 8% to about 12% or from about 10% to about 12%. Non-limiting examples of cationic surfactants include alkyldimethylethanolamine quat (ADMEAQ), cetyltrimethylammonium bromide (CTAB), dimethyldistearylammonium chloride (DSDMAC), and alkylbenzyldimethylammonium, alkyl quaternary ammonium compounds, alkoxylated quaternary ammonium (AQA) compounds, ester quats, and combinations thereof.

When included therein, the detergent will usually contain from about 0.2% to about 40% by weight of a nonionic surfactant, for example from about 0.5% to about 30%, in particular from about 1% to about 20%, from about 3% to about 10%, such as from about 3% to about 5%, from about 8% to about 12%, or from about 10% to about 12%. Non-limiting examples of nonionic surfactants include alcohol ethoxylates (AE or AEO), alcohol propoxylates, propoxylated fatty alcohols (PFA), alkoxylated fatty acid alkyl esters, such as ethoxylated and/or propoxylated fatty acid alkyl esters, alkylphenol ethoxylates (APE), nonylphenol ethoxylates (NPE), alkylpolyglycosides (APG), alkoxylated amines, fatty acid monoethanolamides (FAM), fatty acid diethanolamides (FADA), ethoxylated fatty acid monoethanolamides (EFAM), propoxylated fatty acid monoethanolamides (PFAM), polyhydroxyalkyl fatty acid amides, or N-acyl N-alkyl derivatives of glucosamine (glucamides, GA, or fatty acid glucamides, FAGA), as well as products available under the trade names SPAN and TWEEN, and combinations thereof.

When included therein, the detergent will usually contain from about 0% to about 20% by weight of a semipolar surfactant. Non-limiting examples of semipolar surfactants include amine oxides (AO) such as alkyldimethylamineoxide, *N*-(coco alkyl)-*N*,*N*-dimethylamine oxide and *N*-(tallow-alkyl)-*N*,*N*-bis(2-hydroxyethyl)amine oxide, , and combinations thereof.

When included therein, the detergent will usually contain from about 0% to about 20% by weight of a zwitterionic surfactant. Non-limiting examples of zwitterionic surfactants include betaines such as alkyldimethylbetaines, sulfobetaines, and combinations thereof.

***Hydrotropes***

A hydrotrope is a compound that solubilises hydrophobic compounds in aqueous solutions (or oppositely, polar substances in a non-polar environment). Typically, hydrotropes have both hydrophilic and a hydrophobic character (so-called amphiphilic properties as known from surfactants); however the molecular structure of hydrotropes generally do not favor spontaneous self-aggregation, see e.g. review by Hodgdon and Kaler (2007), Current Opinion in Colloid & Interface Science 12: 121-128. Hydrotropes do not display a critical concentration above which self-aggregation occurs as found for surfactants and lipids forming miceller, lamellar or other well defined meso-phases. Instead, many hydrotropes show a continuous-type aggregation process where the sizes of aggregates grow as concentration increases. However, many hydrotropes alter the phase behavior, stability, and colloidal properties of systems containing substances of polar and non-polar character, including mixtures of water, oil, surfactants, and polymers. Hydrotropes are classically used across industries from pharma, personal care, food, to technical applications. Use of hydrotropes in detergent compositions allow for example more concentrated formulations of surfactants (as in the process of compacting liquid detergents by removing water) without inducing undesired phenomena such as phase separation or high viscosity.

The detergent may contain 0-10% by weight, for example 0-5% by weight, such as about 0.5 to about 5%, or about 3% to about 5%, of a hydrotrope. Any hydrotrope known in the art for use in detergents may be utilized. Non-limiting examples of hydrotropes include sodium benzenesulfonate, sodium p-toluene sulfonate (STS), sodium xylene sulfonate (SXS), sodium cumene sulfonate (SCS), sodium cymene sulfonate, amine oxides, alcohols and polyglycolethers, sodium hydroxynaphthoate, sodium hydroxynaphthalene sulfonate, sodium ethylhexyl sulfate, and combinations thereof.

***Builders and Co-Builders***

The detergent composition may contain about 0-65% by weight, such as about 5% to about 50% of a detergent builder or co-builder, or a mixture thereof. In a washing detergent, the level of builder is typically 40-65%, particularly 50-65%. The builder and/or co-builder may particularly be a chelating agent that forms water-soluble complexes with Ca and Mg. Any builder and/or co-builder known in the art for use in laundry cleaning detergents may be utilized. Non-limiting examples of builders include zeolites, diphosphates (pyrophosphates), triphosphates such as sodium triphosphate (STP or STPP), carbonates such as sodium carbonate, soluble silicates such as sodium metasilicate, layered silicates (*e.g.*, SKS-6 from Hoechst), ethanolamines such as 2-aminoethan-1-ol (MEA), diethanolamine (DEA, also known as 2,2'-iminodiethan-1-ol), triethanolamine (TEA, also known as 2,2',2"-nitrilotriethan-1-ol), and (carboxymethyl)inulin (CMI), and combinations thereof.

The detergent composition may also contain 0-50% by weight, such as about 5% to about 30%, of a detergent co-builder. The detergent composition may include include a co-builder alone, or in combination with a builder, for example a zeolite builder. Non-limiting examples of co-builders include homopolymers of polyacrylates or copolymers thereof, such as poly(acrylic acid) (PAA) or copoly(acrylic acid/maleic acid) (PAA/PMA). Further non-limiting examples include citrate, chelators such as aminocarboxylates, aminopolycarboxylates and phosphonates, and alkyl- or alkenylsuccinic acid. Additional specific examples include 2,2',2"-nitrilotriacetic acid (NTA), ethylenediaminetetraacetic acid (EDTA), diethylenetriaminepentaacetic acid (DTPA), iminodisuccinic acid (IDS), ethylenediamine-*N*,*N*'-disuccinic acid (EDDS), methylglycinediacetic acid (MGDA), glutamic acid-*N*,*N*-diacetic acid (GLDA), 1-hydroxyethane-1,1-diphosphonic acid (HEDP), ethylenediaminetetra(methylenephosphonic acid) (EDTMPA), diethylenetriaminepentakis(methylenephosphonic acid) (DTMPA or DTPMPA), *N*-(2-hydroxyethyl)iminodiacetic acid (EDG), aspartic acid-*N*-monoacetic acid (ASMA), aspartic acid-*N*,*N*-diacetic acid (ASDA), aspartic acid-*N*-monopropionic acid (ASMP), iminodisuccinic acid (IDA), *N*-(2-sulfomethyl)-aspartic acid (SMAS), *N*-(2-sulfoethyl)-aspartic acid (SEAS), *N*-(2-sulfomethyl)-glutamic acid (SMGL), *N*-(2-sulfoethyl)-glutamic acid (SEGL), *N*-methyliminodiacetic acid (MIDA), $\alpha$-alanine-*N*,*N*-diacetic acid ($\alpha$-ALDA), serine-*N*,*N*-diacetic acid (SEDA), isoserine-*N*,*N*-diacetic acid (ISDA), phenylalanine-*N*,*N*-diacetic acid (PHDA), anthranilic acid-*N*,*N*-diacetic acid (ANDA), sulfanilic acid-*N*,*N*-diacetic acid (SLDA), taurine-*N*,*N*-diacetic acid (TUDA) and sulfomethyl-*N*,*N*-diacetic acid (SMDA), *N*-(2-hydroxyethyl)ethylenediamine-*N*,*N*',*N*"-triacetic acid (HEDTA), diethanolglycine (DEG), diethylenetriamine penta(methylenephosphonic acid) (DTPMP), aminotris(methylenephosphonic acid) (ATMP), and combinations and salts thereof. Further exemplary builders and/or co-builders are described in, e.g., WO 09/102854, US 5977053

### *Bleaching Systems*

The detergent may contain 0-30% by weight, such as about 1% to about 20%, of a bleaching system. Any bleaching system known in the art for use in laundry cleaning detergents may be utilized. Suitable bleaching system components include bleaching catalysts, photobleaches, bleach activators, sources of hydrogen peroxide such as sodium percarbonate, sodium perborates and hydrogen peroxide-urea (1:1), preformed peracids and mixtures thereof. Suitable preformed peracids include, but are not limited to, peroxycarboxylic acids and salts, diperoxydicarboxylic acids, perimidic acids and salts, peroxymonosulfuric acids and salts, for example, Oxone (R), and mixtures thereof. Non-limiting examples of bleaching systems include peroxide-based bleaching systems, which may comprise, for example, an inorganic salt, including alkali metal salts such as sodium salts of perborate (usually mono- or tetra-hydrate), percarbonate, persulfate, perphosphate, persilicate salts, in combination with a peracid-forming bleach activator. The term bleach activator is meant herein as a compound which reacts with hydrogen peroxide to form a peracid via perhydrolysis. The peracid thus formed constitutes the activated bleach. Suitable bleach activators to be used herein include those belonging to the class of esters, amides, imides or anhydrides. Suitable examples are tetraacetylethylenediamine (TAED), sodium 4-[(3,5,5-trimethylhexanoyl)oxy]benzene-1-sulfonate (ISONOBS), 4-(dodecanoyloxy)benzene-1-sulfonate (LOBS), 4-(decanoyloxy)benzene-1-sulfonate, 4-(decanoyloxy)benzoate (DOBS or DOBA), 4-(nonanoyloxy)benzene-1-sulfonate (NOBS), and/or those disclosed in WO98/17767. A particular family of bleach activators of interest was disclosed in EP624154 and particulary preferred in that family is acetyl triethyl citrate (ATC). ATC or a short chain triglyceride like triacetin has the advantage that it is environmentally friendly Furthermore acetyl triethyl citrate and triacetin have good hydrolytical stability in the product upon storage and are efficient bleach activators. Finally ATC is multifunctional, as the citrate released in the perhydrolysis reaction may function as a builder. Alternatively, the bleaching system may comprise peroxyacids of, for example, the amide, imide, or sulfone type. The bleaching system may also comprise peracids such as 6-(phthalimido)peroxyhexanoic acid (PAP). The bleaching system may also include a bleach catalyst. In some embodiments the bleach component may be an organic catalyst selected from the group consisting of organic catalysts having the following formulae:

(i)

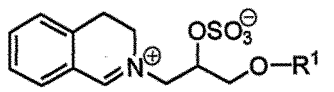

(ii)

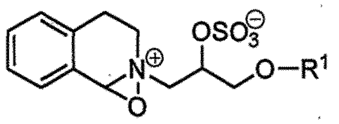

(iii) and mixtures thereof;

wherein each $R^1$ is independently a branched alkyl group containing from 9 to 24 carbons or linear alkyl group containing from 11 to 24 carbons, preferably each $R^1$ is independently a branched alkyl group containing from 9 to 18 carbons or

linear alkyl group containing from 11 to 18 carbons, more preferably each $R^1$ is independently selected from the group consisting of 2-propylheptyl, 2-butyloctyl, 2-pentylnonyl, 2-hexyldecyl, dodecyl, tetradecyl, hexadecyl, octadecyl, isononyl, isodecyl, isotridecyl and isopentadecyl. Other exemplary bleaching systems are described, e.g. in WO2007/087258, WO2007/087244, WO2007/087259, EP1867708 (Vitamin K) and WO2007/087242. Suitable photobleaches may for example be sulfonated zinc or aluminium phthalocyanines.

Preferably the bleach component comprises a source of peracid in addition to bleach catalyst, particularly organic bleach catalyst. The source of peracid may be selected from (a) pre-formed peracid; (b) percarbonate, perborate or persulfate salt (hydrogen peroxide source) preferably in combination with a bleach activator; and (c) perhydrolase enzyme and an ester for forming peracid in situ in the presence of water in a textile or hard surface treatment step.

***Polymers***

The detergent may contain 0-10% by weight, such as 0.5-5%, 2-5%, 0.5-2% or 0.2-1 % of a polymer. Any polymer known in the art for use in detergents may be utilized. The polymer may function as a co-builder as mentioned above, or may provide antiredeposition, fiber protection, soil release, dye transfer inhibition, grease cleaning and/or antifoaming properties. Some polymers may have more than one of the above-mentioned properties and/or more than one of the below-mentioned motifs. Exemplary polymers include (carboxymethyl)cellulose (CMC), poly(vinyl alcohol) (PVA), poly(vinylpyrrolidone) (PVP), poly(ethyleneglycol) or poly(ethylene oxide) (PEG), ethoxylated poly(ethyleneimine), carboxymethyl inulin (CMI), and polycarboxylates such as PAA, PAA/PMA, poly-aspartic acid, and lauryl methacrylate/acrylic acid copolymers , hydrophobically modified CMC (HM-CMC) and silicones, copolymers of terephthalic acid and oligomeric glycols, copolymers of poly(ethylene terephthalate) and poly(oxyethene terephthalate) (PET-POET), PVP, poly(vinylimidazole) (PVI), poly(vinylpyridine-*N*-oxide) (PVPO or PVPNO) and polyvinylpyrrolidone-vinylimidazole (PVPVI). Further exemplary polymers include sulfonated polycarboxylates, polyethylene oxide and polypropylene oxide (PEO-PPO) and diquaternium ethoxy sulfate. Other exemplary polymers are disclosed in, e.g., WO 2006/130575. Salts of the above-mentioned polymers are also contemplated.

***Fabric hueing agents***

The detergent compositions of the present invention may also include fabric hueing agents such as dyes or pigments, which when formulated in detergent compositions can deposit onto a fabric when said fabric is contacted with a wash liquor comprising said detergent compositions and thus altering the tint of said fabric through absorption/reflection of visible light. Fluorescent whitening agents emit at least some visible light. In contrast, fabric hueing agents alter the tint of a surface as they absorb at least a portion of the visible light spectrum. Suitable fabric hueing agents include dyes and dye-clay conjugates, and may also include pigments. Suitable dyes include small molecule dyes and polymeric dyes. Suitable small molecule dyes include small molecule dyes selected from the group consisting of dyes falling into the Colour Index (C.I.) classifications of Direct Blue, Direct Red, Direct Violet, Acid Blue, Acid Red, Acid Violet, Basic Blue, Basic Violet and Basic Red, or mixtures thereof, for example as described in WO2005/03274, WO2005/03275, WO2005/03276 and EP1876226. The detergent composition preferably comprises from about 0.00003 wt% to about 0.2 wt%, from about 0.00008 wt% to about 0.05 wt%, or even from about 0.0001 wt% to about 0.04 wt% fabric hueing agent. The composition may comprise from 0.0001 wt% to 0.2 wt% fabric hueing agent, this may be especially preferred when the composition is in the form of a unit dose pouch. Suitable hueing agents are also disclosed in, e.g. WO 2007/087257 and WO2007/087243.

***Enzymes***

The detergent additive as well as the detergent composition may comprise one or more [additional] enzymes such as a protease, lipase, cutinase, an amylase, carbohydrase, cellulase, pectinase, mannanase, arabinase, galactanase, xylanase, oxidase, *e.g.*, a laccase, and/or peroxidase.

In general, the properties of the selected enzyme(s) should be compatible with the selected detergent, (*i.e.,* pH-optimum, compatibility with other enzymatic and non-enzymatic ingredients, etc.), and the enzyme(s) should be present in effective amounts.

**Cellulases**

Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium, e.g.,* the fungal cellulases produced from *Humicola insolens, Myceliophthora thermophila* and *Fusarium oxysporum* disclosed in US 4,435,307, US 5,648,263, US 5,691,178, US 5,776,757 and WO 89/09259.

Especially suitable cellulases are the alkaline or neutral cellulases having colour care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307 and WO99/001544.

Other cellulases are endo-beta-1,4-glucanase enzyme having a sequence of at least 97% identity to the amino acid sequence of position 1 to position 773 of SEQ ID NO:2 of WO 2002/099091 or a family 44 xyloglucanase, which a xyloglucanase enzyme having a sequence of at least 60% identity to positions 40-559 of SEQ ID NO: 2 of WO 2001/062903.

Commercially available cellulases include Celluzyme™, and Carezyme™ (Novozymes A/S) Carezyme Premium™ (Novozymes A/S), Celluclean™ (Novozymes A/S), Celluclean Classic™ (Novozymes A/S), Cellusoft™ (Novozymes A/S), Whitezyme™ (Novozymes A/S), Clazinase™, and Puradax HA™ (Genencor International Inc.), and KAC-500(B)™ (Kao Corporation).

**Mannanases**

Suitable mannanases include those of bacterial or fungal origin. Chemically or genetically modified mutants are included. The mannanase may be an alkaline mannanase of Family 5 or 26. It may be a wild-type from *Bacillus* or *Humicola,* particularly *B. agaradhaerens, B. licheniformis, B. halodurans, B. clausii,* or *H. insolens.* Suitable mannanases are described in WO 1999/064619. A commercially available mannanase is Mannaway (Novozymes A/S).

<u>Cellulase</u>

Suitable cellulases include complete cellulases or mono-component endoglucanases of bacterial or fungal origin. Chemically or genetically modified mutants are included. The cellulase may for example be a mono-component or a mixture of mono-component endo-1,4-beta-glucanase often just termed endoglucanases. Suitable cellulases include a fungal cellulase from *Humicola insolens* (US 4,435,307) or from *Trichoderma,* e.g. *T. reesei* or *T. viride*. Examples of cellulases are described in EP 0 495 257. Other suitable cellulases are from *Thielavia e.g. Thielavia terrestris as described in* WO 96/29397 or *Fusarium oxysporum as described in* WO 91/17244 or from *Bacillus* as described in, WO 02/099091 and JP 2000210081. Other examples are cellulase variants such as those described in WO 94/07998, EP 0 531 315, US 5,457,046, US 5,686,593, US 5,763,254, WO 95/24471, WO 98/12307 Commercially available cellulases include Carezyme®, Celluzyme®, Celluclean®, Celluclast® and Endolase®; Renozyme®; Whitezyme® (Novozymes A/S) Puradax®, Puradax HA, and Puradax EG (available from Genencor).

**Peroxidases/Oxidases**

Suitable peroxidases/oxidases include those of plant, bacterial or fungal origin. Chemically modified or protein engineered mutants are included. Examples of useful peroxidases include peroxidases from *Coprinus, e.g.,* from *C. cinereus,* and variants thereof as those described in WO 93/24618, WO 95/10602, and WO 98/15257. Commercially available peroxidases include Guardzyme™ (Novozymes A/S).

**Proteases**

Suitable proteases include those of bacterial, fungal, plant, viral or animal origin e.g. vegetable or microbial origin. Microbial origin is preferred. Chemically modified or protein engineered mutants are included. It may be an alkaline protease, such as a serine protease or a metalloprotease. A serine protease may for example be of the S1 family, such as trypsin, or the S8 family such as subtilisin. A metalloproteases protease may for example be a thermolysin from e.g. family M4 or other metalloprotease such as those from M5, M7 or M8 families.

The term "subtilases" refers to a sub-group of serine protease according to Siezen et al., Protein Engng. 4 (1991) 719-737 and Siezen et al. Protein Science 6 (1997) 501-523. Serine proteases are a subgroup of proteases characterized by having a serine in the active site, which forms a covalent adduct with the substrate. The subtilases may be divided into 6 sub-divisions, i.e. the Subtilisin family, the Thermitase family, the Proteinase K family, the Lantibiotic peptidase family, the Kexin family and the Pyrolysin family.

Examples of subtilases are those derived from *Bacillus* such as *Bacillus lentus, B. alkalophilus*, *B. subtilis*, *B. amyloliquefaciens*, *Bacillus pumilus* and *Bacillus gibsonii* described in; US7262042 and WO09/021867, and *subtilisin lentus, subtilisin Novo, subtilisin Carlsberg, Bacillus licheniformis, subtilisin BPN', subtilisin 309, subtilisin* 147 and *subtilisin 168* described in WO89/06279 and protease PD138 described in (WO93/18140). Other useful proteases may be those described in WO92/175177, WO01/016285, WO02/026024 and WO02/016547. Examples of trypsin-like proteases are trypsin (e.g. of porcine or bovine origin) and the *Fusarium* protease described in WO89/06270, WO94/25583 and

WO05/040372, and the chymotrypsin proteases derived from *Cellumonas* described in WO05/052161 and WO05/052146.

A further preferred protease is the alkaline protease from *Bacillus lentus* DSM 5483, as described for example in WO95/23221, and variants thereof which are described in WO92/21760, WO95/23221, EP1921147 and EP1921148.

Examples of metalloproteases are the neutral metalloprotease as described in WO07/044993 (Genencor Int.) such as those derived from *Bacillus amyloliquefaciens*.

Examples of useful proteases are the variants described in: WO92/19729, WO96/034946, WO98/20115, WO98/20116, WO99/011768, WO01/44452, WO03/006602, WO04/03186, WO04/041979, WO07/006305, WO11/036263, WO11/036264, especially the variants with substitutions in one or more of the following positions: 3, 4, 9, 15, 27, 36, 57, 68, 76, 87, 95, 96, 97, 98, 99, 100, 101, 102, 103, 104, 106, 118, 120, 123, 128, 129, 130, 160, 167, 170, 194, 195, 199, 205, 206, 217, 218, 222, 224, 232, 235, 236, 245, 248, 252 and 274 using the BPN' numbering. More preferred the subtilase variants may comprise the mutations: S3T, V4I, S9R, A15T, K27R, *36D, V68A, N76D, N87S,R, *97E, A98S, S99G,D,A, S99AD, S101G,M,R S103A, V104I,Y,N, S106A, G118V,R, H120D,N, N123S, S128L, P129Q, S130A, G160D, Y167A, R170S, A194P, G195E, V199M, V205I, L217D, N218D, M222S, A232V, K235L, Q236H, Q245R, N252K, T274A (using BPN' numbering).

Suitable commercially available protease enzymes include those sold under the trade names Alcalase®, Duralase™, Durazym™, Relase®, Relase® Ultra, Savinase®, Savinase® Ultra, Primase®, Polarzyme®, Kannase®, Liquanase®, Liquanase® Ultra, Ovozyme®, Coronase®, Coronase® Ultra, Neutrase®, Everlase® and Esperase® (Novozymes A/S), those sold under the tradename Maxatase®, Maxacal®, Maxapem®, Purafect®, Purafect Prime®, , Purafect MA®, Purafect Ox®, Purafect OxP®, Puramax®, Properase®, , FN2®, FN3®, FN4®, Excellase®, Eraser®, Opticlean® and Optimase® (Danisco/DuPont), Axapem™ (Gist-Brocases N.V.), BLAP (sequence shown in Figure 29 of US5352604) and variants hereof (Henkel AG) and KAP (*Bacillus alkalophilus* subtilisin) from Kao.

**Lipases and Cutinases**

Suitable lipases and cutinases include those of bacterial or fungal origin. Chemically modified or protein engineered mutant enzymes are included. Examples include lipase from *Thermomyces,* e.g. from *T. lanuginosus* (previously named *Humicola lanuginosa*) as described in EP258068 and EP305216, cutinase from *Humicola,* e.g. *H. insolens* (WO96/13580), lipase from strains of *Pseudomonas* (some of these now renamed to *Burkholderia*), e.g. *P. alcaligenes* or *P. pseudoalcaligenes* (EP218272), *P. cepacia* (EP331376), *P. sp.* strain SD705 (WO95/06720 & WO96/27002), *P. wisconsinensis* (WO96/12012), GDSL-type *Streptomyces* lipases (WO10/065455), cutinase from *Magnaporthe grisea* (WO10/107560), cutinase from *Pseudomonas mendocina* (US5,389,536), lipase from *Thermobifida fusca* (WO11/084412), *Geobacillus stearothermophilus* lipase (WO11/084417), lipase from *Bacillus subtilis* (WO11/084599), and lipase from *Streptomyces griseus* (WO11/150157) and *S. pristinaespiralis* (WO12/137147).

Other examples are lipase variants such as those described in EP407225, WO92/05249, WO94/01541, WO94/25578, WO95/14783, WO95/30744, WO95/35381, WO95/22615, WO96/00292, WO97/04079, WO97/07202, WO00/34450, WO00/60063, WO01/92502, WO07/87508 and WO09/109500.

Preferred commercial lipase products include include Lipolase™, Lipex™; Lipolex™ and Lipoclean™ (Novozymes A/S), Lumafast (originally from Genencor) and Lipomax (originally from Gist-Brocades).

Still other examples are lipases sometimes referred to as acyltransferases or perhydrolases, e.g. acyltransferases with homology to *Candida antarctica* lipase A (WO10/111143), acyltransferase from *Mycobacterium smegmatis* (WO05/56782), perhydrolases from the CE 7 family (WO09/67279), and variants of the *M. smegmatis* perhydrolase in particular the S54V variant used in the commercial product Gentle Power Bleach from Huntsman Textile Effects Pte Ltd (WO10/100028).

**Amylases**

Suitable amylases which can be used together with the enzyme preparation of the invention may be an alpha-amylase or a glucoamylase and may be of bacterial or fungal origin. Chemically modified or protein engineered mutants are included. Amylases include, for example, alpha-amylases obtained from *Bacillus, e.g.,* a special strain of *Bacillus licheniformis,* described in more detail in GB 1,296,839.

Suitable amylases include amylases having SEQ ID NO: 2 in WO 95/10603 or variants having 90% sequence identity to SEQ ID NO: 3 thereof. Preferred variants are described in WO 94/02597, WO 94/18314, WO 97/43424 and SEQ ID NO: 4 of WO 99/019467, such as variants with substitutions in one or more of the following positions: 15, 23, 105, 106, 124, 128, 133, 154, 156, 178, 179, 181, 188, 190, 197, 201, 202, 207, 208, 209, 211, 243, 264, 304, 305, 391, 408, and 444.

Different suitable amylases include amylases having SEQ ID NO: 6 in WO 02/010355 or variants thereof having 90% sequence identity to SEQ ID NO: 6. Preferred variants of SEQ ID NO: 6 are those having a deletion in positions

181 and 182 and a substitution in position 193.

Other amylases which are suitable are hybrid alpha-amylase comprising residues 1-33 of the alpha-amylase derived from *B. amyloliquefaciens* shown in SEQ ID NO: 6 of WO 2006/066594 and residues 36-483 of the *B. licheniformis* alpha-amylase shown in SEQ ID NO: 4 of WO 2006/066594 or variants having 90% sequence identity thereof. Preferred variants of this hybrid alpha-amylase are those having a substitution, a deletion or an insertion in one of more of the following positions: G48, T49, G107, H156, A181, N190, M197, 1201, A209 and Q264. Most preferred variants of the hybrid alpha-amylase comprising residues 1-33 of the alpha-amylase derived from *B. amyloliquefaciens* shown in SEQ ID NO: 6 of WO 2006/066594 and residues 36-483 of SEQ ID NO: 4 are those having the substitutions:

M197T;
H156Y+A181T+N190F+A209V+Q264S; or
G48A+T49I+G107A+H156Y+A181T+N190F+I201F+A209V+Q264S.

Further amylases which are suitable are amylases having SEQ ID NO: 6 in WO 99/019467 or variants thereof having 90% sequence identity to SEQ ID NO: 6. Preferred variants of SEQ ID NO: 6 are those having a substitution, a deletion or an insertion in one or more of the following positions: R181, G182, H183, G184, N195, I206, E212, E216 and K269. Particularly preferred amylases are those having deletion in positions R181 and G182, or positions H183 and G184.

Additional amylases which can be used are those having SEQ ID NO: 1, SEQ ID NO: 3, SEQ ID NO: 2 or SEQ ID NO: 7 of WO 96/023873 or variants thereof having 90% sequence identity to SEQ ID NO: 1, SEQ ID NO: 2, SEQ ID NO: 3 or SEQ ID NO: 7. Preferred variants of SEQ ID NO: 1, SEQ ID NO: 2, SEQ ID NO: 3 or SEQ ID NO: 7 are those having a substitution, a deletion or an insertion in one or more of the following positions: 140, 181, 182, 183, 184, 195, 206, 212, 243, 260, 269, 304 and 476, using SEQ ID 2 of WO 96/023873 for numbering. More preferred variants are those having a deletion in two positions selected from 181, 182, 183 and 184, such as 181 and 182, 182 and 183, or positions 183 and 184. Most preferred amylase variants of SEQ ID NO: 1, SEQ ID NO: 2 or SEQ ID NO: 7 are those having a deletion in positions 183 and 184 and a substitution in one or more of positions 140, 195, 206, 243, 260, 304 and 476.

Other amylases which can be used are amylases having SEQ ID NO: 2 of WO 08/153815, SEQ ID NO: 10 in WO 01/66712 or variants thereof having 90% sequence identity to SEQ ID NO: 2 of WO 08/153815 or 90% sequence identity to SEQ ID NO: 10 in WO 01/66712. Preferred variants of SEQ ID NO: 10 in WO 01/66712 are those having a substitution, a deletion or an insertion in one of more of the following positions: 176, 177, 178, 179, 190, 201, 207, 211 and 264.

Further suitable amylases are amylases having SEQ ID NO: 2 of WO 09/061380 or variants having 90% sequence identity to SEQ ID NO: 2 thereof. Preferred variants of SEQ ID NO: 2 are those having a truncation of the C-terminus and/or a substitution, a deletion or an insertion in one of more of the following positions: Q87, Q98, S125, N128, T131, T165, K178, R180, S181, T182, G183, M201, F202, N225, S243, N272, N282, Y305, R309, D319, Q320, Q359, K444 and G475. More preferred variants of SEQ ID NO: 2 are those having the substitution in one of more of the following positions: Q87E,R, Q98R, S125A, N128C, T131I, T165I, K178L, T182G, M201L, F202Y, N225E,R, N272E,R, S243Q,A,E,D, Y305R, R309A, Q320R, Q359E, K444E and G475K and/or deletion in position R180 and/or S181 or of T182 and/or G183. Most preferred amylase variants of SEQ ID NO: 2 are those having the substitutions:

N128C+K178L+T182G+Y305R+G475K;
N128C+K178L+T182G+F202Y+Y305R+D319T+G475K;
S125A+N128C+K178L+T182G+Y305R+G475K; or
S125A+N128C+T131I+T165I+K178L+T182G+Y305R+G475K wherein the variants are C-terminally truncated and optionally further comprises a substitution at position 243 and/or a deletion at position 180 and/or position 181.

Further suitable amylases are amylases having SEQ ID NO: 1 of WO13184577 or variants having 90% sequence identity to SEQ ID NO: 1 thereof. Preferred variants of SEQ ID NO: 1 are those having a substitution, a deletion or an insertion in one of more of the following positions: K176, R178, G179, T180, G181, E187, N192, M199, I203, S241, R458, T459, D460, G476 and G477. More preferred variants of SEQ ID NO: 1 are those having the substitution in one of more of the following positions: K176L, E187P, N192FYH, M199L, I203YF, S241QADN, R458N, T459S, D460T, G476K and G477K and/or deletion in position R178 and/or S179 or of T180 and/or G181. Most preferred amylase variants of SEQ ID NO: 1 are those having the substitutions:

E187P+I203Y+G476K
E187P+I203Y+R458N+T459S+D460T+G476K

wherein the variants optionally further comprises a substitution at position 241 and/or a deletion at position 178 and/or position 179.

Further suitable amylases are amylases having SEQ ID NO: 1 of WO10104675 or variants having 90% sequence identity to SEQ ID NO: 1 thereof. Preferred variants of SEQ ID NO: 1 are those having a substitution, a deletion or an insertion in one of more of the following positions: N21, D97, V128 K177, R179, S180, I181, G182, M200, L204, E242, G477 and G478. More preferred variants of SEQ ID NO: 1 are those having the substitution in one of more of the following positions: N21D, D97N, V128I K177L, M200L, L204YF, E242QA, G477K and G478K and/or deletion in position R179 and/or S180 or of 1181 and/or G182. Most preferred amylase variants of SEQ ID NO: 1 are those having the substitutions: N21D+D97N+V128I wherein the variants optionally further comprises a substitution at position 200 and/or a deletion at position 180 and/or position 181.

Other suitable amylases are the alpha-amylase having SEQ ID NO: 12 in WO01/66712 or a variant having at least 90% sequence identity to SEQ ID NO: 12. Preferred amylase variants are those having a substitution, a deletion or an insertion in one of more of the following positions of SEQ ID NO: 12 in WO01/66712: R28, R118, N174; R181, G182, D183, G184, G186, W189, N195, M202, Y298, N299, K302, S303, N306, R310, N314; R320, H324, E345, Y396, R400, W439, R444, N445, K446, Q449, R458, N471, N484. Particular preferred amylases include variants having a deletion of D183 and G184 and having the substitutions R118K, N195F, R320K and R458K, and a variant additionally having substitutions in one or more position selected from the group: M9, G149, G182, G186, M202, T257, Y295, N299, M323, E345 and A339, most preferred a variant that additionally has substitutions in all these positions.

Other examples are amylase variants such as those described in WO2011/098531, WO2013/001078 and WO2013/001087.

Commercially available amylases are Duramyl™, Termamyl™, Fungamyl™, Stainzyme ™, Stainzyme Plus™, Natalase™, Liquozyme X and BAN™ (from Novozymes A/S), and Rapidase™, Purastar™/Effectenz™, Powerase, Preferenz S1000, Preferenz S100 and Preferenz S110 (from Genencor International Inc./DuPont).

## Peroxidases/Oxidases

A peroxidase according to the invention is a peroxidase enzyme comprised by the enzyme classification EC 1.11.1.7, as set out by the Nomenclature Committee of the International Union of Biochemistry and Molecular Biology (IUBMB), or any fragment derived therefrom, exhibiting peroxidase activity.

Suitable peroxidases include those of plant, bacterial or fungal origin. Chemically modified or protein engineered mutants are included. Examples of useful peroxidases include peroxidases from *Coprinopsis*, *e.g*., from *C. cinerea* (EP 179,486), and variants thereof as those described in WO 93/24618, WO 95/10602, and WO 98/15257.

A peroxidase according to the invention also includes a haloperoxidase enzyme, such as chloroperoxidase, bromoperoxidase and compounds exhibiting chloroperoxidase or bromoperoxidase activity. Haloperoxidases are classified according to their specificity for halide ions. Chloroperoxidases (E.C. 1.11.1.10) catalyze formation of hypochlorite from chloride ions.

In an embodiment, the haloperoxidase of the invention is a chloroperoxidase. Preferably, the haloperoxidase is a vanadium haloperoxidase, *i.e.,* a vanadate-containing haloperoxidase. In a preferred method of the present invention the vanadate-containing haloperoxidase is combined with a source of chloride ion.

Haloperoxidases have been isolated from many different fungi, in particular from the fungus group dematiaceous hyphomycetes, such as *Caldariomyces*, *e.g*., *C. fumago*, *Alternaria*, *Curvularia, e.g., C. verruculosa* and *C. inaequalis, Drechslera, Ulocladium* and *Botrytis.*

Haloperoxidases have also been isolated from bacteria such as *Pseudomonas, e.g., P. pyrrocinia* and *Streptomyces, e.g., S. aureofaciens.*

In a preferred embodiment, the haloperoxidase is derivable from *Curvularia* sp., in particular *Curvularia verruculosa* or *Curvularia inaequalis,* such as *C. inaequalis* CBS 102.42 as described in WO 95/27046; or *C. verruculosa* CBS 147.63 or *C. verruculosa* CBS 444.70 as described in WO 97/04102; or from *Drechslera hartlebii* as described in WO 01/79459, *Dendryphiella salina* as described in WO 01/79458, *Phaeotrichoconis crotalarie* as described in WO 01/79461, or *Geniculosporium* sp. as described in WO 01/79460.

An oxidase according to the invention include, in particular, any laccase enzyme comprised by the enzyme classification EC 1.10.3.2, or any fragment derived therefrom exhibiting laccase activity, or a compound exhibiting a similar activity, such as a catechol oxidase (EC 1.10.3.1), an o-aminophenol oxidase (EC 1.10.3.4), or a bilirubin oxidase (EC 1.3.3.5).

Preferred laccase enzymes are enzymes of microbial origin. The enzymes may be derived from plants, bacteria or fungi (including filamentous fungi and yeasts).

Suitable examples from fungi include a laccase derivable from a strain of *Aspergillus, Neurospora*, *e.g*., *N. crassa*, *Podospora*, *Botrytis*, *Collybia*, *Fomes*, *Lentinus*, *Pleurotus*, *Trametes, e.g., T. villosa* and *T. versicolor, Rhizoctonia, e.g., R. solani, Coprinopsis, e.g., C. cinerea, C. comatus, C. friesii*, and *C. plicatilis*, *Psathyrella*, *e.g*., *P. condelleana*,

*Panaeolus*, *e.g., P. papilionaceus, Myceliophthora, e.g., M. thermophila, Schytalidium, e.g., S. thermophilum, Polyporus, e.g., P. pinsitus, Phlebia, e.g., P. radiata* (WO 92/01046), or *Coriolus, e.g., C. hirsutus* (JP 2238885).

Suitable examples from bacteria include a laccase derivable from a strain of *Bacillus.*

A laccase derived from *Coprinopsis* or *Myceliophthora* is preferred; in particular a laccase derived from *Coprinopsis cinerea,* as disclosed in WO 97/08325; or from *Myceliophthora thermophila,* as disclosed in WO 95/33836.

The detergent enzyme(s) may be included in a detergent composition by adding separate additives containing one or more enzymes, or by adding a combined additive comprising all of these enzymes. A detergent additive of the invention, *i.e.,* a separate additive or a combined additive, can be formulated, for example, as a granulate, liquid, slurry, etc. Preferred detergent additive formulations are granulates, in particular non-dusting granulates, liquids, in particular stabilized liquids, or slurries.

Non-dusting granulates may be produced, *e.g.* as disclosed in US 4,106,991 and 4,661,452 and may optionally be coated by methods known in the art. Examples of waxy coating materials are polyethyleneglycol (PEG) with mean molar weights of 1000 to 20000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GB 1483591. Liquid enzyme preparations may, for instance, be stabilized by adding a polyol such as propylene glycol, a sugar or sugar alcohol, lactic acid or boric acid according to established methods. Protected enzymes may be prepared according to the method disclosed in EP 238,216.

### *Adjunct materials*

Any detergent components known in the art for use in laundry cleaning detergents may also be utilized. Other optional detergent components include anti-corrosion agents, anti-shrink agents, anti-soil redeposition agents, anti-wrinkling agents, bactericides, binders, corrosion inhibitors, disintegrants/disintegration agents, dyes, enzyme stabilizers (including boric acid, borates, CMC, and/or polyols such as propylene glycol), fabric conditioners including clays, fillers/processing aids, fluorescent whitening agents/optical brighteners, foam boosters, foam (suds) regulators, perfumes, soil-suspending agents, softeners, suds suppressors, tarnish inhibitors, and wicking agents, either alone or in combination. Any ingredient known in the art for use in laundry cleaning detergents may be utilized. The choice of such ingredients is well within the skill of the artisan.

### *Dispersants*

The detergent compositions of the present invention can also contain dispersants. In particular powdered detergents may comprise dispersants. Suitable water-soluble organic materials include the homo- or co-polymeric acids or their salts, in which the polycarboxylic acid comprises at least two carboxyl radicals separated from each other by not more than two carbon atoms. Suitable dispersants are for example described in Powdered Detergents, Surfactant science series volume 71, Marcel Dekker, Inc.

### *Dye Transfer Inhibiting Agents*

The detergent compositions of the present invention may also include one or more dye transfer inhibiting agents. Suitable polymeric dye transfer inhibiting agents include, but are not limited to, polyvinylpyrrolidone polymers, polyamine *N*-oxide polymers, copolymers of *N*-vinylpyrrolidone and *N*-vinylimidazole, polyvinyloxazolidones and polyvinylimidazoles or mixtures thereof. When present in a subject composition, the dye transfer inhibiting agents may be present at levels from about 0.0001 % to about 10%, from about 0.01% to about 5% or even from about 0.1% to about 3% by weight of the composition.

### *Fluorescent whitening agent*

The detergent compositions of the present invention will preferably also contain additional components that may tint articles being cleaned, such as fluorescent whitening agent or optical brighteners. Where present the brightener is preferably at a level of about 0.01% to about 0.5%. Any fluorescent whitening agent suitable for use in a laundry detergent composition may be used in the composition of the present invention. The most commonly used fluorescent whitening agents are those belonging to the classes of diaminostilbene-sulfonic acid derivatives, diarylpyrazoline derivatives and bisphenyl-distyryl derivatives. Examples of the diaminostilbene-sulfonic acid derivative type of fluorescent whitening agents include the sodium salts of: 4,4'-bis-(2-diethanolamino-4-anilino-s-triazin-6-ylamino) stilbene-2,2'-disulfonate, 4,4'-bis-(2,4-dianilino-s-triazin-6-ylamino) stilbene-2.2'-disulfonate, 4,4'-bis-(2-anilino-4-(*N*-methyl-*N*-2-hydroxy-ethylamino)-s-triazin-6-ylamino) stilbene-2,2'-disulfonate, 4,4'-bis-(4-phenyl-1,2,3-triazol-2-yl)stilbene-2,2'-disulfonate and sodium 5-(2*H*-naphtho[1,2-*d*][1,2,3]triazol-2-yl)-2-[(*E*)-2-phenylvinyl]benzenesulfonate. Preferred fluorescent whitening agents are Tinopal DMS and Tinopal CBS available from Ciba-Geigy AG, Basel, Switzerland. Tinopal DMS is the disodium salt of 4,4'-bis-(2-morpholino-4-anilino-s-triazin-6-ylamino) stilbene-2,2'-disulfonate. Tinopal CBS is the disodium salt of 2,2'-bis-(phenyl-styryl)-disulfonate. Also preferred are fluorescent whitening agents is the commercially available Parawhite KX, supplied by Paramount Minerals and Chemicals, Mumbai, India. Other fluorescers suitable for use in the invention include the 1-3-diaryl pyrazolines and the 7-alkylaminocoumarins.

Suitable fluorescent brightener levels include lower levels of from about 0.01, from 0.05, from about 0.1 or even from about 0.2 wt % to upper levels of 0.5 or even 0.75 wt%.

***Soil release polymers***

The detergent compositions of the present invention may also include one or more soil release polymers which aid the removal of soils from fabrics such as cotton and polyester based fabrics, in particular the removal of hydrophobic soils from polyester based fabrics. The soil release polymers may for example be nonionic or anionic terephthalte based polymers, polyvinyl caprolactam and related copolymers, vinyl graft copolymers, polyester polyamides see for example Chapter 7 in Powdered Detergents, Surfactant science series volume 71, Marcel Dekker, Inc. Another type of soil release polymers are amphiphilic alkoxylated grease cleaning polymers comprising a core structure and a plurality of alkoxylate groups attached to that core structure. The core structure may comprise a polyalkylenimine structure or a polyalkanolamine structure as described in detail in WO 2009/087523. Furthermore random graft co-polymers are suitable soil release polymers. Suitable graft co-polymers are described in more detail in WO 2007/138054, WO 2006/108856 and WO 2006/113314. Other soil release polymers are substituted polysaccharide structures especially substituted cellulosic structures such as modified cellulose deriviatives such as those described in EP 1867808 or WO 2003/040279. Suitable cellulosic polymers include cellulose, cellulose ethers, cellulose esters, cellulose amides and mixtures thereof. Suitable cellulosic polymers include anionically modified cellulose, nonionically modified cellulose, cationically modified cellulose, zwitterionically modified cellulose, and mixtures thereof. Suitable cellulosic polymers include methyl cellulose, carboxy methyl cellulose, ethyl cellulose, hydroxyl ethyl cellulose, hydroxyl propyl methyl cellulose, ester carboxy methyl cellulose, and mixtures thereof.

***Anti-redeposition agents***

The detergent compositions of the present invention may also include one or more anti-redeposition agents such as carboxymethylcellulose (CMC), polyvinyl alcohol (PVA), polyvinylpyrrolidone (PVP), polyoxyethylene and/or polyethyleneglycol (PEG), homopolymers of acrylic acid, copolymers of acrylic acid and maleic acid, and ethoxylated polyethyleneimines. The cellulose based polymers described under soil release polymers above may also function as anti-redeposition agents.

**Rheology Modifiers**

The detergent compositions of the present invention may also include one or more rheology modifiers, structurants or thickeners, as distinct from viscosity reducing agents. The rheology modifiers are selected from the group consisting of non-polymeric crystalline, hydroxyfunctional materials, polymeric rheology modifiers which impart shear thinning characteristics to the aqueous liquid matrix of a liquid detergent composition. The rheology and viscosity of the detergent can be modified and adjusted by methods known in the art, for example as shown in EP 2169040.

Other suitable adjunct materials include, but are not limited to, anti-shrink agents, anti-wrinkling agents, bactericides, binders, carriers, dyes, enzyme stabilizers, fabric softeners, fillers, foam regulators, hydrotropes, perfumes, pigments, sod suppressors, solvents, and structurants for liquid detergents and/or structure elasticizing agents.

**Formulation of detergent products**

The detergent composition of the invention may be in any convenient form, e.g., a bar, a homogenous tablet, a tablet having two or more layers, a pouch having one or more compartments, a regular or compact powder, a granule, a paste, a gel, or a regular, compact or concentrated liquid.

Pouches can be configured as single or multicompartments. It can be of any form, shape and material which is suitable for hold the composition, e.g. without allowing the release of the composition to release of the composition from the pouch prior to water contact. The pouch is made from water soluble film which encloses an inner volume. Said inner volume can be divided into compartments of the pouch. Preferred films are polymeric materials preferably polymers which are formed into a film or sheet. Preferred polymers, copolymers or derivates thereof are selected polyacrylates, and water soluble acrylate copolymers, methyl cellulose, carboxy methyl cellulose, sodium dextrin, ethyl cellulose,

hydroxyethyl cellulose, hydroxypropyl methyl cellulose, malto dextrin, poly methacrylates, most preferably polyvinyl alcohol copolymers and, hydroxypropyl methyl cellulose (HPMC). Preferably the level of polymer in the film for example PVA is at least about 60%. Preferred average molecular weight will typically be about 20,000 to about 150,000. Films can also be of blended compositions comprising hydrolytically degradable and water soluble polymer blends such as polylactide and polyvinyl alcohol (known under the Trade reference M8630 as sold by MonoSol LLC, Indiana, USA) plus plasticisers like glycerol, ethylene glycerol, propylene glycol, sorbitol and mixtures thereof. The pouches can comprise a solid laundry cleaning composition or part components and/or a liquid cleaning composition or part components separated by the water soluble film. The compartment for liquid components can be different in composition than compartments containing solids: US2009/0011970 A1.

Detergent ingredients can be separated physically from each other by compartments in water dissolvable pouches or in different layers of tablets. Thereby negative storage interaction between components can be avoided. Different dissolution profiles of each of the compartments can also give rise to delayed dissolution of selected components in the wash solution.

A liquid or gel detergent, which is not unit dosed, may be aqueous, typically containing at least 20% by weight and up to 95% water, such as up to about 70% water, up to about 65% water, up to about 55% water, up to about 45% water, up to about 35% water. Other types of liquids, including without limitation, alkanols, amines, diols, ethers and polyols may be included in an aqueous liquid or gel. An aqueous liquid or gel detergent may contain from 0-30% organic solvent.

A liquid or gel detergent may be non-aqueous.

**Laundry soap bars**

The enzyme preparation of the invention may be added to laundry soap bars and used for hand washing laundry, fabrics and/or textiles. The term laundry soap bar includes laundry bars, soap bars, combo bars, syndet bars and detergent bars. The types of bar usually differ in the type of surfactant they contain, and the term laundry soap bar includes those containing soaps from fatty acids and/or synthetic soaps. The laundry soap bar has a physical form which is solid and not a liquid, gel or a powder at room temperature. The term solid is defined as a physical form which does not significantly change over time, i.e. if a solid object (e.g. laundry soap bar) is placed inside a container, the solid object does not change to fill the container it is placed in. The bar is a solid typically in bar form but can be in other solid shapes such as round or oval.

The laundry soap bar may contain one or more additional enzymes, protease inhibitors such as peptide aldehydes (or hydrosulfite adduct or hemiacetal adduct), boric acid, borate, borax and/or phenylboronic acid derivatives such as 4-formylphenylboronic acid, one or more soaps or synthetic surfactants, polyols such as glycerine, pH controlling compounds such as fatty acids, citric acid, acetic acid and/or formic acid, and/or a salt of a monovalent cation and an organic anion wherein the monovalent cation may be for example $Na^+$, $K^+$ or $NH_4^+$ and the organic anion may be for example formate, acetate, citrate or lactate such that the salt of a monovalent cation and an organic anion may be, for example, sodium formate.

The laundry soap bar may also contain complexing agents like EDTA and HEDP, perfumes and/or different type of fillers, surfactants e.g. anionic synthetic surfactants, builders, polymeric soil release agents, detergent chelators, stabilizing agents, fillers, dyes, colorants, dye transfer inhibitors, alkoxylated polycarbonates, suds suppressers, structurants, binders, leaching agents, bleaching activators, clay soil removal agents, anti-redeposition agents, polymeric dispersing agents, brighteners, fabric softeners, perfumes and/or other compounds known in the art.

The laundry soap bar may be processed in conventional laundry soap bar making equipment such as but not limited to: mixers, plodders, e.g a two stage vacuum plodder, extruders, cutters, logo-stampers, cooling tunnels and wrappers. The invention is not limited to preparing the laundry soap bars by any single method. The premix of the invention may be added to the soap at different stages of the process. For example, the premix containing a soap, enzyme preparation, optionally one or more additional enzymes, a protease inhibitor, and a salt of a monovalent cation and an organic anion may be prepared and and the mixture is then plodded. The enzyme preparation and optional additional enzymes may be added at the same time as the protease inhibitor for example in liquid form. Besides the mixing step and the plodding step, the process may further comprise the steps of milling, extruding, cutting, stamping, cooling and/or wrapping.

**Formulation of enzyme in co-granule**

The enzyme of the invention may be formulated as a granule for example as a co-granule that combines one or more enzymes. Each enzyme will then be present in more granules securing a more uniform distribution of enzymes in the detergent. This also reduces the physical segregation of different enzymes due to different particle sizes. Methods for producing multi-enzyme co-granulates for the detergent industry are disclosed in the IP.com disclosure

IPCOM000200739D.

Another example of formulation of enzymes by the use of co-granulates are disclosed in WO 2013/188331, which relates to a detergent composition comprising (a) a multi-enzyme co-granule; (b) less than 10 wt zeolite (anhydrous basis); and (c) less than 10 wt phosphate salt (anhydrous basis), wherein said enzyme co-granule comprises from 10 to 98 wt% moisture sink component and the composition additionally comprises from 20 to 80 wt% detergent moisture sink component.

WO 2013/188331 also relates to a method of treating and/or cleaning a surface, preferably a fabric surface comprising the steps of (i) contacting said surface with the detergent composition as claimed and described herein in an aqueous wash liquor, (ii) rinsing and/or drying the surface.

The multi-enzyme co-granule may comprise an enzyme of the invention and (a) one or more enzymes selected from the group consisting of first- wash lipases, cleaning cellulases, xyloglucanases, perhydrolases, peroxidases, lipoxygenases, laccases and mixtures thereof; and (b) one or more enzymes selected from the group consisting of hemicellulases, proteases, care cellulases, cellobiose dehydrogenases, xylanases, phospho lipases, esterases, cutinases, pectinases, mannanases, pectate lyases, keratinases, reductases, oxidases, phenoloxidases, ligninases, pullulanases, tannases, pentosanases, lichenases glucanases, arabinosidases, hyaluronidase, chondroitinase, amylases, and mixtures thereof.

**The invention is further summarized in the following paragraphs:**

1. Use of a laundry detergent composition comprising an enzyme preparation comprising one or more enzymes capable of degrading cellulosic material and a surfactant or a fabric softener agent, wherein the one or more enzymes capable of degrading cellulosic material comprises :

    (i) an Aspergillus fumigatus cellobiohydrolase I;
    (ii) an Aspergillus fumigatus cellobiohydrolase II;
    (iii) an Aspergillus fumigatus beta-glucosidase or variant thereof; and
    (iv) a Penicillium sp. GH61 polypeptide having cellulolytic enhancing activity; or homologs thereof;

    and wherein the composition is used for preventing build-up and/or removal of fuzz and pills from a textile and for improving the whiteness of the textile by exposing the textile to the composition during a laundry process.

2. Use according to paragraph 1, wherein the *Aspergillus fumigatus* cellobiohydrolase I or homolog thereof of the enzyme preparation is selected from the group consisting of:

    (i) a cellobiohydrolase I comprising or consisting of the mature polypeptide of SEQ ID NO: 2;
    (ii) a cellobiohydrolase I comprising or consisting of an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ ID NO: 2;
    (iii) a cellobiohydrolase I encoded by a polynucleotide comprising or consisting of a nucleotide sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide coding sequence of SEQ ID NO: 1; and
    (iv) a cellobiohydrolase I encoded by a polynucleotide that hybridizes under at least high stringency conditions, very high stringency conditions, with the mature polypeptide coding sequence of SEQ ID NO: 1 or the full-length complement thereof;

    wherein the *Aspergillus fumigatus* cellobiohydrolase II or homolog thereof is selected from the group consisting of:

    (i) a cellobiohydrolase II comprising or consisting of the mature polypeptide of SEQ ID NO: 4;
    (ii) a cellobiohydrolase II comprising or consisting of an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ ID NO: 4;

(iii) a cellobiohydrolase II encoded by a polynucleotide comprising or consisting of a nucleotide sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide coding sequence of SEQ ID NO: 3; and
(iv) a cellobiohydrolase II encoded by a polynucleotide that hybridizes under at least high stringency conditions, very high stringency conditions, with the mature polypeptide coding sequence of SEQ ID NO: 3 or the full-length complement thereof;

wherein the *Aspergillus fumigatus* beta-glucosidase or homolog thereof is selected from the group consisting of:

(i) a beta-glucosidase comprising or consisting of the mature polypeptide of SEQ ID NO: 6;
(ii) a beta-glucosidase comprising or consisting of an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ ID NO: 6;
(iii) a beta-glucosidase encoded by a polynucleotide comprising or consisting of a nucleotide sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide coding sequence of SEQ ID NO: 5;
(iv) a beta-glucosidase encoded by a polynucleotide that hybridizes under at least high stringency conditions, very high stringency conditions, with the mature polypeptide coding sequence of SEQ ID NO: 5 or the full-length complement thereof; and
(v) a beta-glucosidase variant comprising a substitution at one or more positions corresponding to positions 100, 283, 456, and 512 of the mature polypeptide of SEQ ID NO: 6, wherein the variant has beta-glucosidase activity; and

wherein the *Penicillium* sp. GH61 polypeptide having cellulolytic enhancing activity or homolog thereof is selected from the group consisting of:

(i) a GH61 polypeptide having cellulolytic enhancing activity comprising or consisting of the mature polypeptide of SEQ ID NO: 8;
(ii) a GH61 polypeptide having cellulolytic enhancing activity comprising or consisting of an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ ID NO: 8;
(iii) a GH61 polypeptide having cellulolytic enhancing activity encoded by a polynucleotide comprising or consisting of a nucleotide sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide coding sequence of SEQ ID NO: 7; and
(iv) a GH61 polypeptide having cellulolytic enhancing activity encoded by a polynucleotide that hybridizes under at least high stringency conditions, very high stringency conditions, with the mature polypeptide coding sequence of SEQ ID NO: 7 or the full-length complement thereof.

3. Use according to any of the preceding paragraphs, wherein the beta-glucosidase variant of the enzyme preparation comprises one or more (several) substitutions selected from the group consisting of G142S, Q183R, H266Q, and D703G.
4. Use according to any of the preceding paragraphs, wherein the enzyme preparation further comprises one or more enzymes selected from the group consisting of:

(i) an *Aspergillus fumigatus* xylanase or homolog thereof,
(ii) an *Aspergillus fumigatus* beta-xylosidase or homolog thereof; or
(iii) a combination of (i) and (ii);

wherein the *Aspergillus fumigatus* xylanase or homolog thereof is selected from the group consisting of:

(i) an *Aspergillus fumigatus* xylanase comprising or consisting of the mature polypeptide of SEQ ID NO: 10, SEQ ID NO: 12, or SEQ ID NO: 14;
(ii) a xylanase comprising or consisting of an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ ID NO: 10, SEQ ID NO: 12, or SEQ ID NO: 14;
(iii) a xylanase encoded by a polynucleotide comprising or consisting of a nucleotide sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide coding sequence of SEQ ID NO: 9, SEQ ID NO: 11, or SEQ ID NO: 13; and
(iv) a xylanase encoded by a polynucleotide that hybridizes under at least high stringency conditions, very high stringency conditions, with the mature polypeptide coding sequence of SEQ ID NO: 9, SEQ ID NO: 11, or SEQ ID NO: 13; or the full-length complement thereof; and

wherein the *Aspergillus fumigatus* beta-xylosidase or homolog thereof is selected from the group consisting of:

(i) beta-xylosidase comprising or consisting of the mature polypeptide of SEQ ID NO: 16;
(ii) a beta-xylosidase comprising or consisting of an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ ID NO: 16;
(iii) a beta-xylosidase encoded by a polynucleotide comprising or consisting of a nucleotide sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide coding sequence of SEQ ID NO: 15; and
(iv) a beta-xylosidase encoded by a polynucleotide that hybridizes under at least high stringency conditions, very high stringency conditions, with the mature polypeptide coding sequence of SEQ ID NO: 15 or the full-length complement thereof.

5. Use according to any of the preceding paragraphs, wherein the composition comprises at least one enzyme in addition to the enzymes in the enzyme preparation.
6. Use according to any of the preceding paragraphs, wherein the at least one more enzyme is selected from the group consisting of hemicellulases, peroxidases, proteases, cellulases, xylanases, lipases, phospholipases, esterases, cutinases, pectinases, mannanases, pectate lyases, keratinases, reductases, oxidases, phenoloxidases, lipoxygenases, ligninases, pullulanases, tannases, pentosanases, malanases, β-glucanases, arabinosidases, hyaluronidase, chondroitinase, laccase, DNase chlorophyllases, amylases, perhydrolases, peroxidases, xanthanase and mixtures thereof.
7. Use according to paragraph 6, wherein the at least one more enzyme is selected from the group consisting of proteases, lipases, mannanases, pectate lyases, amylases or mixtures thereof.
8. Use according to any of the preceding paragraphs, wherein the surfactant is selected from the group consisting of anionic, cationic, non-ionic, semi-polar and zwitterionic surfactants.
9. Use according to any of the preceding paragraphs, wherein the composition further comprises one or more builders and one or more polymers.
10. Use according to any of the preceding composition paragraphs, wherein the composition further comprises one or more components selected from the group consisting of polymers, bleaching systems, bleach activators, bleach catalysts, silicates and dyestuff..
11. Use according to any of the preceding paragraphs, wherein the composition further comprises an acidic material.
12. Use according to any of the preceding paragraphs, wherein the acidic material is selected from the group consisting of citric acid, acetic acid, potassium dihydrogen phosphate, boric acid, diethyl barbituric acid, Carmody buffer and Britton-Robinson buffer
13. Use according to any of the preceding paragraphs, wherein the composition is in the form of a powder, a bar, a homogenous tablet, a tablet having two or more layers, a pouch having one or more compartments, a regular or

compact powder, a granule, a paste, a gel, or a regular, compact or concentrated liquid.

14. Use according to any of the preceding paragraphs, wherein the composition is a powder or a granule and the acidic material is the outer layer of the powder granules.

15. Use according to any of the preceding paragraphs, wherein the composition is a tablet having two or more layers, wherein the acidic material is the outer layer of the bar.

16. Use according to any of the preceding paragraphs, wherein the composition is a pouch having at least two compartments, wherein the acidic material is present in one compartment and is released before content of the other compartment(s).

17. Use according to any of the preceding paragraphs, wherein the composition is for used in house hold laundry or industrial laundry.

18. A method for laundering a textile comprising the steps of:

e) Contacting the textile with a wash liquor comprising laundry detergent composition;
f) Completing at least one wash cycle;
g) contacting the textile with water comprising a fabric softener composition; and
h) completing at least one rinse cycle;

wherein the detergent composition and/or the fabric softener composition comprise
an enzyme preparation comprising one or more enzymes capable of degrading cellulosic material and wherein the one or more enzymes capable of degrading cellulosic material comprises:

v. an Aspergillus fumigatus cellobiohydrolase I;
vi. an Aspergillus fumigatus cellobiohydrolase II;
vii. an Aspergillus fumigatus beta-glucosidase or variant thereof; and
viii. a Penicillium sp. GH61 polypeptide having cellulolytic enhancing activity; or homologs thereof.; and

wherein the method prevents build-up and/or removal of fuzz and pills from a textile and improves the whiteness of the textile.

19. Method according to paragraph 18, wherein the method further comprises exposing the textile to an acidic solution.

20. Method according to paragraph 19, wherein the acidic solution having a pH below 5 is exposed to the textile during a pre-wash or during rinsing.

21. Method according to any of the preceding method paragraphs, wherein the laundry method is carried out by hand or machine.

22. Method according to any of paragraphs 18-22, wherein the concentration of the enzyme preparation is in the range of 0.03 to 5 gram/liter wash liquor.

23. Use of an enzyme preparation for cleaning, wherein the enzyme preparation comprises one or more enzymes capable of degrading cellulosic material, wherein enzyme preparation comprises:

(i) an Aspergillus fumigatus cellobiohydrolase I;
(ii) an Aspergillus fumigatus cellobiohydrolase II;
(iii) an Aspergillus fumigatus beta-glucosidase or variant thereof; and
(iv) a Penicillium sp. GH61 polypeptide having cellulolytic enhancing activity; or homologs thereof.

24. Use according to paragraph 23 for laundering of textile or cleaning of the interior of a washing machine such as walls, nozzles, pumps, sump, filters, pipelines, drains, and outlets.

25. Use according to any of the preceding use paragraphs, wherein the *Aspergillus fumigatus* cellobiohydrolase I or homolog thereof of the enzyme preparation is selected from the group consisting of:

(i) a cellobiohydrolase I comprising or consisting of the mature polypeptide of SEQ ID NO: 2;
(ii) a cellobiohydrolase I comprising or consisting of an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ ID NO: 2;
(iii) a cellobiohydrolase I encoded by a polynucleotide comprising or consisting of a nucleotide sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the

mature polypeptide coding sequence of SEQ ID NO: 1; and
(iv) a cellobiohydrolase I encoded by a polynucleotide that hybridizes under at least high stringency conditions, very high stringency conditions, with the mature polypeptide coding sequence of SEQ ID NO: 1 or the full-length complement thereof;

wherein the *Aspergillus fumigatus* cellobiohydrolase II or homolog thereof is selected from the group consisting of:

(i) a cellobiohydrolase II comprising or consisting of the mature polypeptide of SEQ ID NO: 4;
(ii) a cellobiohydrolase II comprising or consisting of an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ ID NO: 4;
(iii) a cellobiohydrolase II encoded by a polynucleotide comprising or consisting of a nucleotide sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide coding sequence of SEQ ID NO: 3; and
(iv) a cellobiohydrolase II encoded by a polynucleotide that hybridizes under at least high stringency conditions, very high stringency conditions, with the mature polypeptide coding sequence of SEQ ID NO: 3 or the full-length complement thereof;

wherein the *Aspergillus fumigatus* beta-glucosidase or homolog thereof is selected from the group consisting of:

(i) a beta-glucosidase comprising or consisting of the mature polypeptide of SEQ ID NO: 6;
(ii) a beta-glucosidase comprising or consisting of an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ ID NO: 6;
(iii) a beta-glucosidase encoded by a polynucleotide comprising or consisting of a nucleotide sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide coding sequence of SEQ ID NO: 5;
(iv) a beta-glucosidase encoded by a polynucleotide that hybridizes under at least high stringency conditions, very high stringency conditions, with the mature polypeptide coding sequence of SEQ ID NO: 5 or the full-length complement thereof; and
(v) a beta-glucosidase variant comprising a substitution at one or more positions corresponding to positions 100, 283, 456, and 512 of the mature polypeptide of SEQ ID NO: 6, wherein the variant has beta-glucosidase activity; and

wherein the *Penicillium* sp. GH61 polypeptide having cellulolytic enhancing activity or homolog thereof is selected from the group consisting of:

(i) a GH61 polypeptide having cellulolytic enhancing activity comprising or consisting of the mature polypeptide of SEQ ID NO: 6;
(ii) a GH61 polypeptide having cellulolytic enhancing activity comprising or consisting of an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ ID NO: 8;
(iii) a GH61 polypeptide having cellulolytic enhancing activity encoded by a polynucleotide comprising or consisting of a nucleotide sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide coding sequence of SEQ ID NO: 7; and
(iv) a GH61 polypeptide having cellulolytic enhancing activity encoded by a polynucleotide that hybridizes under

at least high stringency conditions, very high stringency conditions, with the mature polypeptide coding sequence of SEQ ID NO: 7 or the full-length complement thereof.

26. Use according to any of the preceding use paragraphs, wherein the beta-glucosidase variant of the enzyme preparation comprises one or more (several) substitutions selected from the group consisting of G142S, Q183R, H266Q, and D703G.
27. Use according to any of the preceding use paragraphs, wherein the enzyme preparation further comprises one or more enzymes selected from the group consisting of:

(i) an *Aspergillus fumigatus* xylanase or homolog thereof,
(ii) an *Aspergillus fumigatus* beta-xylosidase or homolog thereof; or
(iii) a combination of (i) and (ii);

wherein the *Aspergillus fumigatus* xylanase or homolog thereof is selected from the group consisting of:

(i) an *Aspergillus fumigatus* xylanase comprising or consisting of the mature polypeptide of SEQ ID NO: 10, SEQ ID NO: 12, or SEQ ID NO: 14;
(ii) a xylanase comprising or consisting of an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ ID NO: 10, SEQ ID NO: 12, or SEQ ID NO: 14;
(iii) a xylanase encoded by a polynucleotide comprising or consisting of a nucleotide sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide coding sequence of SEQ ID NO: 9, SEQ ID NO: 11, or SEQ ID NO: 13; and
(iv) a xylanase encoded by a polynucleotide that hybridizes under at least high stringency conditions, very high stringency conditions, with the mature polypeptide coding sequence of SEQ ID NO: 9, SEQ ID NO: 11, or SEQ ID NO: 13; or the full-length complement thereof; and

wherein the *Aspergillus fumigatus* beta-xylosidase or homolog thereof is selected from the group consisting of:

(i) beta-xylosidase comprising or consisting of the mature polypeptide of SEQ ID NO: 16;
(ii) a beta-xylosidase comprising or consisting of an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ ID NO: 16;
(iii) a beta-xylosidase encoded by a polynucleotide comprising or consisting of a nucleotide sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide coding sequence of SEQ ID NO: 15; and
(iv) a beta-xylosidase encoded by a polynucleotide that hybridizes under at least high stringency conditions, very high stringency conditions, with the mature polypeptide coding sequence of SEQ ID NO: 15 or the full-length complement thereof.

28. Use according to any of the preceding use paragraphs comprising the use of an acidic material.
29. A cleaning method for cleaning the interior of a washing machine, which method comprises exposing the interior of the washing machine to one or more enzymes capable of degrading cellulosic material.
30. Cleaning method according to paragraph 29, wherein the one or more enzymes capable of degrading cellulosic material is an enzyme preparation comprising:

(i) an Aspergillus fumigatus cellobiohydrolase I;
(ii) an Aspergillus fumigatus cellobiohydrolase II;
(iii) an Aspergillus fumigatus beta-glucosidase or variant thereof; and
(iv) a Penicillium sp. GH61 polypeptide having cellulolytic enhancing activity; or homologs thereof.

31. Cleaning method according to any of paragraphs 28-29, wherein the *Aspergillus fumigatus* cellobiohydrolase I or homolog thereof of the enzyme preparation is selected from the group consisting of:

(i) a cellobiohydrolase I comprising or consisting of the mature polypeptide of SEQ ID NO: 2;
(ii) a cellobiohydrolase I comprising or consisting of an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ ID NO: 2;
(iii) a cellobiohydrolase I encoded by a polynucleotide comprising or consisting of a nucleotide sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide coding sequence of SEQ ID NO: 1; and
(iv) a cellobiohydrolase I encoded by a polynucleotide that hybridizes under at least high stringency conditions, very high stringency conditions, with the mature polypeptide coding sequence of SEQ ID NO: 1 or the full-length complement thereof;

wherein the *Aspergillus fumigatus* cellobiohydrolase II or homolog thereof is selected from the group consisting of:

(v) a cellobiohydrolase II comprising or consisting of the mature polypeptide of SEQ ID NO: 4;
(vi) a cellobiohydrolase II comprising or consisting of an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ ID NO: 4;
(vii) a cellobiohydrolase II encoded by a polynucleotide comprising or consisting of a nucleotide sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide coding sequence of SEQ ID NO: 3; and
(viii) a cellobiohydrolase II encoded by a polynucleotide that hybridizes under at least high stringency conditions, very high stringency conditions, with the mature polypeptide coding sequence of SEQ ID NO: 3 or the full-length complement thereof;

wherein the *Aspergillus fumigatus* beta-glucosidase or homolog thereof is selected from the group consisting of:

(vi) a beta-glucosidase comprising or consisting of the mature polypeptide of SEQ ID NO: 6;
(vii) a beta-glucosidase comprising or consisting of an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ ID NO: 6;
(viii) a beta-glucosidase encoded by a polynucleotide comprising or consisting of a nucleotide sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide coding sequence of SEQ ID NO: 5;
(ix) a beta-glucosidase encoded by a polynucleotide that hybridizes under at least high stringency conditions, very high stringency conditions, with the mature polypeptide coding sequence of SEQ ID NO: 5 or the full-length complement thereof; and
(x) a beta-glucosidase variant comprising a substitution at one or more positions corresponding to positions 100, 283, 456, and 512 of the mature polypeptide of SEQ ID NO: 6, wherein the variant has beta-glucosidase activity; and

wherein the *Penicillium* sp. GH61 polypeptide having cellulolytic enhancing activity or homolog thereof is selected from the group consisting of:

(v) a GH61 polypeptide having cellulolytic enhancing activity comprising or consisting of the mature polypeptide of SEQ ID NO: 6;
(vi) a GH61 polypeptide having cellulolytic enhancing activity comprising or consisting of an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ ID NO: 8;
(vii) a GH61 polypeptide having cellulolytic enhancing activity encoded by a polynucleotide comprising or consisting of a nucleotide sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide coding sequence of SEQ ID NO: 7; and
(viii) a GH61 polypeptide having cellulolytic enhancing activity encoded by a polynucleotide that hybridizes under at least high stringency conditions, very high stringency conditions, with the mature polypeptide coding sequence of SEQ ID NO: 7 or the full-length complement thereof.

32. Cleaning method according to any of paragraphs 29-31, wherein the beta-glucosidase variant of the enzyme preparation comprises one or more (several) substitutions selected from the group consisting of G142S, Q183R, H266Q, and D703G.
33. Cleaning method according to any of paragraphs 29-32, wherein the enzyme preparation further comprises one or more enzymes selected from the group consisting of:

(i) an *Aspergillus fumigatus* xylanase or homolog thereof,
(ii) an *Aspergillus fumigatus* beta-xylosidase or homolog thereof; or
(iii) a combination of (i) and (ii);

wherein the *Aspergillus fumigatus* xylanase or homolog thereof is selected from the group consisting of:

(v) an *Aspergillus fumigatus* xylanase comprising or consisting of the mature polypeptide of SEQ ID NO: 10, SEQ ID NO: 12, or SEQ ID NO: 14;
(vi) a xylanase comprising or consisting of an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ ID NO: 10, SEQ ID NO: 12, or SEQ ID NO: 14;
(vii) a xylanase encoded by a polynucleotide comprising or consisting of a nucleotide sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide coding sequence of SEQ ID NO: 9, SEQ ID NO: 11, or SEQ ID NO: 13; and
(viii) a xylanase encoded by a polynucleotide that hybridizes under at least high stringency conditions, very high stringency conditions, with the mature polypeptide coding sequence of SEQ ID NO: 9, SEQ ID NO: 11, or SEQ ID NO: 13; or the full-length complement thereof; and

wherein the *Aspergillus fumigatus* beta-xylosidase or homolog thereof is selected from the group consisting of:

(v) beta-xylosidase comprising or consisting of the mature polypeptide of SEQ ID NO: 16;
(vi) a beta-xylosidase comprising or consisting of an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ ID NO: 16;
(vii) a beta-xylosidase encoded by a polynucleotide comprising or consisting of a nucleotide sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide coding sequence of SEQ ID NO: 15; and
(viii) a beta-xylosidase encoded by a polynucleotide that hybridizes under at least high stringency conditions,

very high stringency conditions, with the mature polypeptide coding sequence of SEQ ID NO: 15 or the full-length complement thereof.

34. Cleaning method according to any of paragraphs 29-34, wherein the method comprises exposing the interior of the washing machine to an aqueous solution of an acidic material.
35. Cleaning method according to any of paragraphs 29-35, wherein the method is carried out at the same time as washing a textile in the washing machine.
36. Cleaning method according to paragraph 29-36, wherein the enzyme capable of degrading cellulosic material is comprised in the composition of paragraphs 1-17.
37. Use according to any of claims 1-17, wherein the composition is a laundry detergent composition or a fabric softener composition.
38. Use according to any of claims 1-3, 5-17 and 37, wherein the composition comprises

(i) an Aspergillus fumigatus cellobiohydrolase I comprising or consisting of the mature polypeptide of SEQ ID NO: 2;
(ii) an Aspergillus fumigatus cellobiohydrolase II comprising or consisting of the mature polypeptide of SEQ ID NO: 4;
(iii) an Aspergillus fumigatus beta-glucosidase comprising or consisting of the mature polypeptide of SEQ ID NO: 6; or a variant hereof comprising the following substitutions F100D, S283G, N456E and F512Y; and
(iv) a Penicillium sp. GH61 polypeptide having cellulolytic enhancing activity comprising or consisting of the mature polypeptide of SEQ ID NO: 8.

39. Method according to paragraph 22, wherein the concentration of the enzyme preparation is in the range of 0.05-4 gram/liter, in the range of 0.1-3 gram/liter, in the range of 0.2-2.5 gram/liter, in the range of 0.2-2 gram/liter or in the range of 0.3-1 gram/liter.
40. Method according to any of paragraphs 18-22, wherein the laundry method is carried out by hand or machine.
41. Method according to any of paragraphs 18-22, wherein the method is carried out at least 5 times.
42. Method according to paragraph 41, wherein the method is carried out at least 10 times, at least 15 times or at least 20 times.

**Laundry detergent compositions**

**Composition of Ariel Sensitive White & Color, liquid detergent composition:**

Aqua, Alcohol Ethoxy Sulfate, Alcohol Ethoxylate, Amino Oxide, Citrid Acid, C12-18 topped palm kernel fatty acid, Protease, Glycosidase, Amylase, Ethanol, 1,2 Propanediol, Sodium Formate, Calcium Chloride, Sodium hydroxide, Silicone Emulsion, Trans-sulphated EHDQ (the ingredients are listed in descending order).

**Brilhante HDL (Unilever, Brazil)**

Surfactant (LAS) 7%, Builders 5%, Fillers 86%, Polymers 0.5%, Thickner 1% and fragrance 0.5%. (w/w%)

**Composition of Persil Megaperls (powder)**

15 - 30 % of the following: anionic surfactants, oxygen-based bleaching agent and zeolites, less than 5 % of the following: non-ionic surfactants, phosphonates, polycarboxylates, soap, Further ingredients: Perfumes, Hexyl cinnamal, Benzyl salicylate, Linalool, optical brighteners, Enzymes and Citronellol.

**Fabric softener compositions**

The enzyme preparation of the invention can be comprised in the below mentioned fabric softener compositions and can be used for preventing build-up and/or removal of fuzz and pills from a textile and for improving the whiteness of the textile

Downy® Fabric Softener Dryer Sheets – all Variesties (Procter & Gamble)

Dipalmethyl Hydroxyethylammoinum Methosulfate, Fatty acid, Polyester Substrate, Clay and Fragrance.

**Gain Original Fresh Fabric Softener**

Water, Diethyl ester dimethyl ammonium chloride, Perfume, Calcium Chloride, Formic acid, Dimethicone copolymer, Liquitint™ Green, Hydrochloric Acid, Quaternary acrylate polymer, Ethoxylated cocoalkyl bis(2-hydroxyethyl) methyl ammonium chloride, Perfume microcapsules, Methylchoroisothiazolinone/Methylisothiazolinone, Diethylenetriamine pentaacetate (sodium salt).

**Bamseline Creations Jasmin & Blåbær (Unilever, Denmark)**

Aqua, Ditallowoylethyl Hydroxyethylmonium Methosulfate, Isopropyl alcohol, Perfume, Limonene, Butylphenyl Methylpropional, Coumarin, Alpha-Isomethyl Ionone, Polyoxymethylene Melamine, Dimethicone, Imidazolidinone, Benzisothiazolinone, Polymeric Pink Colourant, Etidronic Acid, Trimethylsiloxysilicate, Calcium chloride, Hydrogenated Vegetable Glycerides, Glycol Stearate, Cellulose Gum, Xanthan gum, Polymeric Blue Colourant, Iodopropynyl Butylcarbamate.

**Ype fabric softener**

4% cationic surfactant, 0.3% fatty acid, 0.1% formol 37%, 0.6% perfume, 0.3% color protector, 0.7% thickner and 94% water.

**EXAMPLES**

**Enzyme preparation 2 (Enz 2) capable of degrading cellulosic material used in example 1, 2 and 6.**

The enzyme preparation capable of degrading cellulosic material comprises a blend of an *Aspergillus fumigatus* GH10 xylanase and *Aspergillus fumigatus* beta-xylosidase with a *Trichoderma reesei* cellulase preparation containing *Aspergillus fumigatus* cellobiohydrolase I, *Aspergillus fumigatus* cellobiohydrolase II, *Aspergillus fumigatus* beta-glucosidase variant, and *Penicillium* sp. (*emersonii*) GH61 polypeptide. The enzyme preparation can be produced as described in examples 1-19 of WO 2013/028928.
This enzyme preparation is different that the enzyme preparation used in example 3, 4 and 5.

**Example 1**

Cellulose degrading experiments are performed in order to assess the wash performance - degradation - on cellulose based material like cellulose fibres. One application is to clean the filters and pipes in the laundry washing machine. The cellulose fibres used in this evaluation have been collected from two sources, one from the filter from tumble dryers, in this case Miele SOFTTRONIC T8627 WP with the program Automatic+ and where 75% cotton and 25% polyester had been tumble dried and another one from paper tissues; KIMCARE, Medical Wipes, code 3020 - Kimberly-Clark professional.

First the cellulose fibres are acid treated with 10 ml solution of citric acid (6 gram/liter) reaching a pH of about pH 2-3 in a beaker for about 10 minutes. After this, the cellulose fibres are placed on a sieve over a beaker for drying overnight at room temperature. The next day the dry cellulose fibres are placed in a beaker with water at 40°C and the enzyme preparation 2 capable of degrading cellulosic material with the amount 0.5 gram/liter, are added. After 30 minutes the fibers are taken up and dried again on a filter, the sieve over the beaker, overnight at room temperature. This process is repeated 5 times.

| Fiber weight loss, wt% loss | Fibres from tumble dryer Test 1 | Fibres from tumble dryer Test 2 | Paper tissue Test 1 | Paper tissue Test 2 |
|---|---|---|---|---|
| Only water | 18 | 22 | 4 | 9 |
| Enzyme preparation 2 capable of degrading cellulosic material | 35 | 26 | 39 | 42 |

**Example 2**

Tensile strength evaluation

Tensile strength evaluation is done in order to find out if the enzyme preparation 2 material used will degrade the cotton textiles too much so that it will be negative for a consumer using a commercial product containing the enzyme preparation 2 capable of degrading cellulosic. The tensile strength evaluation was performed at wfk Testgewebe GmbH, Germany. The determination of tensile strength is done according to ISO 13934 T01 and DIN 53919 part 2, see description below. Textile used is wfk 11A, 100% woven cotton.

**Determination of tensile strength loss according to DIN EN ISO 13934-1 and DIN 53919 part 2**

DIN 53919 part 1 describes the fabric construction of the wfk 11A and DIN 53919 part 2 describes how to prepare the specimen for each kind of test. DIN EN ISO 13934-1 describes testing conditions for tensile strength testing.

Repeated wash cycles usually reduce the tensile strength of the cotton control cloth due to the mechanical action and chemical damage during the cycles.

Any decrease in breaking strength expressed as a percentage of the initial breaking strength is determined from the variation in breaking strength measured in the direction of the warp of the control cloth before and after laundering.

The strips for testing tensile strength should be 30 cm (length/warp direction) x 6 cm (width). Wfk testgewebe GmbH uses 10 strips (30 x 6 cm) of the prepared control sheets (see page 3). The strips are cut along the outer green threads (see page 3). After cutting the strips, the outer green warp thread and the white warp threads between the outer and the inner green warp thread have to be pulled out on both sides of the strip; also the inner green warp thread should be pulled out. This leaves exactly 132 warp (white) threads remaining in each single test sample. The width of the samples is approx. 5 cm.

Because the width of the material may change (e.g. due to shrinkage) the number of white threads between the inner green warp threads are very important for comparing the washed values with the initial values.

After separating the 10 strips of one control sheet, the average and the standard deviation of tensile strength values of the control sheet are calculated. If strips tear along the clamps the result of that strips are invalid. If this occurs, new strips of the same laundry control sheet should be tested.

Testing conditions at wfk Testgewebe GmbH for testing tensile strength of laundry control sheet:

- -distance between the clamps: 200 mm
- rate of loading: 100 mm/min
- specimen length.30 cm
- Amount of load applied to the fabric before starting the tear tester: 1N
- testing in warp direction
- testing wet (the ten strips of one control sheet are treated in a bath of water with surface active agent for wetting the fabric - wetting time 1 hour)
- Number of strips per laundry control sheet minimum 10

The detergent used in this evaluation was model detergent B with two different cellulase preparations and without any enzyme preparation 2 for comparison.

Cellulase preparation 1: the enzyme preparation 2 capable of degrading cellulosic material: 1 wt%.
Cellulase preparation 2: a commercial cellulase of SEQ ID NO:17, 0.13 wt%
Model detergent B consisting of:

7% LAS
3% AEOS/SLES - alkyl ethoxylates / Sodium lauryl ether sulfate
6.6% Non-ionic surfactant
5.5% soap
Add up 100 % with water

Surfactant content: 16.6%

Model detergent B was dosed with 50g/wash at 15dH and 30°C in Miele Softtronic W2245,washing machine with washing time 1 h 26 minutes. 20 wash cycles were run for each of the two cellulase preparations with tumble drying (Miele SOFTTRONIC T8627 WP with the program Automatic+) in between every wash:

The wash performed without a cellulase preparation is used as baseline in terms of tensile strength.

The results shows the tensile strength of the textile washed with the enzyme preparation 2 capable of degrading cellulosic material gives about 16% strength loss after the 20 washes at whereas the commercial cellulase of SEQ ID NO:17 with gives about 22 % strength loss after 20 washes.

The conclusion is therefore that the enzyme preparation 2 capable of degrading cellulosic material is less aggressive towards the wfk 11A textile compared to the commercial cellulase of SEQ ID NO: 17.

**Example 3**

**SEQ ID NO: 17 vs Enz 1 - Full scale wash**

This is a multicycle test (20 wash cycles) used to compare performance of SEQ ID NO: 1 versus the enzyme preparation of the invention in full scale wash under Latin American conditions (washing in a top loader washing machine). Fabrics and ballast are added to each wash together with laundry detergent composition and enzymes. After wash, fabrics were line dried. Fabrics appearance was evaluated visually by a group panel.

Equipment used:

- Washing machine: BWL11A Brastemp

Ballast

The ballast consists of clean white cloth without optical whitener made of 100% cotton. The ballast weight, dryness and item composition must be the same in each wash. Ballast Example: (Standard LA ballast composition, total 1kg)

- 15 pieces 45 x 45 cm ballast 100% cotton

Wash conditions

**20 cycle test**

**Machine:** TOP Load BWL11A Brastemp
**Washing temperature (°C):** 25
**Washing cycle:** cycle "dia-a-dia" (normal)
1h30min washing cycle, with two rinsing steps.
**Detergent concentration (g/L):** 1,8 **HDL Brilhante (UL)**
**Water hardness (ppm):** 70 (tap water)
**Washing volume (L):** 35
**Rinsing volume (L) :** 35
**Rinsing time:** 15 minutes
**Ballast:** weight fabrics (see table 1) and complete ballast to **1kg**
**Enzyme dosage:** see table 2, below:

Table 1. Fabrics.

| Fabric | Size | Composition |
|---|---|---|
| Purple striped polycotton from malharia Fremetax | 10 pieces 15 x 15 cm | 50/50% cotton/polyester |
| Rayon elastane from malharia Fremetax | 10 pieces 15 x 15 cm | 96% rayon/ 4% elastane |
| Blue stripped polycotton from Malharia Fremetex | 10 pieces 15 x 15 cm | 50/50% cotton/polyester |
| Empa 252 from CFT | 10 pieces 10 x 10 cm | Standard cotton from CFT |
| Cotton socks from Hannes | 10 socks | 100% cotton |

Table 2. Enzyme dosage for the test.

| | Laundry Detergent composition | Enzyme % | Enzyme |
|---|---|---|---|
| A | HDL Brilhante | 0,3 | SEQ ID NO: 17 |
| B | HDL Brilhante | 0,5 | SEQ ID NO: 17 |
| C | HDL Brilhante | 0,5 | Enz 1 |
| D | HDL Brilhante | 1,0 | Enz 1 |
| E | HDL Brilhante | 0 | 0 |

Enzyme preparation 1 (Enz 1) comprises:

(i) an Aspergillus fumigatus cellobiohydrolase I comprising or consisting of the mature polypeptide of SEQ ID NO: 2;

(ii) an Aspergillus fumigatus cellobiohydrolase II comprising or consisting of the mature polypeptide of SEQ ID NO: 4;

(iii) an Aspergillus fumigatus beta-glucosidase comprising or consisting of the mature polypeptide of SEQ ID NO: 6; or a variant hereof comprising the following substitutions F100D, S283G, N456E and F512Y; and

(iv) a Penicillium sp. GH61 polypeptide having cellulolytic enhancing activity comprising or consisting of the mature polypeptide of SEQ ID NO: 8.

Detailed steps to carry out full scale wash trial:

1) Choose normal cycle (dia-a-dia) at machine, low volume (35L),

2) Start program to fill up the machines with water and adjust the temperature (25 C) manually.

3) Add detergent and enzymes/enzyme preparation directly to water (table 2)

4) Add ballast and fabrics - 2 pieces in each machine (table 1)

5) Close the machine lid and start the cycle.

6) When the cycle ends (after centrifugation), remove the ballast and separate for the next washing cycle.

7) Restart from step 1

8) Run 20 times steps #1 to #7.

9) After the $20^{th}$ cycle remove the fabrics and line dry in accordance with procedure.

Drying procedure

Hang the fabrics in line and dry at room temperature after the $20^{th}$ cycle. The room has a de-humidifier working for 24h per day to keep the room dry

Measurement

20 trained test persons evaluated the fabric by giving a note from 1 to 10 to each coded sample based on better appearance and renewal of the fibers, were 10 is the best one corresponding to appearance of newly produced fabric. Notes presented are the average to give a single rating number.

Five fabrics were tested:

Purple striped polycotton from Malharia Fremetex; Rayon elastane from malharia Fremetax; Cotton socks from Hannes ; Empa 252 from CFT ; Blue stripped polycotton from Malharia Fremetex.

Washing experiment

1.8g/L of HDL Brilhante was dissolved directly into machine with water hardness of 70 ppm (tap water). In formulas with SEQ ID NO: 17 or Enz 1, enzymes were added based on Table 2 directly to the wash liquor, w/w% from detergent. Furthermore, fabrics plus ballast were added in a total of 1kg. Add fabrics as prepared on table 1 (2 pieces of each fabric in each machine) and start the program as indicated on washing conditions above. After the 20$^{th}$ washing cycle, fabrics were line dried overnight. Measurement was made using group of people as panelists, given notes from 1 to 10 to each fabric (10 is the best, look as new), based on visual appearance compared to a new one.

Results

Based on sensory panel analysis, average of rates for each fabric and laundry detergent composition are shown on table 3, below:

Table 3. Influence of enzymatic treatment when applied to different fabrics after 20 wash cycles.

| **Detergent** | **Fabric** | **Enzyme** | **Rate** |
|---|---|---|---|
| HDL Brilhante | Purple striped polycotton from malharia Fremetax | 0,3% SEQ ID NO: 17 | 7 |
| HDL Brilhante | Purple striped polycotton from malharia Fremetax | 0,5% SEQ ID NO: 17 | 8,75 |
| HDL Brilhante | Purple striped polycotton from malharia Fremetax | 0,5% Enz 1 | 4,375 |
| HDL Brilhante | Purple striped polycotton from malharia Fremetax | 1% Enz 1 | 7,25 |
| HDL Brilhante | Purple striped polycotton from malharia Fremetax | no enzymes | 1,375 |
| HDL Brilhante | Rayon elastane from malharia Fremetax | 0,3% SEQ ID NO: 17 | 5 |
| HDL Brilhante | Rayon elastane from malharia Fremetax | 0,5% SEQ ID NO: 17 | 6,75 |
| HDL Brilhante | Rayon elastane from malharia Fremetax | 0,5% Enz 1 | 6,5 |
| HDL Brilhante | Rayon elastane from malharia Fremetax | 1% Enz 1 | 6,375 |
| HDL Brilhante | Rayon elastane from malharia Fremetax | no enzymes | 1,75 |
| HDL Brilhante | Blue stripped polycotton from Malharia Fremetex | 0,3% SEQ ID NO: 17 | 6,625 |
| HDL Brilhante | Blue stripped polycotton from Malharia Fremetex | 0,5% SEQ ID NO: 17 | 7,5 |
| HDL Brilhante | Blue stripped polycotton from Malharia Fremetex | 0,5% Enz 1 | 5,5 |
| HDL Brilhante | Blue stripped polycotton from Malharia Fremetex | 1% Enz 1 | 7,875 |
| HDL Brilhante | Blue stripped polycotton from Malharia Fremetex | no enzymes | 1,375 |
| HDL Brilhante | Empa 252 from CFT | 0,3% SEQ ID NO: 17 | 6,875 |
| HDL Brilhante | Empa 252 from CFT | 0,5% SEQ ID NO: 17 | 6,25 |

(continued)

| Detergent | Fabric | Enzyme | Rate |
|---|---|---|---|
| HDL Brilhante | Empa 252 from CFT | 0,5% Enz 1 | 5,75 |
| HDL Brilhante | Empa 252 from CFT | 1% Enz 1 | 7,125 |
| HDL Brilhante | Empa 252 from CFT | no enzymes | 1,625 |
| HDL Brilhante | Cotton socks from Hannes | 0,3% SEQ ID NO: 17 | 7,87 |
| HDL Brilhante | Cotton socks from Hannes | 0,5% SEQ ID NO: 17 | 7,75 |
| HDL Brilhante | Cotton socks from Hannes | 0,5% Enz 1 | 6,625 |
| HDL Brilhante | Cotton socks from Hannes | 1% Enz 1 | 6,375 |
| HDL Brilhante | Cotton socks from Hannes | no enzymes | 1,875 |

**Example 4**

**Enzyme preparation 1 - Full scale wash**

This is a multicycle test (20 wash cycles) used to prove performance (softness and fiber care) of Enzyme preparation 1 (Enz 1) in fabric softeners on full scale wash under latin american conditions (washing in a top loader washing machine). Fabrics and ballast are added to each wash together with laundry detergent composition, fabric softeners and enzymes. After wash, fabrics were line dried. Fabrics appearance was evaluated visually by a group panel.

The equipment used, the ballast and the wash conditions are the same as in example 3.

Table 4. Fabrics.

| Fabric | Size | Composition |
|---|---|---|
| Blue stripped polycotton from Malharia Fremetex | 10 pieces 15 x 15 cm | 50/50% cotton/polyester |
| Rayon elastane from malharia Fremetax | 10 pieces 15 x 15 cm | 96% rayon/ 4% elastane |
| Cotton towel from Budmeyer | 10 pieces 20 x 20 cm | 200g/cm2 cotton towel |
| Empa 252 from CFT | 10 pieces 10 x 10 cm | Standard cotton from CFT |

Table 5. Enzyme dosage for the test.

| | Laundry Detergent Composition | Fabric Softener | Enzyme % | Enzyme |
|---|---|---|---|---|
| A | HDL Brilhante | Ype | 0,5 | Enz 1 |
| B | HDL Brilhante | Ype | 2,5 | Enz 1 |
| C | HDL Brilhante | Ype | 5,0 | Enz 1 |
| D | HDL Brilhante | Ype | 0 | - |
| Enzyme preparation 1 (Enz 1): see details of composition in Example 3. | | | | |

Detailed steps to carry out full scale wash trial:

1) Choose normal cycle (dia-a-dia) at machine, low volume (35L), and add an extra rinsing step for softeners.
2) Start program to fill up the machines with water and adjust the temperature (25 C) manually.
3) Add detergent directly to water.
4) Weigh Softeners and Enzyme preparation (table 5) and mix them together on softener special compartment of machine (the softener will be delivered only during the second rinsing step)
5) Add ballast and fabrics - 2 pieces in each machine (table 4)

6) Close the machine lid and start the cycle.
7) When the cycle ends (after centrifugation), remove the ballast and separate for the next washing cycle.
8) Restart from step 1
9) Run 20 times steps #1 to #7.
10) After the 20th cycle remove the fabrics and line dry in accordance with procedure.

Drying procedure

Hang the fabrics in line and dry at room temperature after the 20th cycle. The room has a de-humidifier working for 24h per day to keep the room dry

Measurement

20 trained test persons evaluated the fabric by giving a note from 1 to 10 to each coded sample based on better appearance (renewal of the fibers) and softness for the towels, were 10 is the best one corresponding to appearance and softness of newly produced fabric. Notes presented are the average to give a single rating number.

Four fabrics were tested:

Blue stripped polycotton from Malharia Fremetex.; Rayon elastane from malharia Fremetax; Cotton towel from Budmeyer; Empa 252 from CFT ;

Washing experiment

1,8g/L of HDL Brilhante was dissolved directly into machine with water hardness of 70 ppm (tap water). At formulas with Enz 1, enzymes were added together with softener into softener special compartment on the machine, based on Table 5, w/w% from softener. Furthermore, fabrics plus ballast were added in a total of 1kg, as prepared on table 4 (2 pieces of each fabric in each machine) and start the "dia a dia"program, following the steps described on washing conditions. After the 20th washing cycle, fabrics were line dried overnight. Measurement was made using group of people as panelists, given notes from 1 to 10 to each fabirc (10 is the best, look as new), based on visual appearance and softness compared to a new one.

Results

Based on sensory panel analysis, average of rates for each fabric and formula are shown on table 6 (for appearance) and table 7 (for softness), below:

Table 6. rates of appearance when enzymes are applied.

| Laundry Detergent composition | Fabric Softener | Fabric | Enzyme | Rate |
|---|---|---|---|---|
| HDL Brilhante | Ype | All fabrics | 0,5% Enz 1 | 3,5 |
| HDL Brilhante | Ype | All fabrics | 2,5% Enz 1 | 5,79 |
| HDL Brilhante | Ype | All fabrics | 5% Enz 1 | 9,3 |
| HDL Brilhante | Ype | All fabrics | 0 | 2,13 |

Table 7. rates of softness when enzymes are applied.

| Laundry Detergent composition | Fabric Softener | Fabric | Enzyme | Rate |
|---|---|---|---|---|
| HDL Brilhante | Ype | All fabrics | 0,5% Enz 1 | 4,22 |
| HDL Brilhante | Ype | All fabrics | 2,5% Enz 1 | 6,41 |
| HDL Brilhante | Ype | All fabrics | 5% Enz 1 | 9,8 |
| HDL Brilhante | Ype | All fabrics | 0 | 4,16 |

**Example 5**

**Exposure test with Enz 1 - Full scale wash**

This is a shaking test used to prove safety for end user of Enz 1 applied to a fabric softener formulation, on full scale wash under LA conditions (washing in a top loader washing machine).T-shirts were added to wash together with detergent, softeners and enzymes. After wash, t-shirts were line dried.

Equipment used:

• Washing machine: BWL11A Brastemp

Wash conditions

**1 cycle wash**

**Machine:** TOP Load BWL11A Brastemp
**Washing temperature (°C):** 25
**Washing cycle:** cycle "dia-a-dia" (normal) 1h30min washing cycle, with two rinsing steps. Softeners + enzymes added on second rinsing step.
**Detergent concentration (g/L):** 1,8 HDL Brilhante (UL)
**Water hardness (ppm):** 70 (tap water)
**Washing volume (L):** 75 (high)
**Rinsing volume (L) :** 75 (high)
**Rinsing time:** 15 minutes
**Agitation speed (rpm):** 100 rpm
**Ballast:** 2 kg of tshirts - table 8
**Enzyme Dosages:** table 9
**Measurement:** ELISA assay
**Softener:** 50g

Table 8. Fabrics.

| **Fabric** | **#** | **Composition** |
|---|---|---|
| Hering super cotton t-shirt | 30 t-shirts | 100% cotton |

Table 9. Enzyme dosage for the test.

| | Detergent | Softener | Enzyme % | Enzyme Name |
|---|---|---|---|---|
| A | HDL Brilhante | Ype | 0,5 | Enz 1 |
| B | HDL Brilhante | Ype | 2,5 | Enz 1 |
| C | HDL Brilhante | Ype | 5,0 | Enz 1 |

Enzyme preparation 1 (Enz 1): see details of composition in Example 3.

Detailed steps to carry out full scale wash trial:

1) Choose normal cycle (dia-a-dia) at machine, low volume (35L), and add an extra rinsing step for softeners.
2) Start program to fill up the machines with water and adjust the temperature (25 C) manually.
3) Add detergent directly to water.
4) Weigh Softeners and Enzymes (table 9) and mix them together on softener special compartment of machine (the softener will be delivered only during the second rinsing step)
5) Add t-shirts - 10 pieces in each machine (table 8)
6) Close the machine lid and start the cycle.
7) When the cycle ends (after centrifugation), remove the t-shirts and line dry in accordance with procedure.

Drying procedure

Hang the t-shirts in line and dry at room temperature after the $20^{th}$ cycle. The room has a de-humidifier working for 24h per day to keep the room dry

Washing experiment

1,8g/L of HDL Brilhante was dissolved directly into machine with water hardness of 70 ppm (tap water). Enzymes were added together with softener into softener special compartment on the machine, based on Table 9, w/w% from softener. Furthermore, t-shirts were added in a total of 2kg, as prepared on table 8 (10 pieces of t-shirts in each machine) and start the "dia a dia"program, following the steps described on washing conditions. After the washing cycle, t-shirts were line dried overnight.

**Example 6**

**Full scale wash**

This is the test method used to test the wash performance of enzyme preparations in full scale wash under EU conditions (washing in a front loader washing machine).
For wash cycles with SEQ ID NO: 17 tea towel with swatches of EMPA 252 and pre-aged EMPA 252 (washed 6 hours) in addition to laundry control sheet WFK 11A, WFK 80A were added to each wash cycle with liquid model detergent, enzymes and soil. After each wash cycle tea towels with swatches of EMPA 252 were tumble dried between every wash cycle dried overnight on filter paper and remission was measured at 460 nm as described above.. The L values of the black color of EMPA 252.For wash cycles with SEQ ID NO: 18 swatches (6/wash cycle) of CFT (CN-11, CN-42, CT-01, PCN-01, PN-01), EMPA 221, WFK (12AW, 20A, 30A, 80A) and knitted cotton t-shirt (2/wash cycle) were added. Soil donor (WFK greying swatch I, 'wfk sock', wfk Testgewebe GmbH) was added to each wash. Swatches were not tumble dried between every wash cycle.

Equipment used:

- Washing machine: Miele Softtronic W2445
- Water meters and automatically data collection system
- (L value is a color eye measurement without UV)

Equipment used for pre-aging:

Wascator FOM 71 CLS (supplied by SDL Atlas) program 150, 6 Hour, 40°C, 21 L water, 15dH

For the preparation and adjustment of water hardness the following ingredients are needed:

- Calcium chloride ($CaCl_2 \cdot 2H_2O$)
- Magnesium chloride ($MgCL_2 \cdot 6H_2O$)
- Sodium Hydrogen Carbonate ($NaHCO_3$)

Laundry detergent composition

| Liquid Model detergent | wt% |
|---|---|
| LAS | 7,2 |
| AEOS(SLES) | 4,2 |
| Soap -cocoa | 2,75 |
| Soap-soya | 2,75 |
| AEO biosoft N25-7 | 6,6 |
| NAOH | 1,2 |
| Ethanol | 3 |
| MPG | 6 |
| glycerol | 2 |
| Tea | 3 |
| Sodium formiate | 1 |
| sodium citrate | 2 |
| DMPA | 0,2 |
| PCA | 0,2 |
| ion exchanged water | 55,08 |

Ballast

The ballast consists of clean white cloth without optical whitener made of cotton, polyester or cotton/polyester. The composition of the ballast is a mix of different items at a cotton/polyester ratio of 65/35 based on weight. The ballast weight, dryness and item composition must be the same in each wash cycle.

After each wash cycle the ballast is inactivated in an industrial washer at 85°C/15 min or in a 95°C wash (EU machine) without detergent

Ballast Example: (Standard EU ballast composition, total 3kg)

- 2 T-shirts (100% cotton)
- 5 shirts, short sleeves (55% cotton 45% polyester)
- 1 pillow cases (35% cotton, 65%polyester),110x75cm
- 1 small bed sheets, size 100x75cm (100% cotton)
- Socks (80% cotton 20% polyester) as balance

Wash conditions

- Temperature: 30°C.
- Washing programme: Normal cotton wash without pre-wash: "Cottons".
- Water level 13-14L with "water plus"
- Water hardness: Standard EU conditions: 15°dH, $Ca^{2+}:Mg^{2+}:HCO_3$ = 4:1:7.5
- Various cellulose dosages (SEQ ID NO: 17 0,13 wt% (=0,731 mg enzyme protein (EP)/wash cycle) and 0,26 wt% (=1,46 mg EP/wash), Enzyme preparation 2 1 wt% (=60 mg EP/wash cycle)
- Liquid model detergent dosage 50g/wash cycle

Detailed steps to carry out full scale wash trial

1. Select wash program as in study plan.
2. The detergent and cellulase are placed in the wash drum in a "washing ball" (both liquid and powder detergents). Place it at the bottom.
3. Place the tea towels with swatches and and ballast in the wash drum.

4. Start digital water meter
5. Start the washer by pressing the knob START
6. After wash, take out teatowels with swatches and ballast, dry items.

Repeat the above procedure up to 20 times.

Table 10. Whiteness effect is determined by measuring remission on WFK 11A, WFK 80A at 460 nm as described above. Remission after 3 and 5 wash cycles of SUM of 10 white fabrics (WFK 11A, WFK 80A). The higher remission value, the whiter the fabric.

| Conclusion: Some whiteness effect with 1 % enzyme preparation 2 but not as good as SEQ ID NO: 18. | | |
|---|---|---|
| SUM of all (10 white swatches/T-shirt) | | |
| | 460nm | 460nm |
| Laundry detergent composition and enzyme | 3 wash cycles | 5 wash cycles |
| Liquid model detergent | 786 | 766 |
| Liquid model detergent + 0,13% SEQ ID NO: 18 | 830 | 819 |
| Liquid model detergent + 0,26% SEQ ID NO: 18 | 836 | 820 |
| Liquid model detergent + 1,00% Enzyme preparation 2 | 813 | 799 |
| Liquid model detergent + 1,00% Enzyme preparation 2 + 0,13% SEQ ID NO: 18 | 820 | 806 |

Table 11. Prevention of pilling. The lower value, the better anti-pilling effect.

| | EMPA 252 - not pre-aged | L-value | | | | |
|---|---|---|---|---|---|---|
| condition | | Before wash | 5 wash cycles | 10 wash cycles | 15 wash cycles | 20 wash cycles |
| 1 | Liquid model detergent | 20,3 | 26,0 | 30,2 | 31,3 | 32,2 |
| 2 | Liquid model detergent + 0.13% SEQ ID NO: 17 | 20,3 | 26,4 | 29,0 | 28,8 | 28,2 |
| 3 | Liquid model detergent + 0.26% SEQ ID NO: 17 | 20,3 | 25,7 | 28,1 | 27,1 | 26,7 |
| 7 | Liquid model detergent + 1,00% Enzyme preparation 2 | 20,3 | 26,0 | 28,6 | 30,6 | 30,3 |

Table 12. removal of fuzz and pills at aged textile (Rejuvenation) (pre-aged EMPA 252).

| | Pre-aged EMPA 252 | L-value | | | | |
|---|---|---|---|---|---|---|
| condition | | Before wash | 5 wash cycles | 10 wash cycles | 15 wash cycles | 20 wash cycles |
| 1 | Liquid model detergent | 32,31 | 32,57 | 33,60 | 33,64 | 33,61 |
| 2 | Liquid model detergent + 0.13% SEQ ID NO: 17 | 32,31 | 32,12 | 30,61 | 29,06 | 27,92 |
| 3 | Liquid model detergent + 0.26% SEQ ID NO: 17 | 32,31 | 31,11 | 28,49 | 27,07 | 26,39 |
| 7 | Liquid model detergent + 1,00% Enzyme preparation 2 | 32,31 | 32,77 | 31,96 | 30,66 | 30,07 |

Wash performance ΔL, Δa and Δb

Wash performance can be expressed as Lab color vector (ΔL). After washing and rinsing the swatches were spread out flat and allowed to air dry at room temperature overnight. All washes are evaluated the day after the wash. To evaluate the specific nature of the cleaning, the CIE L*, a* and b* values were also recorded by the Macbeth Color Eye 7000 reflectance spectrophotometer during the measurement. The Lab color measurements taken using the Color Eye 7000 are calculated from the CIE (Commission internationale de l'éclairage) XYZ color space co-ordinates. Lab is the abbreviation used to describe the CIE 1976 L*, a*, b* color space, where L is lightness and a, and b are color dimensions.
ΔL denotes the change in L* when taken the measurements from swatches washed with the enzyme of the invention and subtract with the measurements from swatches washed without enzyme for each stain.
Δa denotes the change in a* when taken the measurements from swatches washed with the enzyme of the invention and subtract with the measurements from swatches washed without enzyme for each stain.
Δb denotes the change in b* when taken the measurements from swatches washed with the enzyme of the invention and subtract with the measurements from swatches washed without enzyme for each stain.
Reference is made to János Schanda (2007). Colorimetry. Wiley-Interscience. p. 61. ISBN 978-0-470-04904-4.

## Claims

**1.** Use of a laundry composition comprising an enzyme preparation comprising one or more enzymes capable of degrading cellulosic material and a surfactant or a fabric softener agent, wherein the one or more enzymes capable of degrading cellulosic material comprises:

a. an *Aspergillus fumigatus* cellobiohydrolase I;
b. an *Aspergillus fumigatus* cellobiohydrolase II;
c. an *Aspergillus fumigatus* beta-glucosidase or variant thereof; and

d. a *Penicillium* sp. GH61 polypeptide having cellulolytic enhancing activity; or homologs thereof;

wherein the *Aspergillus fumigatus* cellobiohydrolase I or homolog thereof is selected from the group consisting of:

(i) a cellobiohydrolase I comprising or consisting of the mature polypeptide of SEQ ID NO: 2;
(ii) a cellobiohydrolase I comprising or consisting of an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ ID NO: 2;
(iii) a cellobiohydrolase I encoded by a polynucleotide comprising or consisting of a nucleotide sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide coding sequence of SEQ ID NO: 1; and
(iv) a cellobiohydrolase I encoded by a polynucleotide that hybridizes under at least high stringency conditions, very high stringency conditions, with the mature polypeptide coding sequence of SEQ ID NO: 1 or the full-length complement thereof;

wherein the Aspergillus fumigatus cellobiohydrolase II or homolog thereof is selected from the group consisting of:

(i) a cellobiohydrolase II comprising or consisting of the mature polypeptide of SEQ ID NO: 4;
(ii) a cellobiohydrolase II comprising or consisting of an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ ID NO: 4;
(iii) a cellobiohydrolase II encoded by a polynucleotide comprising or consisting of a nucleotide sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide coding sequence of SEQ ID NO: 3; and
(iv) a cellobiohydrolase II encoded by a polynucleotide that hybridizes under at least high stringency conditions, very high stringency conditions, with the mature polypeptide coding sequence of SEQ ID NO: 3 or the full-length complement thereof;

wherein the Aspergillus fumigatus beta-glucosidase or homolog thereof is selected from the group consisting of:

(i) a beta-glucosidase comprising or consisting of the mature polypeptide of SEQ ID NO: 6;
(ii) a beta-glucosidase comprising or consisting of an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ ID NO: 6;
(iii) a beta-glucosidase encoded by a polynucleotide comprising or consisting of a nucleotide sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide coding sequence of SEQ ID NO: 5;
(iv) a beta-glucosidase encoded by a polynucleotide that hybridizes under at least high stringency conditions, very high stringency conditions, with the mature polypeptide coding sequence of SEQ ID NO: 5 or the full-length complement thereof; and
(v) a beta-glucosidase variant comprising a substitution at one or more positions corresponding to positions 100, 283, 456, and 512 of the mature polypeptide of SEQ ID NO: 6, wherein the variant has beta-glucosidase activity; and

wherein the Penicillium sp. GH61 polypeptide having cellulolytic enhancing activity or homolog thereof is selected from the group consisting of:

(i) a GH61 polypeptide having cellulolytic enhancing activity comprising or consisting of the mature polypeptide of SEQ ID NO: 8;
(ii) a GH61 polypeptide having cellulolytic enhancing activity comprising or consisting of an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ ID NO: 8;
(iii) a GH61 polypeptide having cellulolytic enhancing activity encoded by a polynucleotide comprising or consisting of a nucleotide sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide coding sequence of SEQ ID NO: 7; and

a GH61 polypeptide having cellulolytic enhancing activity encoded by a polynucleotide that hybridizes under at least high stringency conditions, very high stringency conditions, with the mature polypeptide coding sequence of SEQ ID NO: 7 or the full-length complement thereof and wherein the composition is used for preventing build-up and/or removal of fuzz and pills from a textile and for improving the whiteness of the textile by exposing the textile to the composition during a laundry process.

**2.** Use according to claim 1, wherein the composition is a laundry detergent composition or a fabric softener composition.

**3.** Use according to any of claim 1 or 2, wherein the composition comprises

(i) an Aspergillus fumigatus cellobiohydrolase I comprising or consisting of the mature polypeptide of SEQ ID NO: 2;
(ii) an Aspergillus fumigatus cellobiohydrolase II comprising or consisting of the mature polypeptide of SEQ ID NO: 4;
(iii) an Aspergillus fumigatus beta-glucosidase comprising or consisting of the mature polypeptide of SEQ ID NO: 6; or a variant hereof comprising the following substitutions F100D, S283G, N456E and F512Y; and
(iv) a Penicillium sp. GH61 polypeptide having cellulolytic enhancing activity comprising or consisting of the mature polypeptide of SEQ ID NO: 8.

**4.** Use according to any of claims 1-3, wherein the enzyme preparation further comprises one or more enzymes selected from the group consisting of:

(i) an *Aspergillus fumigatus* xylanase or homolog thereof,
(ii) an *Aspergillus fumigatus* beta-xylosidase or homolog thereof; or
(iii) a combination of (i) and (ii);

wherein the *Aspergillus fumigatus* xylanase or homolog thereof is selected from the group consisting of:

(i) an *Aspergillus fumigatus* xylanase comprising or consisting of the mature polypeptide of SEQ ID NO: 10, SEQ ID NO: 12, or SEQ ID NO: 14;
(ii) a xylanase comprising or consisting of an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ ID NO: 10, SEQ ID NO: 12, or SEQ ID NO: 14;
(iii) a xylanase encoded by a polynucleotide comprising or consisting of a nucleotide sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide coding sequence of SEQ ID NO: 9, SEQ ID NO: 11, or SEQ ID NO: 13; and
(iv) a xylanase encoded by a polynucleotide that hybridizes under at least high stringency conditions, very high stringency conditions, with the mature polypeptide coding sequence of SEQ ID NO: 9, SEQ ID NO: 11, or SEQ ID NO: 13; or the full-length complement thereof; and

wherein the *Aspergillus fumigatus* beta-xylosidase or homolog thereof is selected from the group consisting of:

(i) beta-xylosidase comprising or consisting of the mature polypeptide of SEQ ID NO: 16;
(ii) a beta-xylosidase comprising or consisting of an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ ID NO: 16;
(iii) a beta-xylosidase encoded by a polynucleotide comprising or consisting of a nucleotide sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide coding sequence of SEQ ID NO: 15; and
(iv) a beta-xylosidase encoded by a polynucleotide that hybridizes under at least high stringency conditions, very high stringency conditions, with the mature polypeptide coding sequence of SEQ ID NO: 15 or the full-length complement thereof.

**5.** Use according to any of the preceding claims, wherein the composition comprises at least one enzyme in addition to the enzymes in the enzyme preparation, wherein the at least one more enzyme is selected from the group consisting of hemicellulases, peroxidases, proteases, cellulases, xylanases, lipases, phospholipases, esterases, cutinases, pectinases, mannanases, pectate lyases, keratinases, reductases, oxidases, phenoloxidases, lipoxygenases, ligninases, pullulanases, tannases, pentosanases, malanases, β-glucanases, arabinosidases, hyaluronidase, chondroitinase, laccase, DNase chlorophyllases, amylases, perhydrolases, peroxidases, xanthanase and mixtures thereof.

**6.** A method for laundering a textile comprising the steps of:

a) Contacting the textile with a wash liquor comprising laundry detergent composition;
b) Completing at least one wash cycle;
c) contacting the textile with water comprising a fabric softener composition; and
d) completing at least one rinse cycle;

wherein the detergent composition and/or the fabric softener composition comprise
an enzyme preparation comprising one or more enzymes capable of degrading cellulosic material and wherein the one or more enzymes capable of degrading cellulosic material comprises:

i. an Aspergillus fumigatus cellobiohydrolase I;
ii. an Aspergillus fumigatus cellobiohydrolase II;
iii. an Aspergillus fumigatus beta-glucosidase or variant thereof; and
iv. a Penicillium sp. GH61 polypeptide having cellulolytic enhancing activity; or homologs thereof;

wherein the Aspergillus fumigatus cellobiohydrolase I or homolog thereof is selected from the group consisting of:

(i) a cellobiohydrolase I comprising or consisting of the mature polypeptide of SEQ ID NO: 2;
(ii) a cellobiohydrolase I comprising or consisting of an amino acid sequence having at least 70%, at least 75%, at least 80%, at least 81 %, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ ID NO: 2;
(iii) a cellobiohydrolase I encoded by a polynucleotide comprising or consisting of a nucleotide sequence having at least 70%, at least 75%, at least 80%, at least 81 %, at least 82%, at least 83%, at least 84%, at least 85%, at least 86%, at least 87%, at least 88%, at least 89%, at least 90%, at least 91 %, at least 92%, at least 93%, at least 94%, at least 95%, at least 96%, at least 97%, at least 98%, or at least 99% sequence identity to the mature polypeptide coding sequence of SEQ ID NO: 1; and
(iv) a cellobiohydrolase I encoded by a polynucleotide that hybridizes under at least high stringency conditions, very high stringency conditions, with the mature polypeptide coding sequence of SEQ ID NO: 1 or the full-length complement thereof;

wherein the Aspergillus fumigatus cellobiohydrolase II or homolog thereof is selected from the group consisting of:

(i) a cellobiohydrolase II comprising or consisting of the mature polypeptide of SEQ ID NO: 4;
(ii) a cellobiohydrolase II comprising or consisting of an amino acid sequence having at least 70%, at least 75%, at least 80%, at least 81 %, at least 82%, at least 83%, at least 84%, at least 85%, at least 86%, at least 87%, at least 88%, at least 89%, at least 90%, at least 91 %, at least 92%, at least 93%, at least 94%, at least 95%, at least 96%, at least 97%, at least 98%, or at least 99% sequence identity to the mature polypeptide of SEQ ID NO: 4;
(iii) a cellobiohydrolase II encoded by a polynucleotide comprising or consisting of a nucleotide sequence having at least 70%, at least 75%, at least 80%, at least 81 %, at least 82%, at least 83%, at least 84%, at least 85%, at least 86%, at least 87%, at least 88%, at least 89%, at least 90%, at least 91 %, at least 92%, at least 93%, at least 94%, at least 95%, at least 96%, at least 97%, at least 98%, or at least 99% sequence identity to the mature polypeptide coding sequence of SEQ ID NO: 3; and
(iv) a cellobiohydrolase II encoded by a polynucleotide that hybridizes under at least high stringency conditions, very high stringency conditions, with the mature polypeptide coding sequence of SEQ ID NO: 3 or the full-length complement thereof;

wherein the Aspergillus fumigatus beta-glucosidase or homolog thereof is selected from the group consisting of:

(i) a beta-glucosidase comprising or consisting of the mature polypeptide of SEQ ID NO: 6;
(ii) a beta-glucosidase comprising or consisting of an amino acid sequence having at least 70%, at least 75%, at least 80%, at least 81 %, at least 82%, at least 83%, at least 84%, at least 85%, at least 86%, at least 87%, at least 88%, at least 89%, at least 90%, at least 91 %, at least 92%, at least 93%, at least 94%, at least 95%, at least 96%, at least 97%, at least 98%, or at least 99% sequence identity to the mature polypeptide of SEQ ID NO: 6;
(iii) a beta-glucosidase encoded by a polynucleotide comprising or consisting of a nucleotide sequence having at least 70%, at least 75%, at least 80%, at least 81%, at least 82%, at least 83%, at least 84%, at least 85%, at least 86%, at least 87%, at least 88%, at least 89%, at least 90%, at least 91 %, at least 92%, at least 93%, at least 94%, at least 95%, at least 96%, at least 97%, at least 98%, or at least 99% sequence identity to the mature polypeptide coding sequence of SEQ ID NO: 5;
(iv) a beta-glucosidase encoded by a polynucleotide that hybridizes under at least high stringency conditions, very high stringency conditions, with the mature polypeptide coding sequence of SEQ ID NO: 5 or the full-length complement thereof; and
(v) a beta-glucosidase variant comprising a substitution at one or more positions corresponding to positions 100, 283, 456, and 512 of the mature polypeptide of SEQ ID NO: 6, wherein the variant has beta-glucosidase activity;

and wherein the Penicillium sp. GH61 polypeptide having cellulolytic enhancing activity or homolog thereof is selected from the group consisting of:

(i) a GH61 polypeptide having cellulolytic enhancing activity comprising or consisting of the mature polypeptide of SEQ ID NO: 8;
(ii) a GH61 polypeptide having cellulolytic enhancing activity comprising or consisting of an amino acid sequence having at least 70%, at least 75%, at least 80%, at least 81 %, at least 82%, at least 83%, at least 84%, at least 85%, at least 86%, at least 87%, at least 88%, at least 89%, at least 90%, at least 91 %, at least 92%, at least 93%, at least 94%, at least 95%, at least 96%, at least 97%, at least 98%, or at least 99% sequence identity to the mature polypeptide of SEQ ID NO: 8;
(iii) a GH61 polypeptide having cellulolytic enhancing activity encoded by a polynucleotide comprising or consisting of a nucleotide sequence having at least 70%, at least 75%, at least 80%, at least 81 %, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide coding sequence of SEQ ID NO: 7; and

a GH61 polypeptide having cellulolytic enhancing activity encoded by a polynucleotide that hybridizes under at least high stringency conditions, very high stringency conditions, with the mature polypeptide coding sequence of SEQ ID NO: 7 or the full-length complement thereof;
and wherein the method prevents build-up and/or removal of fuzz and pills from a textile and improves the whiteness of the textile.

7. Method according to claim 6, wherein the laundry detergent composition and/or the fabric softener composition comprises

(iv) an Aspergillus fumigatus cellobiohydrolase I comprising or consisting of the mature polypeptide of SEQ ID NO: 2;
(v) an Aspergillus fumigatus cellobiohydrolase II comprising or consisting of the mature polypeptide of SEQ ID NO: 4;
(vi) an Aspergillus fumigatus beta-glucosidase comprising or consisting of the mature polypeptide of SEQ ID NO: 6; or a variant hereof comprising the following substitutions F100D, S283G, N456E, F512Y,
(vii)a Penicillium sp. GH61 polypeptide having cellulolytic enhancing activity comprising or consisting of the mature polypeptide of SEQ ID NO: 8.

8. Method according to any of claims 6-7, wherein the laundry method is carried out by hand or machine.

9. Method according to any of claims 6-8, wherein the method is carried out at least 5 times.

10. Method according to claim 9, wherein the method is carried out at least 10 times, at least 15 times or at least 20 times.

11. Method according to any of claims 6-10, wherein the concentration of the enzyme preparation is in the range of 0.03 to 5 gram/liter.

12. Method according to claim 11, wherein the concentration of the enzyme preparation is in the range of 0.05-4 gram/liter, in the range of 0.1-3 gram/liter, in the range of 0.2-2.5 gram/liter, in the range of 0.2-2 gram/liter or in the range of 0.3-1 gram/liter.

13. A cleaning method for cleaning the interior of a washing machine, which method comprises exposing the interior of the washing machine to one or more enzymes capable of degrading cellulosic material, wherein the one or more enzymes capable of degrading cellulosic material is an enzyme preparation comprising:

(i) an Aspergillus fumigatus cellobiohydrolase I;
(ii) an Aspergillus fumigatus cellobiohydrolase II;
(iii) an Aspergillus fumigatus beta-glucosidase or variant thereof; and
(iv) a Penicillium sp. GH61 polypeptide having cellulolytic enhancing activity; or homologs thereof;

wherein the *Aspergillus fumigatus* cellobiohydrolase I or homolog thereof is selected from the group consisting of:

(i) a cellobiohydrolase I comprising or consisting of the mature polypeptide of SEQ ID NO: 2;
(ii) a cellobiohydrolase I comprising or consisting of an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ ID NO: 2;
(iii) a cellobiohydrolase I encoded by a polynucleotide comprising or consisting of a nucleotide sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide coding sequence of SEQ ID NO: 1; and
(iv) a cellobiohydrolase I encoded by a polynucleotide that hybridizes under at least high stringency conditions, very high stringency conditions, with the mature polypeptide coding sequence of SEQ ID NO: 1 or the full-length complement thereof;

wherein the *Aspergillus fumigatus* cellobiohydrolase II or homolog thereof is selected from the group consisting of:

(i) a cellobiohydrolase II comprising or consisting of the mature polypeptide of SEQ ID NO: 4;
(ii) a cellobiohydrolase II comprising or consisting of an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ

ID NO: 4;

(iii) a cellobiohydrolase II encoded by a polynucleotide comprising or consisting of a nucleotide sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide coding sequence of SEQ ID NO: 3; and

(iv) a cellobiohydrolase II encoded by a polynucleotide that hybridizes under at least high stringency conditions, very high stringency conditions, with the mature polypeptide coding sequence of SEQ ID NO: 3 or the full-length complement thereof;

wherein the *Aspergillus fumigatus* beta-glucosidase or homolog thereof is selected from the group consisting of:

(i) a beta-glucosidase comprising or consisting of the mature polypeptide of SEQ ID NO: 6;

(ii) a beta-glucosidase comprising or consisting of an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ ID NO: 6;

(iii) a beta-glucosidase encoded by a polynucleotide comprising or consisting of a nucleotide sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide coding sequence of SEQ ID NO: 5;

(iv) a beta-glucosidase encoded by a polynucleotide that hybridizes under at least high stringency conditions, very high stringency conditions, with the mature polypeptide coding sequence of SEQ ID NO: 5 or the full-length complement thereof; and

(v) a beta-glucosidase variant comprising a substitution at one or more positions corresponding to positions 100, 283, 456, and 512 of the mature polypeptide of SEQ ID NO: 6, wherein the variant has beta-glucosidase activity; and

wherein the Penicillium sp. GH61 polypeptide having cellulolytic enhancing activity or homolog thereof is selected from the group consisting of:

(i) a GH61 polypeptide having cellulolytic enhancing activity comprising or consisting of the mature polypeptide of SEQ ID NO: 8;

(ii) a GH61 polypeptide having cellulolytic enhancing activity comprising or consisting of an amino acid sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ ID NO: 8;

(iii) a GH61 polypeptide having cellulolytic enhancing activity encoded by a polynucleotide comprising or consisting of a nucleotide sequence having at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide coding sequence of SEQ ID NO: 7; and

a GH61 polypeptide having cellulolytic enhancing activity encoded by a polynucleotide that hybridizes under at least high stringency conditions, very high stringency conditions, with the mature polypeptide coding sequence of SEQ ID NO: 7 or the full-length complement thereof.

## Patentansprüche

1. Verwendung einer Wäschewaschzusammensetzung, die eine Enzymzubereitung, die ein oder mehrere zum Abbauen von cellulosischem Material fähige Enzyme umfasst, und ein oberflächenaktives Mittel oder einen Gewebeweichspüler umfasst, wobei das eine oder die mehreren zum Abbauen von cellulosischem Material fähigen Enzyme umfasst/umfassen:

a. eine *Aspergillus fumigatus*-Cellobiohydrolase I;
b. eine *Aspergillus fumigatus*-Cellobiohydrolase II;
c. eine *Aspergillus fumigatus*-beta-Glucosidase oder eine Variante davon; und
d. ein *Penicillium* sp. GH61-Polypeptid mit cellulolytisch verstärkender Aktivität; oder Homologe davon;

wobei die *Aspergillus* fumigatus-Cellobiohydrolase I oder das Homologe davon ausgewählt ist aus der Gruppe bestehend aus:

(i) einer Cellobiohydrolase I, die das reife Polypeptid von SEQ ID NO: 2 umfasst oder daraus besteht;
(ii) einer Cellobiohydrolase I, die eine Aminosäuresequenz mit mindestens 70%, mindestens 75%, mindestens 80%, mindestens 81%, mindestens 82%, mindestens 83%, mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98%, oder mindestens 99% Sequenzidentität zum reifen Polypeptid von SEQ ID NO: 2 umfasst oder daraus besteht;
(iii) einer Cellobiohydrolase I, die durch ein Polynukleotid kodiert ist, das eine Nukleotidsequenz mit mindestens 70%, mindestens 75%, mindestens 80%, mindestens 81%, mindestens 82%, mindestens 83%, mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98% oder mindestens 99% Sequenzidentität zur das reife Polypeptid kodierenden Sequenz von SEQ ID NO: 1 umfasst oder daraus besteht; und
(iv) einer Cellobiohydrolase I, die durch ein Polynukleotid kodiert ist, das unter zumindest hohen Stringenzbedingungen, sehr hohen Stringenzbedingungen mit der das reife Polypeptid kodierenden Sequenz von SEQ ID NO: 1 oder dem Komplement voller Länge davon hybridisiert;

wobei die *Aspergillus* fumigatus-Cellobiohydrolase II oder das Homologe davon aus der Gruppe ausgewählt ist bestehend aus:

(i) einer Cellobiohydrolase II, die das reife Polypeptid von SEQ ID NO: 4 umfasst oder daraus besteht;
(ii) einer Cellobiohydrolase II, die eine Aminosäuresequenz mit mindestens 70%, mindestens 75%, mindestens 80%, mindestens 81%, mindestens 82%, mindestens 83%, mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98% oder mindestens 99% Sequenzidentität zum reifen Polypeptid von SEQ ID NO: 4 umfasst oder daraus besteht;
(iii) einer Cellobiohydrolase II, die durch ein Polynukleotid kodiert ist, das eine Nukleotidsequenz mit mindestens 70%, mindestens 75%, mindestens 80%, mindestens 81%, mindestens 82%, mindestens 83%, mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98% oder mindestens 99% Sequenzidentität zur das reife Polypeptid kodierenden Sequenz von SEQ ID NO: 3 umfasst oder daraus besteht; und
(iv) einer Cellobiohydrolase II, die durch ein Polynukleotid kodiert ist, das unter zumindest hohen Stringenzbedingungen, sehr hohen Stringenzbedingungen mit der das reife Polypeptid kodierenden Sequenz von SEQ ID NO: 3 oder dem Komplement voller Länge davon hybridisiert;

wobei die *Aspergillus fumigatus*-beta-Glucosidase oder das Homologe davon aus der Gruppe ausgewählt ist bestehend aus:

(i) einer beta-Glucosidase, die das reife Polypeptid von SEQ ID NO: 6 umfasst oder daraus besteht;
(ii) einer beta-Glucosidase, die eine Aminosäuresequenz mit mindestens 70%, mindestens 75%, mindestens 80%, mindestens 81%, mindestens 82%, mindestens 83%, mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98% oder mindestens 99% Sequenzidentität zum reifen Polypeptid von SEQ NO: 6 umfasst oder daraus besteht;
(iii) einer beta-Glucosidase, die durch ein Polynukleotid kodiert ist, das eine Nukleotidsequenz mit mindestens 70%, mindestens 75%, mindestens 80%, mindestens 81%, mindestens 82%, mindestens 83%, mindestens 84 mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%,

mindestens 97%, mindestens 98% oder mindestens 99% Sequenzidentität zur das reife Polypeptid kodierenden Sequenz von SEQ ID NO: 5 umfasst oder daraus besteht;
(iv) einer beta-Glucosidase, die durch ein Polynukleotid kodiert ist, das unter zumindest hohen Stringenzbedingungen, sehr hohen Stringenzbedingungen mit der das reife Polypeptid kodierenden Sequenz von SEQ ID NO: 5 oder dem Komplement voller Länge davon hybridisiert; und
(v) einer beta-Glucosidasevariante, die eine Substitution an einer oder mehreren Positionen entsprechend Positionen 100, 283, 456 und 512 des reifen Polypeptids von SEQ ID NO: 6 umfasst, wobei die Variante beta-Glucosidaseaktivität aufweist; und

wobei das *Penicillium* sp. GH61-Polypeptid mit cellulolytisch verstärkender Aktivität oder das Homologe davon aus der Gruppe ausgewählt ist bestehend aus:

(i) einem GH61-Polypeptid mit cellulolytisch verstärkender Aktivität, das das reife Polypeptid von SEQ ID NO: 8 umfasst oder daraus besteht;
(ii) einem GH61-Polypeptid mit cellulolytisch verstärkender Aktivität, das eine Aminosäuresequenz mit mindestens 70%, mindestens 75%, mindestens 80%, mindestens 81%, mindestens 82%, mindestens 83%, mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98% oder mindestens 99% Sequenzidentität zum reifen Polypeptid von SEQ ID NO: 8 umfasst oder daraus besteht;
(iii) einem GH61-Polypeptid mit cellulolytisch verstärkender Aktivität, das durch ein Polynukleotid kodiert ist, das eine Nukleotidsequenz mit mindestens 70%, mindestens 75%, mindestens 80%, mindestens 81%, mindestens 82%, mindestens 83%, mindestens 84%, mindestens 85% mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98% oder mindestens 99% Sequenzidentität zur das reife Polypeptid kodierenden Sequenz von SEQ ID NO: 7 umfasst oder daraus besteht;

und einem GH61-Polypeptid mit cellulolytisch verstärkender Aktivität, das durch ein Polynukleotid kodiert ist, das unter zumindest hohen Stringenzbedingungen, sehr hohen Stringenzbedingungen mit der das reife Polypeptid kodierenden Sequenz von SEQ ID NO: 7 oder dem Komplement voller Länge davon hybridisiert,
und wobei die Zusammensetzung zum Vorbeugen der Bildung und/oder zum Entfernen von Fusseln und Pilling von einem Textil, und zum Verbessern des Weißgrads des Textils durch Aussetzen des Textils der Zusammensetzung während eines Waschverfahrens verwendet wird.

**2.** Verwendung nach Anspruch 1, wobei die Zusammensetzung eine Waschmitteldetergenszusammensetzung oder eine Gewebeweichspülerzusammensetzung ist.

**3.** Verwendung nach einem beliebigen der Ansprüche 1 oder 2, wobei die Zusammensetzung umfasst

(i) eine *Aspergillus fumigatus*-Cellobiohydrolase I, die das reife Polypeptid von SEQ ID NO: 2 umfasst oder daraus besteht;
(ii) an *Aspergillus fumigatus*-Cellobiohydrolase II, die das reife Polypeptid von SEQ ID NO: 4 umfasst oder daraus besteht;
(iii) an *Aspergillus fumigatus*-beta-Glucosidase, die das reife Polypeptid von SEQ ID NO: 6 umfasst oder daraus besteht; oder eine Variante davon, die die folgenden Substitutionen F100D, S283G, N456E und F512Y umfasst; und
(iv) ein *Penicillium* sp. GH61-Polypeptid mit cellulolytisch verstärkender Aktivität, das das reife Polypeptid von SEQ ID NO: 8 umfasst oder daraus besteht.

**4.** Verwendung nach einem beliebigen der Ansprüche 1-3, wobei die Enzymzubereitung des Weiteren ein oder mehrere Enzyme umfasst, die ausgewählt sind aus der Gruppe bestehend aus:

(i) einer *Aspergillus fumigatus*-Xylanase oder einem Homologen davon,
(ii) einer *Aspergillus fumigatus*-beta-Xylosidase oder einem Homologen davon; oder
(iii) einer Kombination von (i) und (ii);

wobei die *Aspergillus fumigatus*-Xylanase oder das Homologe davon aus der Gruppe ausgewählt ist bestehend aus:

(i) einer *Aspergillus fumigatus*-Xylanase, die das reife Polypeptid von SEQ ID NO: 10, SEQ ID NO: 12 oder SEQ ID NO: 14 umfasst oder daraus besteht;
(ii) einer Xylanase, die eine Aminosäuresequenz mit mindestens 70%, mindestens 75%, mindestens 80%, mindestens 81%, mindestens 82%, mindestens 83%, mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98% oder mindestens 99% Sequenzidentität zum reifen Polypeptid von SEQ ID NO: 10, SEQ ID NO: 12 oder SEQ ID NO:14 umfasst oder daraus besteht;
(iii) einer Xylanase, die durch ein Polynukleotid kodiert ist, das eine Nukleotidsequenz mit mindestens 70%, mindestens 75%, mindestens 80%, mindestens 81%, mindestens 82%, mindestens 83%, mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98% oder mindestens 99% Sequenzidentität zur das reife Polypeptid kodierenden Sequenz von SEQ ID NO: 9, SEQ ID NO: 11 oder SEQ ID NO: 13 umfasst oder daraus besteht; und
(iv) einer Xylanase, die durch ein Polynukleotid kodiert ist, das unter zumindest hohen Stringenzbedingungen, sehr hohen Stringenzbedingungen mit der das reife Polypeptid kodierenden Sequenz von SEQ ID NO: 9, SEQ ID NO: 11 oder SEQ ID NO: 13; oder dem Komplement voller Länge davon hybridisiert; und

wobei die *Aspergillus fumigatus*-beta-Xylosidase oder das Homologe davon ausgewählt ist aus der Gruppe bestehend aus:

(i) einer beta-Xylosidase, die das reife Polypeptid von SEQ ID NO: 16 umfasst oder daraus besteht;
(ii) einer beta-Xylosidase, die eine Aminosäuresequenz mit mindestens 70%, mindestens 75%, mindestens 80%, mindestens 81%, mindestens 82%, mindestens 83%, mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98% oder mindestens 99% Sequenzidentität zum reifen Polypeptid von SEQ ID NO: 16 umfasst oder daraus besteht;
(iii) einer beta-Xylosidase, die durch ein Polynukleotid kodiert ist, das eine Nukleotidsequenz mit mindestens 70%, mindestens 75%, mindestens 80%, mindestens 81%, mindestens 82%, mindestens 83%, mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98% oder mindestens 99% Sequenzidentität zur das reife Polypeptid kodierenden Sequenz von SEQ ID NO: 15 umfasst oder daraus besteht; und
(iv) einer beta-Xylosidase, die durch ein Polynukleotid kodiert ist, das unter zumindest hohen Stringenzbedingungen, sehr hohen Stringenzbedingungen mit der das reife Polypeptid kodierenden Sequenz von SEQ ID NO: 15 oder dem Komplement voller Länge davon hybridisiert.

**5.** Verwendung nach einem beliebigen der voranstehenden Ansprüche, wobei die Zusammensetzung mindestens ein Enzym zusätzlich zu den Enzymen in der Enzymzubereitung umfasst, wobei das mindestens eine weitere Enzym aus der Gruppe bestehend aus Hemicellulasen, Peroxidasen, Proteasen, Cellulasen, Xylanasen, Lipasen, Phospholipasen, Esterasen, Cutinasen, Pectinasen, Mannanasen, Pectatlyasen, Keratinasen, Reduktasen, Oxidasen, Phenoloxidasen, Lipoxygenasen, Ligninasen, Pullulanasen, Tannasen, Pentosanasen, Malanasen, β-Glucanasen, Arabinosidasen, Hyaluronidase, Chondroitinase, Laccase, DNase, Chlorophyllasen, Amylasen, Perhydrolasen, Peroxidasen, Xanthanase und Gemischen davon ausgewählt ist.

**6.** Verfahren zum Waschen eines Textils, das die Schritte umfasst:

a) Inkontaktbringen des Textils mit einer Waschflotte, die die Waschmitteldetergenszusammensetzung umfasst;
b) Vollenden von mindestens einem Waschzyklus;
c) Inkontaktbringen des Textils mit Wasser, das eine Gewebeweichspülerzusammensetzung umfasst; und
d) Vollenden von mindestens einem Spülzyklus;

wobei die Waschmitteldetergenszusammensetzung und/oder die Gewebeweichspülerzusammensetzung eine Enzymzubereitung umfasst, die ein oder mehrere Enzyme umfasst, die zum Abbauen von cellulosischem Material fähig sind, und das eine oder die mehreren zum Abbauen von cellulosischem Material fähigen Enzyme umfassen:

i. eine *Aspergillus fumigatus*-Cellobiohydrolase I;

ii. eine *Aspergillus fumigatus*-Cellobiohydrolase II;
iii. eine *Aspergillus fumigatus*-beta-Glucosidase oder eine Variante davon; und
iv. ein *Penicillium* sp. GH61-Polypeptid mit cellulolytisch verstärkender Aktivität; oder Homologe davon;

wobei die *Aspergillus fumigatus*-Cellobiohydrolase I oder das Homologe davon ausgewählt ist aus der Gruppe bestehend aus:

(i) einer Cellobiohydrolase I, die das reife Polypeptid von SEQ ID NO: 2 umfasst oder daraus besteht;
(ii) einer Cellobiohydrolase I, die eine Aminosäuresequenz mit mindestens 70%, mindestens 75%, mindestens 80%, mindestens 81 %, mindestens 82%, mindestens 83%, mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98% oder mindestens 99% Sequenzidentität zum reifen Polypeptid von SEQ ID NO: 2 umfasst oder daraus besteht;
(iii) einer Cellobiohydrolase I, die durch ein Polynukleotid kodiert ist, das eine Nukleotidsequenz mit mindestens 70%, mindestens 75%, mindestens 80%, mindestens 81 %, mindestens 82%, mindestens 83%, mindestens 84%, mindestens 85%, mindestens 86%, mindestens 87%, mindestens 88%, mindestens 89%, mindestens 90%, mindestens 91 %, mindestens 92%, mindestens 93%, mindestens 94%, mindestens 95%, mindestens 96%, mindestens 97%, mindestens 98% oder mindestens 99% Sequenzidentität mit der das reife Polypeptid kodierenden Sequenz von SEQ ID NO: 1 umfasst oder daraus besteht; und
(iv) einer Cellobiohydrolase I, die durch ein Polynukleotid kodiert ist, das unter zumindest hohen Stringenzbedingungen, sehr hohen Stringenzbedingungen mit der das reife Polypeptid kodierenden Sequenz von SEQ ID NO: 1 oder dem Komplement voller Länge davon hybridisiert;

wobei die *Aspergillus fumigatus*-Cellobiohydrolase II oder das Homologe davon ausgewählt ist aus der Gruppe bestehend aus:

(i) einer Cellobiohydrolase II, die das reife Polypeptid von SEQ ID NO: 4 umfasst oder daraus besteht;
(ii) einer Cellobiohydrolase II, die eine Aminosäuresequenz mit mindestens 70%, mindestens 75%, mindestens 80%, mindestens 81 %, mindestens 82%, mindestens 83%, mindestens 84%, mindestens 85%, mindestens 86%, mindestens 87%, mindestens 88%, mindestens 89%, mindestens 90%, mindestens 91 %, mindestens 92%, mindestens 93%, mindestens 94%, mindestens 95%, mindestens 96%, mindestens 97%, mindestens 98% oder mindestens 99% Sequenzidentität zum reifen Polypeptid von SEQ ID NO: 4 umfasst oder daraus besteht;
(iii) einer Cellobiohydrolase II, die durch ein Polynukleotid kodiert ist, das eine Nukleotidsequenz mit mindestens 70%, mindestens 75%, mindestens 80%, mindestens 81 %, mindestens 82%, mindestens 83%, mindestens 84%, mindestens 85%, mindestens 86%, mindestens 87%, mindestens 88%, mindestens 89%, mindestens 90%, mindestens 91 %, mindestens 92%, mindestens 93%, mindestens 94%, mindestens 95%, mindestens 96%, mindestens 97%, mindestens 98% oder mindestens 99% Sequenzidentität mit der das reife Polypeptid kodierenden Sequenz von SEQ ID NO: 3 umfasst oder daraus besteht; und
(iv) einer Cellobiohydrolase II, die durch ein Polynukleotid kodiert ist, das unter zumindest hohen Stringenzbedingungen, sehr hohen Stringenzbedingungen mit der das reife Polypeptid kodierenden Sequenz von SEQ ID NO: 3 oder dem Komplement voller Länge davon hybridisiert;

wobei die *Aspergillus fumigatus*-beta-Glucosidase oder das Homologe davon ausgewählt ist aus der Gruppe bestehend aus:

(i) einer beta-Glucosidase, die das reife Polypeptid von SEQ ID NO: 6 umfasst oder daraus besteht;
(ii) einer beta-Glucosidase, die eine Aminosäuresequenz mit mindestens 70%, mindestens 75%, mindestens 80%, mindestens 81 %, mindestens 82%, mindestens 83%, mindestens 84%, mindestens 85%, mindestens 86%, mindestens 87%, mindestens 88%, mindestens 89%, mindestens 90%, mindestens 91 %, mindestens 92%, mindestens 93%, mindestens 94%, mindestens 95%, mindestens 96%, mindestens 97%, mindestens 98% oder mindestens 99% Sequenzidentität zum reifen Polypeptid von SEQ ID NO: 6 umfasst oder daraus besteht;
(iii) einer beta-Glucosidase, die durch ein Polynukleotid kodiert ist, das eine Nukleotidsequenz mit mindestens 70%, mindestens 75%, mindestens 80%, mindestens 81%, mindestens 82%, mindestens 83%, mindestens 84%, mindestens 85%, mindestens 86%, mindestens 87%, mindestens 88%, mindestens 89%, mindestens 90%, mindestens 91 %, mindestens 92%, mindestens 93%, mindestens 94%, mindestens 95%, mindestens

96%, mindestens 97%, mindestens 98% oder mindestens 99% Sequenzidentität mit der das reife Polypeptid kodierenden Sequenz von SEQ ID NO: 5 umfasst oder daraus besteht;
(iv) einer beta-Glucosidase, die durch ein Polynukleotid kodiert ist, das unter zumindest hohen Stringenzbedingungen, sehr hohen Stringenzbedingungen mit der das reife Polypeptid kodierenden Sequenz von SEQ ID NO: 5 oder dem Komplement voller Länge davon hybridisiert; und
(v) einer beta-Glucosidasevariante, die eine Substitution an einer oder mehreren Positionen entsprechend Positionen 100, 283, 456 und 512 des reifen Polypeptids von SEQ ID NO: 6 umfasst, wobei die Variante beta-Glucosidaseaktivität aufweist;

und wobei das *Penicillium* sp.-GH61-Polypeptid mit cellulolytisch verstärkender Aktivität oder das Homologe davon ausgewählt ist aus der Gruppe bestehend aus:

(i) einem GH61-Polypeptid mit cellulolytisch verstärkender Aktivität, das das reife Polypeptid von SEQ ID NO: 8 umfasst oder daraus besteht;
(ii) einem GH61-Polypeptid mit cellulolytisch verstärkender Aktivität, das eine Aminosäuresequenz mit mindestens 70%, mindestens 75%, mindestens 80%, mindestens 81 %, mindestens 82%, mindestens 83%, mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98% oder mindestens 99% Sequenzidentität zum reifen Polypeptid von SEQ ID NO: 8 umfasst oder daraus besteht;
(iii) einem GH61-Polypeptid mit cellulolytisch verstärkender Aktivität, das durch ein Polynukleotid kodiert ist, das eine Nukleotidsequenz mit mindestens 70%, mindestens 75%, mindestens 80%, mindestens 81%, mindestens 82%, mindestens 83%, mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98%, oder mindestens 99% Sequenzidentität mit der das reife Polypeptid kodierenden Sequenz von SEQ ID NO: 7 umfasst oder daraus besteht; und einem GH61-Polypeptid mit cellulolytisch verstärkender Aktivität, das durch ein Polynukleotid kodiert ist, das unter zumindest hohen Stringenzbedingungen, sehr hohen Stringenzbedingungen mit der das reife Polypeptid kodierenden Sequenz von SEQ ID NO: 7 oder dem Komplement voller Länge davon hybridisiert;

und wobei das Verfahren der Bildung und/oder dem Entfernen von Fusseln und Pilling von einem Textil vorbeugt, und den Weißgrad des Textils verbessert.

**7.** Verfahren nach Anspruch 6, wobei die Waschmitteldetergenszusammensetzung und/oder die Gewebeweichspülerzusammensetzung umfasst

(iv) eine *Aspergillus fumigatus*-Cellobiohydrolase I, die das reife Polypeptid von SEQ ID NO: 2 umfasst oder daraus besteht;
(v) eine *Aspergillus fumigatus*-Cellobiohydrolase II, die das reife Polypeptid von SEQ ID NO: 4 umfasst oder daraus besteht;
(vi) eine *Aspergillus fumigatus*-beta-Glucosidase, die das reife Polypeptid von SEQ ID NO: 6 umfasst oder daraus besteht; oder eine Variante davon, die die folgenden Substitutionen F100D, S283G, N456E, F512Y umfasst,
(vii) ein *Penicillium* sp. GH61-Polypeptid mit cellulolytisch verstärkender Aktivität, das das reife Polypeptid von SEQ ID NO: 8 umfasst oder daraus besteht.

**8.** Verfahren nach einem beliebigen der Ansprüche 6-7, wobei das Wäschewaschverfahren händisch oder maschinell ausgeführt wird.

**9.** Verfahren nach einem beliebigen der Ansprüche 6-8, wobei das Verfahren mindestens 5 Mal ausgeführt wird.

**10.** Verfahren nach Anspruch 9, wobei das Verfahren mindestens 10 Mal, mindestens 15 Mal oder mindestens 20 Mal ausgeführt wird.

**11.** Verfahren nach einem beliebigen der Ansprüche 6-10, wobei die Konzentration der Enzymzubereitung im Bereich von 0,03 bis 5 Gramm/Liter liegt.

**12.** Verfahren nach Anspruch 11, wobei die Konzentration der Enzymzubereitung im Bereich von 0,05-4 Gramm/Liter,

im Bereich von 0,1-3 Gramm/Liter, im Bereich von 0,2-2,5 Gramm/Liter, im Bereich von 0,2-2 Gramm/Liter oder im Bereich von 0,3-1 Gramm/Liter liegt.

**13.** Reinigungsverfahren zum Reinigen des Inneren einer Waschmaschine, welches Verfahren Aussetzen des Inneren der Waschmaschine einem oder mehreren Enzymen, die zum Abbauen von cellulosischem Material fähig sind, umfasst, wobei das eine einem oder die mehreren zum Abbauen von cellulosischem Material fähigen Enzyme eine Enzymzubereitung ist, die umfasst:

(i) eine *Aspergillus fumigatus*-Cellobiohydrolase I;
(ii) eine *Aspergillus fumigatus*-Cellobiohydrolase II;
(iii) eine *Aspergillus* fumigatus-beta-Glucosidase oder eine Variante davon; und
(iv) ein *Penicillium* sp. GH61-Polypeptid mit cellulolytisch verstärkender Aktivität; oder Homologe davon;

wobei die *Aspergillus fumigatus*-Cellobiohydrolase I oder das Homologe davon ausgewählt ist aus der Gruppe bestehend aus:

(i) einer Cellobiohydrolase I, die das reife Polypeptid von SEQ ID NO: 2 umfasst oder daraus besteht;
(ii) einer Cellobiohydrolase I, die eine Aminosäuresequenz mit mindestens 70%, mindestens 75%, mindestens 80%, mindestens 81%, mindestens 82%, mindestens 83%, mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98% oder mindestens 99% Sequenzidentität zum reifen Polypeptid von SEQ ID NO: 2 umfasst oder daraus besteht;
(iii) einer Cellobiohydrolase I, die durch ein Polynukleotid kodiert ist, das eine Nukleotidsequenz mit mindestens 70%, mindestens 75%, mindestens 80%, mindestens 81%, mindestens 82%, mindestens 83%, mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98% oder mindestens 99% Sequenzidentität mit der das reife Polypeptid kodierenden Sequenz von SEQ ID NO: 1 umfasst oder daraus besteht; und
(iv) einer Cellobiohydrolase I, die durch ein Polynukleotid kodiert ist, das unter zumindest hohen Stringenzbedingungen, sehr hohen Stringenzbedingungen mit der das reife Polypeptid kodierenden Sequenz von SEQ ID NO: 1 oder dem Komplement voller Länge davon hybridisiert;

wobei die *Aspergillus fumigatus*-Cellobiohydrolase II oder das Homologe davon ausgewählt ist aus der Gruppe bestehend aus:

(i) einer Cellobiohydrolase II, die das reife Polypeptid von SEQ ID NO: 4 umfasst oder daraus besteht;
(ii) einer Cellobiohydrolase II, die eine Aminosäuresequenz mit mindestens 70%, mindestens 75%, mindestens 80%, mindestens 81%, mindestens 82%, mindestens 83%, mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98% oder mindestens 99% Sequenzidentität zum reifen Polypeptid von SEQ ID NO: 4 umfasst oder daraus besteht;
(iii) einer Cellobiohydrolase II, die durch ein Polynukleotid kodiert ist, das eine Nukleotidsequenz mit mindestens 70%, mindestens 75%, mindestens 80%, mindestens 81%, mindestens 82%, mindestens 83%, mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98%, oder mindestens 99% Sequenzidentität mit der das reife Polypeptid kodierenden Sequenz von SEQ ID NO: 3 umfasst oder daraus besteht; und
(iv) einer Cellobiohydrolase II, die durch ein Polynukleotid kodiert ist, das unter zumindest hohen Stringenzbedingungen, sehr hohen Stringenzbedingungen mit der das reife Polypeptid kodierenden Sequenz von SEQ ID NO: 3 oder dem Komplement voller Länge davon hybridisiert;

wobei die *Aspergillus fumigatus*-beta-Glucosidase oder das Homologe davon ausgewählt ist aus der Gruppe bestehend aus:

(i) einer beta-Glucosidase, die das reife Polypeptid von SEQ ID NO: 6 umfasst oder daraus besteht;
(ii) einer beta-Glucosidase, die eine Aminosäuresequenz mit mindestens 70%, mindestens 75%, mindestens

80%, mindestens 81%, mindestens 82%, mindestens 83%, mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98% oder mindestens 99% Sequenzidentität zum reifen Polypeptid von SEQ ID NO: 6 umfasst oder daraus besteht;
(iii) einer beta-Glucosidase, die durch ein Polynukleotid kodiert ist, das eine Nukleotidsequenz mit mindestens 70%, mindestens 75%, mindestens 80%, mindestens 81%, mindestens 82%, mindestens 83%, mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98%, oder mindestens 99% Sequenzidentität mit der das reife Polypeptid kodierenden Sequenz von SEQ ID NO: 5 umfasst oder daraus besteht;
(iv) einer beta-Glucosidase, die durch ein Polynukleotid kodiert ist, das unter zumindest hohen Stringenzbedingungen, sehr hohen Stringenzbedingungen mit der das reife Polypeptid kodierenden Sequenz von SEQ ID NO: 5 oder dem Komplement voller Länge davon hybridisiert; und
(v) einer beta-Glucosidasevariante, die eine Substitution an einer oder mehreren Positionen entsprechen Positionen 100, 283, 456 und 512 des reifen Polypeptids von SEQ ID NO: 6 umfasst,

wobei die Variante beta-Glucosidaseaktivität aufweist; und
wobei das *Penicillium* sp. GH61-Polypeptid mit cellulolytisch verstärkender Aktivität oder das Homologe davon ausgewählt ist aus der Gruppe bestehend aus:

(i) einem GH61-Polypeptid mit cellulolytisch verstärkender Aktivität, das das reife Polypeptid von SEQ ID NO: 8 umfasst oder daraus besteht;
(ii) einem GH61-Polypeptid mit cellulolytisch verstärkender Aktivität, das eine Aminosäuresequenz mit mindestens 70%, mindestens 75%, mindestens 80%, mindestens 81%, mindestens 82%, mindestens 83%, mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98% oder mindestens 99% Sequenzidentität zum reifen Polypeptid von SEQ NO: 8 umfasst oder daraus besteht;
(iii) einem GH61-Polypeptid mit cellulolytisch verstärkender Aktivität, das durch ein Polynukleotid kodiert ist, das eine Nukleotidsequenz mit mindestens 70%, mindestens 75%, mindestens 80%, mindestens 81%, mindestens 82%, mindestens 83%, mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98% oder mindestens 99% Sequenzidentität mit der das reife Polypeptid kodierenden Sequenz von SEQ ID NO: 7 umfasst oder daraus besteht; und einem GH61-Polypeptid mit cellulolytisch verstärkender Aktivität, das durch ein Polynukleotid kodiert ist, das unter zumindest hohen Stringenzbedingungen, sehr hohen Stringenzbedingungen mit der das reife Polypeptid kodierenden Sequenz von SEQ ID NO: 7 oder dem Komplement voller Länge davon hybridisiert.

## Revendications

**1.** Utilisation d'une composition pour le linge comprenant une préparation enzymatique comprenant une ou plusieurs enzymes capables de dégrader un matériau cellulosique et un tensioactif ou un agent assouplissant textile, dans laquelle la ou les enzymes capables de dégrader un matériau cellulosique comprennent :

a. une cellobiohydrolase I d'*Aspergillus fumigatus* ;
b. une cellobiohydrolase II d'*Aspergillus fumigatus* ;
c. une bêta-glucosidase d'*Aspergillus fumigatus* ou un variant de celle-ci ; et
d. un polypeptide GH61 de *Penicillium* sp. ayant une activité d'amplification cellulolytique ; ou ses homologues ;

dans laquelle la cellobiohydrolase I d'*Aspergillus fumigatus* ou son homologue est choisi dans le groupe constitué par :

(i) une cellobiohydrolase I comprenant ou consistant en le polypeptide mature de la SEQ ID NO : 2 ;
(ii) une cellobiohydrolase I comprenant ou consistant en une séquence d'acides aminés ayant une identité de séquence d'au moins 70 %, d'au moins 75 %, d'au moins 80 %, d'au moins 81 %, d'au moins 82 %, d'au moins 83 %, d'au moins 84 %, d'au moins 85 %, d'au moins 86 %, d'au moins 87 %, d'au moins 88 %, d'au moins 89

%, d'au moins 90 %, d'au moins 91 %, d'au moins 92 %, d'au moins 93 %, d'au moins 94 %, d'au moins 95 %, d'au moins 96 %, d'au moins 97 %, d'au moins 98 %, ou d'au moins 99 % avec le polypeptide mature de SEQ ID NO : 2 ;

(iii) une cellobiohydrolase I codée par un polynucléotide comprenant ou consistant en une séquence de nucléotides ayant une identité de séquences d'au moins 70 %, d'au moins 75 %, d'au moins 80 %, d'au moins 81 %, d'au moins 82 %, d'au moins 83 %, d'au moins 84 %, d'au moins 85 %, d'au moins 86 %, d'au moins 87 %, d'au moins 88 %, d'au moins 89 %, d'au moins 90 %, d'au moins 91 %, d'au moins 92 %, d'au moins 93 %, d'au moins 94 %, d'au moins 95 %, d'au moins 96 %, d'au moins 97 %, d'au moins 98 %, ou d'au moins 99 % avec la séquence codante de polypeptide mature de SEQ ID NO : 1 ; et

(iv) une cellobiohydrolase I codée par un polynucléotide qui s'hybride au moins dans des conditions de stringence élevée, dans des conditions de stringence très élevées, avec la séquence codante de polypeptide mature de SEQ ID NO : 1 ou son complément de pleine longueur ;

dans laquelle la cellobiohydrolase II d'*Aspergillus fumigatus* ou son homologue est choisi dans le groupe constitué par :

(i) une cellobiohydrolase II comprenant ou consistant en le polypeptide mature de SEQ ID NO : 4 ;

(ii) une cellobiohydrolase II comprenant ou consistant en une séquence d'acides aminés ayant une identité de séquence d'au moins 70 %, d'au moins 75 %, d'au moins 80 %, d'au moins 81 %, d'au moins 82 %, d'au moins 83 %, d'au moins 84 %, d'au moins 85 %, d'au moins 86 %, d'au moins 87 %, d'au moins 88 %, d'au moins 89 %, d'au moins 90 %, d'au moins 91 %, d'au moins 92 %, d'au moins 93 %, d'au moins 94 %, d'au moins 95 %, d'au moins 96 %, d'au moins 97 %, d'au moins 98 %, ou d'au moins 99 % avec le polypeptide mature de SEQ ID NO : 4 ;

(iii) une cellobiohydrolase II codée par un polynucléotide comprenant ou consistant en une séquence de nucléotides ayant une identité de séquences d'au moins 70 %, d'au moins 75 %, d'au moins 80 %, d'au moins 81 %, d'au moins 82 %, d'au moins 83 %, d'au moins 84 %, d'au moins 85 %, d'au moins 86 %, d'au moins 87 %, d'au moins 88 %, d'au moins 89 %, d'au moins 90 %, d'au moins 91 %, d'au moins 92 %, d'au moins 93 %, d'au moins 94 %, d'au moins 95 %, d'au moins 96 %, d'au moins 97 %, d'au moins 98 %, ou d'au moins 99 % avec la séquence codante de polypeptide mature de SEQ ID NO : 3 ; et

(iv) une cellobiohydrolase II codée par un polynucléotide qui s'hybride au moins dans des conditions de stringence élevée, dans des conditions de stringence très élevées, avec la séquence codante de polypeptide mature de SEQ ID NO : 3 ou son complément de pleine longueur ;

dans laquelle la bêta-glucosidase *d'Aspergillus fumigatus* ou son homologue est choisi dans le groupe constitué par :

(i) une bêta-glucosidase comprenant ou consistant en le polypeptide mature de SEQ ID NO : 6 ;

(ii) une bêta-glucosidase comprenant ou consistant en une séquence d'acides aminés ayant une identité de séquence d'au moins 70 %, d'au moins 75 %, d'au moins 80 %, d'au moins 81 %, d'au moins 82 %, d'au moins 83 %, d'au moins 84 %, d'au moins 85 %, d'au moins 86 %, d'au moins 87 %, d'au moins 88 %, d'au moins 89 %, d'au moins 90 %, d'au moins 91 %, d'au moins 92 %, d'au moins 93 %, d'au moins 94 %, d'au moins 95 %, d'au moins 96 %, d'au moins 97 %, d'au moins 98 %, ou d'au moins 99 % avec le polypeptide mature de SEQ ID NO : 6 ;

(iii) une bêta-glucosidase codée par un polynucléotide comprenant ou consistant en une séquence de nucléotides ayant une identité de séquences d'au moins 70 %, d'au moins 75 %, d'au moins 80 %, d'au moins 81 %, d'au moins 82 %, d'au moins 83 %, d'au moins 84 %, d'au moins 85 %, d'au moins 86 %, d'au moins 87 %, d'au moins 88 %, d'au moins 89 %, d'au moins 90 %, d'au moins 91 %, d'au moins 92 %, d'au moins 93 %, d'au moins 94 %, d'au moins 95 %, d'au moins 96 %, d'au moins 97 %, d'au moins 98 %, ou d'au moins 99 % avec la séquence codante de polypeptide mature de SEQ ID NO : 5 ;

(iv) une bêta-glucosidase codée par un polynucléotide qui s'hybride au moins dans des conditions de stringence élevée, dans des conditions de stringence très élevées, avec la séquence codante de polypeptide mature de SEQ ID NO : 5 ou son complément de pleine longueur ; et

(v) un variant de bêta-glucosidase comprenant une substitution en une ou plusieurs positions correspondant aux positions 100, 283, 456 et 512 du polypeptide mature de SEQ ID NO : 6, lequel variant a une activité de bêta-glucosidase ; et

dans laquelle le polypeptide GH61 de *Penicillium* sp. ayant une activité d'amplification cellulolytique ou son homologue est choisi dans le groupe constitué par :

(i) un polypeptide GH61 ayant une activité d'amplification cellulolytique comprenant ou consistant en le polypeptide mature de SEQ ID NO : 8 ;
(ii) un polypeptide GH61 ayant une activité d'amplification cellulolytique comprenant ou consistant en une séquence d'acides aminés ayant une identité de séquence d'au moins 70 %, d'au moins 75 %, d'au moins 80 %, d'au moins 81 %, d'au moins 82 %, d'au moins 83 %, d'au moins 84 %, d'au moins 85 %, d'au moins 86 %, d'au moins 87 %, d'au moins 88 %, d'au moins 89 %, d'au moins 90 %, d'au moins 91 %, d'au moins 92 %, d'au moins 93 %, d'au moins 94 %, d'au moins 95 %, d'au moins 96 %, d'au moins 97 %, d'au moins 98 %, ou d'au moins 99 % avec le polypeptide mature de SEQ ID NO : 8 ;
(iii) un polypeptide GH61 ayant une activité d'amplification cellulolytique codé par un polynucléotide comprenant ou consistant en une séquence de nucléotides ayant une identité de séquences d'au moins 70 %, d'au moins 75 %, d'au moins 80 %, d'au moins 81 %, d'au moins 82 %, d'au moins 83 %, d'au moins 84 %, d'au moins 85 %, d'au moins 86 %, d'au moins 87 %, d'au moins 88 %, d'au moins 89 %, d'au moins 90 %, d'au moins 91 %, d'au moins 92 %, d'au moins 93 %, d'au moins 94 %, d'au moins 95 %, d'au moins 96 %, d'au moins 97 %, d'au moins 98 %, ou d'au moins 99 % avec la séquence codante de polypeptide mature de SEQ ID NO : 7 ; et
(iv) un polypeptide GH61 ayant une activité d'amplification cellulolytique codé par un polynucléotide qui s'hybride au moins dans des conditions de stringence élevée, dans des conditions de stringence très élevées, avec la séquence codante de polypeptide mature de SEQ ID NO :7 ou son complément de pleine longueur ;

et dans laquelle la composition est utilisée pour empêcher l'accumulation et/ou l'élimination de peluches et bouloches issues d'un textile et pour améliorer la blancheur du textile par exposition du textile à la composition durant un procédé de nettoyage.

**2.** Utilisation selon la revendication 1, dans laquelle la composition est une composition détergente pour le linge ou une composition d'assouplissant textile.

**3.** Utilisation selon l'une quelconque de la revendication 1 ou 2, dans laquelle la composition comprend

(i) une cellobiohydrolase I d'*Aspergillus fumigatus* comprenant ou consistant en le polypeptide mature de SEQ ID NO : 2 ;
(ii) une cellobiohydrolase II d'*Aspergillus fumigatus* comprenant ou consistant en le polypeptide de SEQ ID NO : 4 ;
(iii) une bêta-glucosidase d'*Aspergillus fumigatus* comprenant ou consistant en le polypeptide mature de SEQ ID NO : 6 ; ou un variant de celui-ci comprenant les substitutions suivantes : F100D, S283G, N456E et F512Y ; et
(iv) un polypeptide GH61 de *Penicillium* sp. ayant une activité d'amplification cellulolytique comprenant ou consistant en le polypeptide mature de SEQ ID NO : 8.

**4.** Utilisation selon l'une quelconque des revendications 1 à 3, dans laquelle la préparation enzymatique comprend en outre une ou plusieurs enzymes choisies dans le groupe constitué par :

(i) une xylanase d'*Aspergillus fumigatus* ou un homologue de celle-ci,
(ii) une bêta-xylosidase d'*Aspergillus fumigatus* ou un homologue de celle-ci ; ou
(iii) une combinaison de (i) et (ii) ;

dans laquelle la xylanase d'*Aspergillus fumigatus* ou son homologue est choisi dans le groupe constitué par :

(i) une xylanase d'*Aspergillus fumigatus* comprenant ou consistant en le polypeptide mature de SEQ ID NO : 10, SEQ ID NO : 12 ou SEQ ID NO : 14;
(ii) une xylanase comprenant ou consistant en une séquence d'acides aminés ayant une identité de séquence d'au moins 70 %, d'au moins 75 %, d'au moins 80 %, d'au moins 81 %, d'au moins 82 %, d'au moins 83 %, d'au moins 84 %, d'au moins 85 %, d'au moins 86 %, d'au moins 87 %, d'au moins 88 %, d'au moins 89 %, d'au moins 90 %, d'au moins 91 %, d'au moins 92 %, d'au moins 93 %, d'au moins 94 %, d'au moins 95 %, d'au moins 96 %, d'au moins 97 %, d'au moins 98 %, ou d'au moins 99 % avec le polypeptide mature de SEQ ID NO : 10, SEQ ID NO : 12 ou SEQ ID NO : 14 ;
(iii) une xylanase codée par un polynucléotide comprenant ou consistant en une séquence de nucléotides ayant une identité de séquences d'au moins 70 %, d'au moins 75 %, d'au moins 80 %, d'au moins 81 %, d'au moins 82 %, d'au moins 83 %, d'au moins 84 %, d'au moins 85 %, d'au moins 86 %, d'au moins 87 %, d'au moins 88 %, d'au moins 89 %, d'au moins 90 %, d'au moins 91 %, d'au moins 92 %, d'au moins 93 %, d'au moins 94 %, d'au moins 95 %, d'au moins 96 %, d'au moins 97 %, d'au moins 98 %, ou d'au moins 99 % avec la séquence

codante de polypeptide mature de SEQ ID NO : 9, SEQ ID NO : 11 ou SEQ ID NO : 13 ;
(iv) une xylanase codée par un polynucléotide qui s'hybride au moins dans des conditions de stringence élevée, dans des conditions de stringence très élevées, avec la séquence codante de polypeptide mature de SEQ ID NO : 9, SEQ ID NO : 11 ou SEQ ID NO : 13 ; ou son complément de pleine longueur ; et

dans laquelle la bêta-xylosidase d'*Aspergillus fumigatus* ou son homologue est choisie dans le groupe constitué par :

(i) une bêta-xylosidase comprenant ou consistant en le polypeptide mature de la SEQ ID NO : 16 ;
(ii) une bêta-xylosidase comprenant ou consistant en une séquence d'acides aminés ayant une identité de séquence d'au moins 70 %, d'au moins 75 %, d'au moins 80 %, d'au moins 81 %, d'au moins 82 %, d'au moins 83 %, d'au moins 84 %, d'au moins 85 %, d'au moins 86 %, d'au moins 87 %, d'au moins 88 %, d'au moins 89 %, d'au moins 90 %, d'au moins 91 %, d'au moins 92 %, d'au moins 93 %, d'au moins 94 %, d'au moins 95 %, d'au moins 96 %, d'au moins 97 %, d'au moins 98 %, ou d'au moins 99 % avec le polypeptide mature de SEQ ID NO : 16;
(iii) une bêta-xylosidase codée par un polynucléotide comprenant ou consistant en une séquence de nucléotides ayant une identité de séquences d'au moins 70 %, d'au moins 75 %, d'au moins 80 %, d'au moins 81 %, d'au moins 82 %, d'au moins 83 %, d'au moins 84 %, d'au moins 85 %, d'au moins 86 %, d'au moins 87 %, d'au moins 88 %, d'au moins 89 %, d'au moins 90 %, d'au moins 91 %, d'au moins 92 %, d'au moins 93 %, d'au moins 94 %, d'au moins 95 %, d'au moins 96 %, d'au moins 97 %, d'au moins 98 %, ou d'au moins 99 % avec la séquence codante de polypeptide mature de SEQ ID NO : 15 ; et
(iv) une bêta-xylosidase codée par un polynucléotide qui s'hybride au moins dans des conditions de stringence élevée, dans des conditions de stringence très élevées, avec la séquence codante de polypeptide mature de SEQ ID NO : 15 ou son complément de pleine longueur.

**5.** Utilisation selon l'une quelconque des revendications précédentes, dans laquelle la composition comprend au moins une enzyme en plus des enzymes dans la préparation enzymatique, dans laquelle l'au moins une autre enzyme est choisie dans le groupe constitué par les hémicellulases, peroxydases, protéases, cellulases, xylanases, lipases, phospholipases, estérases, cutinases, pectinases, mannanases, pectate lyases, kératinases, réductases, oxydases, phénol oxydases, lipoxygénases, ligninases, pullulanases, tannases, pentosanases, malanases, $\beta$-glucanases, arabinosidases, hyaluronidase, chondroïtinase, laccase, DNase, chlorophyllases, amylases, perhydrolases, peroxydases, xanthanase, et leurs mélanges.

**6.** Méthode de nettoyage d'un textile, comprenant les étapes suivantes :

a) mise en contact du textile avec une liqueur de lavage comprenant une composition détergente pour le linge ;
b) réalisation d'au moins un cycle de lavage ;
c) mise en contact du textile avec de l'eau comprenant une composition d'assouplissant textile ; et
d) réalisation d'au moins un cycle de rinçage ;

dans laquelle la composition détergente et/ou la composition d'assouplissant textile comprennent
une préparation enzymatique comprenant une ou plusieurs enzymes capables de dégrader un matériau cellulosique, et dans laquelle la ou les enzymes capables de dégrader un matériau cellulosique comprennent :

i. une cellobiohydrolase I d'*Aspergillus fumigatus* ;
ii. une cellobiohydrolase II d'*Aspergillus fumigatus* ;
iii. une bêta-glucosidase d'*Aspergillus fumigatus* ou un variant de celle-ci ; et
iv. un polypeptide GH61 de *Penicillium* sp. ayant une activité d'amplification cellulolytique ; ou ses homologues ;

dans laquelle la cellobiohydrolase I d'*Aspergillus fumigatus* ou son homologue est choisi dans le groupe constitué par :

(i) une cellobiohydrolase I comprenant ou consistant en le polypeptide mature de SEQ ID NO : 2 ;
(ii) une cellobiohydrolase I comprenant ou consistant en une séquence d'acides aminés ayant une identité de séquence d'au moins 70 %, d'au moins 75 %, d'au moins 80 %, d'au moins 81 %, d'au moins 82 %, d'au moins 83 %, d'au moins 84 %, d'au moins 85 %, d'au moins 86 %, d'au moins 87 %, d'au moins 88 %, d'au moins 89 %, d'au moins 90 %, d'au moins 91 %, d'au moins 92 %, d'au moins 93 %, d'au moins 94 %, d'au moins 95 %, d'au moins 96 %, d'au moins 97 %, d'au moins 98 %, ou d'au moins 99 % avec le polypeptide mature de SEQ ID NO : 2 ;

(iii) une cellobiohydrolase I codée par un polynucléotide comprenant ou consistant en une séquence de nucléotides ayant une identité de séquences d'au moins 70 %, d'au moins 75 %, d'au moins 80 %, d'au moins 81 %, d'au moins 82 %, d'au moins 83 %, d'au moins 84 %, d'au moins 85 %, d'au moins 86 %, d'au moins 87 %, d'au moins 88 %, d'au moins 89 %, d'au moins 90 %, d'au moins 91 %, d'au moins 92 %, d'au moins 93 %, d'au moins 94 %, d'au moins 95 %, d'au moins 96 %, d'au moins 97 %, d'au moins 98 %, ou d'au moins 99 % avec la séquence codante de polypeptide mature de SEQ ID NO : 1 ; et
(iv) une cellobiohydrolase I codée par un polynucléotide qui s'hybride au moins dans des conditions de stringence élevée, dans des conditions de stringence très élevées, avec la séquence codante de polypeptide mature de SEQ ID NO : 1 ou son complément de pleine longueur ;

dans laquelle la cellobiohydrolase II d'*Aspergillus fumigatus* ou son homologue est choisi dans le groupe constitué par :

(i) une cellobiohydrolase II comprenant ou consistant en le polypeptide mature de SEQ ID NO : 4 ;
(ii) une cellobiohydrolase II comprenant ou consistant en une séquence d'acides aminés ayant une identité de séquence d'au moins 70 %, d'au moins 75 %, d'au moins 80 %, d'au moins 81 %, d'au moins 82 %, d'au moins 83 %, d'au moins 84 %, d'au moins 85 %, d'au moins 86 %, d'au moins 87 %, d'au moins 88 %, d'au moins 89 %, d'au moins 90 %, d'au moins 91 %, d'au moins 92 %, d'au moins 93 %, d'au moins 94 %, d'au moins 95 %, d'au moins 96 %, d'au moins 97 %, d'au moins 98 %, ou d'au moins 99 % avec le polypeptide mature de SEQ ID NO : 4 ;
(iii) une cellobiohydrolase II codée par un polynucléotide comprenant ou consistant en une séquence de nucléotides ayant une identité de séquences d'au moins 70 %, d'au moins 75 %, d'au moins 80 %, d'au moins 81 %, d'au moins 82 %, d'au moins 83 %, d'au moins 84 %, d'au moins 85 %, d'au moins 86 %, d'au moins 87 %, d'au moins 88 %, d'au moins 89 %, d'au moins 90 %, d'au moins 91 %, d'au moins 92 %, d'au moins 93 %, d'au moins 94 %, d'au moins 95 %, d'au moins 96 %, d'au moins 97 %, d'au moins 98 %, ou d'au moins 99 % avec la séquence codante de polypeptide mature de SEQ ID NO : 3 ; et
(iv) une cellobiohydrolase II codée par un polynucléotide qui s'hybride au moins dans des conditions de stringence élevée, dans des conditions de stringence très élevées, avec la séquence codante de polypeptide mature de SEQ ID NO : 3 ou son complément de pleine longueur ;

dans laquelle la bêta-glucosidase d'*Aspergillus fumigatus* ou son homologue est choisi dans le groupe constitué par :

(i) une bêta-glucosidase comprenant ou consistant en le polypeptide mature de SEQ ID NO : 6 ;
(ii) une bêta-glucosidase comprenant ou consistant en une séquence d'acides aminés ayant une identité de séquence d'au moins 70 %, d'au moins 75 %, d'au moins 80 %, d'au moins 81 %, d'au moins 82 %, d'au moins 83 %, d'au moins 84 %, d'au moins 85 %, d'au moins 86 %, d'au moins 87 %, d'au moins 88 %, d'au moins 89 %, d'au moins 90 %, d'au moins 91 %, d'au moins 92 %, d'au moins 93 %, d'au moins 94 %, d'au moins 95 %, d'au moins 96 %, d'au moins 97 %, d'au moins 98 %, ou d'au moins 99 % avec le polypeptide mature de SEQ ID NO : 6 ;
(iii) une bêta-glucosidase codée par un polynucléotide comprenant ou consistant en une séquence de nucléotides ayant une identité de séquences d'au moins 70 %, d'au moins 75 %, d'au moins 80 %, d'au moins 81 %, d'au moins 82 %, d'au moins 83 %, d'au moins 84 %, d'au moins 85 %, d'au moins 86 %, d'au moins 87 %, d'au moins 88 %, d'au moins 89 %, d'au moins 90 %, d'au moins 91 %, d'au moins 92 %, d'au moins 93 %, d'au moins 94 %, d'au moins 95 %, d'au moins 96 %, d'au moins 97 %, d'au moins 98 %, ou d'au moins 99 % avec la séquence codante de polypeptide mature de SEQ ID NO : 5 ;
(iv) une bêta-glucosidase codée par un polynucléotide qui s'hybride au moins dans des conditions de stringence élevée, dans des conditions de stringence très élevées, avec la séquence codante de polypeptide mature de SEQ ID NO : 5 ou son complément de pleine longueur ; et
(v) un variant de bêta-glucosidase comprenant une substitution en une ou plusieurs positions correspondant aux positions 100, 283, 456 et 512 du polypeptide mature de SEQ ID NO : 6, lequel variant a une activité de bêta-glucosidase ;

et dans laquelle le polypeptide GH61 de *Penicillium* sp. ayant une activité d'amplification cellulolytique ou son homologue est choisi dans le groupe constitué par :

(i) un polypeptide GH61 ayant une activité d'amplification cellulolytique comprenant ou consistant en le polypeptide mature de SEQ ID NO : 8 ;
(ii) un polypeptide GH61 ayant une activité d'amplification cellulolytique comprenant ou consistant en une

séquence d'acides aminés ayant une identité de séquence d'au moins 70 %, d'au moins 75 %, d'au moins 80 %, d'au moins 81 %, d'au moins 82 %, d'au moins 83 %, d'au moins 84 %, d'au moins 85 %, d'au moins 86 %, d'au moins 87 %, d'au moins 88 %, d'au moins 89 %, d'au moins 90 %, d'au moins 91 %, d'au moins 92 %, d'au moins 93 %, d'au moins 94 %, d'au moins 95 %, d'au moins 96 %, d'au moins 97 %, d'au moins 98 %, ou d'au moins 99 % avec le polypeptide mature de SEQ ID NO : 8 ;
(iii) un polypeptide GH61 ayant une activité d'amplification cellulolytique codé par un polynucléotide comprenant ou consistant en une séquence de nucléotides ayant une identité de séquences d'au moins 70 %, d'au moins 75 %, d'au moins 80 %, d'au moins 81 %, d'au moins 82 %, d'au moins 83 %, d'au moins 84 %, d'au moins 85 %, d'au moins 86 %, d'au moins 87 %, d'au moins 88 %, d'au moins 89 %, d'au moins 90 %, d'au moins 91 %, d'au moins 92 %, d'au moins 93 %, d'au moins 94 %, d'au moins 95 %, d'au moins 96 %, d'au moins 97 %, d'au moins 98 %, ou d'au moins 99 % avec la séquence codante de polypeptide mature de SEQ ID NO : 7 ; et

un polypeptide GH61 ayant une activité d'amplification cellulolytique codé par un polynucléotide qui s'hybride au moins dans des conditions de stringence élevée, dans des conditions de stringence très élevées, avec la séquence codante de polypeptide mature de SEQ ID NO :7 ou son complément de pleine longueur ;
et laquelle méthode empêche l'accumulation et/ou l'élimination de peluches et bouloches issues d'un textile et améliore la blancheur du textile.

7. Méthode selon la revendication 6, dans laquelle la composition détergente pour le linge et/ou la composition d'assouplissant textile comprend

(iv) une cellobiohydrolase I d'*Aspergillus fumigatus* comprenant ou consistant en le polypeptide mature de SEQ ID NO : 2 ;
(v) une cellobiohydrolase II d'*Aspergillus fumigatus* comprenant ou consistant en le polypeptide de SEQ ID NO : 4 ;
(vi) une bêta-glucosidase d'*Aspergillus fumigatus* comprenant ou consistant en le polypeptide mature de SEQ ID NO : 6 ; ou un variant de celui-ci comprenant les substitutions suivantes : F100D, S283G, N456E et F512Y,
(vii) un polypeptide GH61 de *Penicillium* sp. ayant une activité d'amplification cellulolytique comprenant ou consistant en le polypeptide mature de SEQ ID NO : 8.

8. Méthode selon l'une quelconque des revendications 6 et 7, laquelle méthode de nettoyage est effectuée à la main ou en machine.

9. Méthode selon l'une quelconque des revendications 6 à 8, laquelle méthode est effectuée au moins 5 fois.

10. Méthode selon la revendication 9, laquelle méthode est effectuée au moins 10 fois, au moins 15 fois ou au moins 20 fois.

11. Méthode selon l'une quelconque des revendications 6 à 10, dans laquelle la concentration de la préparation enzymatique est située dans la plage allant de 0,03 à 5 grammes par litre.

12. Méthode selon la revendication 11, dans laquelle la concentration de la préparation enzymatique est située dans la plage allant de 0,05 à 4 grammes par litre, dans la plage allant de 0,1 à 3 grammes par litre, dans la plage de 0,2 à 2,5 grammes par litre, dans la plage allant de 0,2 à 2 grammes par litre, ou dans la plage allant de 0,3 à 1 gramme par litre.

13. Méthode de nettoyage pour nettoyer l'intérieur d'une machine à laver, laquelle méthode comprend l'exposition de l'intérieur de la machine à laver à une ou plusieurs enzymes capables de dégrader un matériau cellulosique, dans laquelle la ou les enzymes capables de dégrader un matériau cellulosique sont une préparation enzymatique comprenant :

(i) une cellobiohydrolase I d'*Aspergillus fumigatus* ;
(ii) une cellobiohydrolase II d'*Aspergillus fumigatus* ;
(iii) une bêta-glucosidase d'*Aspergillus fumigatus* ou un variant de celle-ci ; et
(iv) un polypeptide GH61 de *Penicillium* sp. ayant une activité d'amplification cellulolytique ; ou ses homologues ;

dans laquelle la cellobiohydrolase I d'*Aspergillus fumigatus* ou son homologue est choisi dans le groupe constitué par :

(i) une cellobiohydrolase I comprenant ou consistant en le polypeptide mature de la SEQ ID NO : 2 ;
(ii) une cellobiohydrolase I comprenant ou consistant en une séquence d'acides aminés ayant une identité de séquence d'au moins 70 %, d'au moins 75 %, d'au moins 80 %, d'au moins 81 %, d'au moins 82 %, d'au moins 83 %, d'au moins 84 %, d'au moins 85 %, d'au moins 86 %, d'au moins 87 %, d'au moins 88 %, d'au moins 89 %, d'au moins 90 %, d'au moins 91 %, d'au moins 92 %, d'au moins 93 %, d'au moins 94 %, d'au moins 95 %, d'au moins 96 %, d'au moins 97 %, d'au moins 98 %, ou d'au moins 99 % avec le polypeptide mature de SEQ ID NO : 2 ;
(iii) une cellobiohydrolase I codée par un polynucléotide comprenant ou consistant en une séquence de nucléotides ayant une identité de séquences d'au moins 70 %, d'au moins 75 %, d'au moins 80 %, d'au moins 81 %, d'au moins 82 %, d'au moins 83 %, d'au moins 84 %, d'au moins 85 %, d'au moins 86 %, d'au moins 87 %, d'au moins 88 %, d'au moins 89 %, d'au moins 90 %, d'au moins 91 %, d'au moins 92 %, d'au moins 93 %, d'au moins 94 %, d'au moins 95 %, d'au moins 96 %, d'au moins 97 %, d'au moins 98 %, ou d'au moins 99 % avec la séquence codante de polypeptide mature de SEQ ID NO : 1 ; et
(iv) une cellobiohydrolase I codée par un polynucléotide qui s'hybride au moins dans des conditions de stringence élevée, dans des conditions de stringence très élevées, avec la séquence codante de polypeptide mature de SEQ ID NO : 1 ou son complément de pleine longueur ;

dans laquelle la cellobiohydrolase II d'*Aspergillus fumigatus* ou son homologue est choisi dans le groupe constitué par :

(i) une cellobiohydrolase II comprenant ou consistant en le polypeptide mature de SEQ ID NO : 4 ;
(ii) une cellobiohydrolase II comprenant ou consistant en une séquence d'acides aminés ayant une identité de séquence d'au moins 70 %, d'au moins 75 %, d'au moins 80 %, d'au moins 81 %, d'au moins 82 %, d'au moins 83 %, d'au moins 84 %, d'au moins 85 %, d'au moins 86 %, d'au moins 87 %, d'au moins 88 %, d'au moins 89 %, d'au moins 90 %, d'au moins 91 %, d'au moins 92 %, d'au moins 93 %, d'au moins 94 %, d'au moins 95 %, d'au moins 96 %, d'au moins 97 %, d'au moins 98 %, ou d'au moins 99 % avec le polypeptide mature de SEQ ID NO : 4 ;
(iii) une cellobiohydrolase II codée par un polynucléotide comprenant ou consistant en une séquence de nucléotides ayant une identité de séquences d'au moins 70 %, d'au moins 75 %, d'au moins 80 %, d'au moins 81 %, d'au moins 82 %, d'au moins 83 %, d'au moins 84 %, d'au moins 85 %, d'au moins 86 %, d'au moins 87 %, d'au moins 88 %, d'au moins 89 %, d'au moins 90 %, d'au moins 91 %, d'au moins 92 %, d'au moins 93 %, d'au moins 94 %, d'au moins 95 %, d'au moins 96 %, d'au moins 97 %, d'au moins 98 %, ou d'au moins 99 % avec la séquence codante de polypeptide mature de SEQ ID NO : 3 ; et
(iv) une cellobiohydrolase II codée par un polynucléotide qui s'hybride au moins dans des conditions de stringence élevée, dans des conditions de stringence très élevées, avec la séquence codante de polypeptide mature de SEQ ID NO : 3 ou son complément de pleine longueur ;

dans laquelle la bêta-glucosidase d'*Aspergillus fumigatus* ou son homologue est choisi dans le groupe constitué par :

(i) une bêta-glucosidase comprenant ou consistant en le polypeptide mature de SEQ ID NO : 6 ;
(ii) une bêta-glucosidase comprenant ou consistant en une séquence d'acides aminés ayant une identité de séquence d'au moins 70 %, d'au moins 75 %, d'au moins 80 %, d'au moins 81 %, d'au moins 82 %, d'au moins 83 %, d'au moins 84 %, d'au moins 85 %, d'au moins 86 %, d'au moins 87 %, d'au moins 88 %, d'au moins 89 %, d'au moins 90 %, d'au moins 91 %, d'au moins 92 %, d'au moins 93 %, d'au moins 94 %, d'au moins 95 %, d'au moins 96 %, d'au moins 97 %, d'au moins 98 %, ou d'au moins 99 % avec le polypeptide mature de SEQ ID NO : 6 ;
(iii) une bêta-glucosidase codée par un polynucléotide comprenant ou consistant en une séquence de nucléotides ayant une identité de séquences d'au moins 70 %, d'au moins 75 %, d'au moins 80 %, d'au moins 81 %, d'au moins 82 %, d'au moins 83 %, d'au moins 84 %, d'au moins 85 %, d'au moins 86 %, d'au moins 87 %, d'au moins 88 %, d'au moins 89 %, d'au moins 90 %, d'au moins 91 %, d'au moins 92 %, d'au moins 93 %, d'au moins 94 %, d'au moins 95 %, d'au moins 96 %, d'au moins 97 %, d'au moins 98 %, ou d'au moins 99 % avec la séquence codante de polypeptide mature de SEQ ID NO : 5 ;
(iv) une bêta-glucosidase codée par un polynucléotide qui s'hybride au moins dans des conditions de stringence élevée, dans des conditions de stringence très élevées, avec la séquence codante de polypeptide mature de SEQ ID NO : 5 ou son complément de pleine longueur ; et
(v) un variant de bêta-glucosidase comprenant une substitution en une ou plusieurs positions correspondant aux positions 100, 283, 456 et 512 du polypeptide mature de SEQ ID NO : 6, lequel variant a une activité de bêta-glucosidase ; et

dans laquelle le polypeptide GH61 de *Penicillium* sp. ayant une activité d'amplification cellulolytique ou son homologue est choisi dans le groupe constitué par :

(i) un polypeptide GH61 ayant une activité d'amplification cellulolytique comprenant ou consistant en le polypeptide mature de SEQ ID NO : 8 ;
(ii) un polypeptide GH61 ayant une activité d'amplification cellulolytique comprenant ou consistant en une séquence d'acides aminés ayant une identité de séquence d'au moins 70 %, d'au moins 75 %, d'au moins 80 %, d'au moins 81 %, d'au moins 82 %, d'au moins 83 %, d'au moins 84 %, d'au moins 85 %, d'au moins 86 %, d'au moins 87 %, d'au moins 88 %, d'au moins 89 %, d'au moins 90 %, d'au moins 91 %, d'au moins 92 %, d'au moins 93 %, d'au moins 94 %, d'au moins 95 %, d'au moins 96 %, d'au moins 97 %, d'au moins 98 %, ou d'au moins 99 % avec le polypeptide mature de SEQ ID NO : 8 ;
(iii) un polypeptide GH61 ayant une activité d'amplification cellulolytique codé par un polynucléotide comprenant ou consistant en une séquence de nucléotides ayant une identité de séquences d'au moins 70 %, d'au moins 75 %, d'au moins 80 %, d'au moins 81 %, d'au moins 82 %, d'au moins 83 %, d'au moins 84 %, d'au moins 85 %, d'au moins 86 %, d'au moins 87 %, d'au moins 88 %, d'au moins 89 %, d'au moins 90 %, d'au moins 91 %, d'au moins 92 %, d'au moins 93 %, d'au moins 94 %, d'au moins 95 %, d'au moins 96 %, d'au moins 97 %, d'au moins 98 %, ou d'au moins 99 % avec la séquence codante de polypeptide mature de SEQ ID NO : 7 ; et

un polypeptide GH61 ayant une activité d'amplification cellulolytique codé par un polynucléotide qui s'hybride au moins dans des conditions de stringence élevée, dans des conditions de stringence très élevées, avec la séquence codante de polypeptide mature de SEQ ID NO :7 ou son complément de pleine longueur.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013028928 A1
- WO 2014026630 A1
- WO 02095014 A
- WO 2002095014 A
- WO 2011041397 A
- WO 2005047499 A
- WO 9219709 A
- WO 9219708 A
- WO 9707202 A
- WO 09102854 A
- US 5977053 A
- WO 9817767 A
- EP 624154 A
- WO 2007087258 A
- WO 2007087244 A
- WO 2007087259 A
- EP 1867708 A
- WO 2007087242 A
- WO 2006130575 A
- WO 200503274 A
- WO 200503275 A
- WO 200503276 A
- EP 1876226 A
- WO 2007087257 A
- WO 2007087243 A
- US 4435307 A
- US 5648263 A
- US 5691178 A
- US 5776757 A
- WO 8909259 A
- EP 0495257 A
- EP 0531372 A
- WO 9611262 A
- WO 9629397 A
- WO 9808940 A
- WO 9407998 A
- EP 0531315 A
- US 5457046 A
- US 5686593 A
- US 5763254 A
- WO 9524471 A
- WO 9812307 A
- WO 99001544 A
- WO 2002099091 A
- WO 2001062903 A
- WO 1999064619 A
- WO 9117244 A
- WO 02099091 A
- JP 2000210081 B
- WO 9324618 A
- WO 9510602 A
- WO 9815257 A
- US 7262042 B
- WO 09021867 A
- WO 8906279 A
- WO 9318140 A
- WO 92175177 A
- WO 01016285 A
- WO 02026024 A
- WO 02016547 A
- WO 8906270 A
- WO 9425583 A
- WO 05040372 A
- WO 05052161 A
- WO 05052146 A
- WO 9523221 A
- WO 9221760 A
- EP 1921147 A
- EP 1921148 A
- WO 07044993 A
- WO 9219729 A
- WO 96034946 A
- WO 9820115 A
- WO 9820116 A
- WO 99011768 A
- WO 0144452 A
- WO 03006602 A
- WO 0403186 A
- WO 04041979 A
- WO 07006305 A
- WO 11036263 A
- WO 11036264 A
- US 5352604 A
- EP 258068 A
- EP 305216 A
- WO 9613580 A
- EP 218272 A
- EP 331376 A
- WO 9506720 A
- WO 9627002 A
- WO 9612012 A
- WO 10065455 A
- WO 10107560 A
- US 5389536 A
- WO 11084412 A
- WO 11084417 A
- WO 11084599 A
- WO 11150157 A

- WO 12137147 A
- EP 407225 A
- WO 9205249 A
- WO 9401541 A
- WO 9425578 A
- WO 9514783 A
- WO 9530744 A
- WO 9535381 A
- WO 9522615 A
- WO 9600292 A
- WO 9704079 A
- WO 0034450 A
- WO 0060063 A
- WO 0192502 A
- WO 0787508 A
- WO 09109500 A
- WO 10111143 A
- WO 0556782 A
- WO 0967279 A
- WO 10100028 A
- GB 1296839 A
- WO 9510603 A
- WO 9402597 A
- WO 9418314 A
- WO 9743424 A
- WO 99019467 A
- WO 02010355 A
- WO 2006066594 A
- WO 96023873 A
- WO 08153815 A
- WO 0166712 A
- WO 09061380 A
- WO 13184577 A
- WO 10104675 A
- WO 2011098531 A
- WO 2013001078 A
- WO 2013001087 A
- EP 179486 A
- WO 9527046 A
- WO 9704102 A
- WO 0179459 A
- WO 0179458 A
- WO 0179461 A
- WO 0179460 A
- WO 9201046 A
- JP 2238885 A
- WO 9708325 A
- WO 9533836 A
- US 4106991 A
- US 4661452 A
- GB 1483591 A
- EP 238216 A
- WO 2009087523 A
- WO 2007138054 A
- WO 2006108856 A
- WO 2006113314 A
- EP 1867808 A
- WO 2003040279 A
- EP 2169040 A
- US 20090011970 A1
- WO 2013188331 A
- WO 2013028928 A

**Non-patent literature cited in the description**


- **VENTURI et al.** Extracellular beta-D-glucosidase from Chaetomium thermophilum var. coprophilum: production, purification and some biochemical properties. *J. Basic Microbiol.,* 2002, vol. 42, 55-66
- **TEERI.** Crystalline cellulose degradation: New insight into the function of cellobiohydrolases. *Trends in Biotechnology,* 1997, vol. 15, 160-167
- **TEERI et al.** Trichoderma reesei cellobiohydrolases: why so efficient on crystalline cellulose?. *Biochem. Soc. Trans.,* 1998, vol. 26, 173-178
- **LEVER et al.** *Anal. Biochem.,* 1972, vol. 47, 273-279
- **VAN TILBEURGH et al.** *FEBS Letters,* 1982, vol. 149, 152-156
- **VAN TILBEURGH ; CLAEYSSENS.** *FEBS Letters,* 1985, vol. 187, 283-288
- **TOMME et al.** *Eur. J. Biochem.,* 1988, vol. 170, 575-581
- **ZHANG et al.** Outlook for cellulase improvement: Screening and selection strategies. *Biotechnology Advances,* 2006, vol. 24, 452-481
- **GHOSE.** Measurement of cellulase activities. *Pure Appl. Chem.,* 1987, vol. 59, 257-68
- **ZHANG et al.** *Biotechnology Advances,* 2006, vol. 24, 452-481
- **GHOSE.** *Pure and Appl. Chem.,* 1987, vol. 59, 257-268
- **HENRISSAT B.** A classification of glycosyl hydrolases based on amino-acid sequence similarities. *Biochem. J.,* 1991, vol. 280, 309-316
- **HENRISSAT B. ; BAIROCH A.** Updating the sequence-based classification of glycosyl hydrolases. *Biochem. J.,* 1996, vol. 316, 695-696
- **SHALLOM, D. ; SHOHAM, Y.** Microbial hemicellulases. *Current Opinion In Microbiology,* 2003, vol. 6 (3), 219-228
- **GHOSE ; BISARIA.** *Pure & Appl. Chem.,* 1987, vol. 59, 1739-1752
- **NEEDLEMAN ; WUNSCH.** *J. Mol. Biol,* 1970, vol. 48, 443-453
- **RICE et al.** EMBOSS: The European Molecular Biology Open Software Suite. *Trends Genet.,* 2000, vol. 16, 276-277
- **RICE et al.** *EMBOSS: The European Molecular Biology Open Software Suite,* 2000

- **EBRINGEROVA et al.** *Adv. Polym. Sci.,* 2005, vol. 186, 1-67
- **BIELY ; PUCHARD.** Recent progress in the assays of xylanolytic enzymes. *Journal of the Science of Food and Agriculture,* 2006, vol. 86 (11), 1636-1647
- **SPANIKOVA ; BIELY.** Glucuronoyl esterase - Novel carbohydrate esterase produced by Schizophyllum commune. *FEBS Letters,* 2006, vol. 580 (19), 4597-4601
- **HERRMANN ; VRSANSKA ; JURICKOVA ; HIRSCH ; BIELY ; KUBICEK.** The beta-D-xylosidase of Trichoderma reesei is a multifunctional beta-D-xylan xylohydrolase. *Biochemical Journal,* 1997, vol. 321, 375-381
- **BAILEY ; BIELY ; POUTANEN.** Interlaboratory testing of methods for assay of xylanase activity. *Journal of Biotechnology,* 1992, vol. 23 (3), 257-270
- **LEVER.** A new reaction for colorimetric determination of carbohydrates. *Anal. Biochem,* 1972, vol. 47, 273-279
- **HODGDON ; KALER.** *Current Opinion in Colloid & Interface Science,* 2007, vol. 12, 121-128
- **SIEZEN et al.** *Protein Engng.,* 1991, vol. 4, 719-737
- **SIEZEN et al.** *Protein Science,* 1997, vol. 6, 501-523
- Powdered Detergents. Surfactant science series. Marcel Dekker, Inc, vol. 71
- **JÁNOS SCHANDA.** Colorimetry. Wiley-Interscience, 2007, vol. 61